# EUROPEAN PATENT SPECIFICATION

(11) **EP 4 011 898 B1**
(45) Date of publication and mention of the grant of the patent: **07.01.2026**
(21) Application number: 20852240.9
(22) Date of filing: 07.08.2020
(51) Int. Cl.: A61K 31/57, A61K 31/58, A61P 25/00, A61P 25/22, A61P 25/24, A61P 25/20, A61P 25/28, A61P 25/18, A61P 25/08, A61P 25/16, A61P 25/14, A61P 25/30, C07J 41/00, C07J 43/00

(54) **3-HYDROXY-5-PREGNANE-20-ONE DERIVATIVE AND USE THEREOF**
2-HYDROXY-5-PREGNAN-20-ON-DERIVATE UND VERWENDUNG DAVON
DÉRIVÉ DE 3-HYDROXY-5-PREGNANE-20-ONE ET SON UTILISATION

(30) Priority: 09.08.2019 CN 201910736020
(43) Date of publication of application: 15.06.2022
(73) Proprietor: TWi Biotechnology, Inc., Taipei City 114 (TW)
(72) Inventor: LIU, Fei, Nanjing, Jiangsu 210046 (CN); WU, Gang, Nanjing, Jiangsu 210046 (CN); LIN, Chenggang, Nanjing, Jiangsu 210046 (CN); WANG, Xiaobo, Nanjing, Jiangsu 210046 (CN); WANG, Xiaoqiang, Nanjing, Jiangsu 210046 (CN); WANG, Ruzhi, Nanjing, Jiangsu 210046 (CN); ZHU, Bin, Nanjing, Jiangsu 210046 (CN); XU, Kongchao, Nanjing, Jiangsu 210046 (CN); SUN, Xiaoyan, Nanjing, Jiangsu 210046 (CN); CAI, Jun, Nanjing, Jiangsu 210046 (CN); WANG, Lin, Nanjing, Jiangsu 210046 (CN); JIANG, Weihua, Nanjing, Jiangsu 210046 (CN); ZHENG, Minqiang, Nanjing, Jiangsu 210046 (CN)
(74) Representative: Mewburn Ellis LLP
(86) International application number: PCT/CN2020/107965
(87) International publication number: WO 2021/027744

(56) References cited:
- WO-A1-2011/120044
- WO-A1-2019/108673
- WO-A1-93/03732
- WO-A1-95/21617
- WO-A2-2006/110172
- WO-A2-2009/108804
- WO-A2-2009/120565
- WO-A2-2010/088409
- WO-A2-2015/081170
- CN-A- 101 585 862
- CN-A- 102 753 181
- CN-A- 103 772 469
- CN-A- 108 148 106
- CN-A- 108 517 001
- CN-A- 109 776 647
- US-A1- 2014 005 132
- JIHUA LIU, JINPING LIU, DAN LU, PINGYA LI, YING WANG: "The Synthesis of 20(S)-Protopanaxadiol Amino Acid Derivatives", CHINESE JOURNAL OF MODERN APPLIED PHARMACY, CHINESE PHARMACEUTICAL ASSOCIATION, CN, vol. 27, no. 20, 1 January 2010 (2010-01-01), CN, pages 916 - 920, XP055779662, ISSN: 1007-7693, DOI: 10.13748/j.cnki.issn1007-7693.2010.10.011
- J.D. COCKER, J. ELKS, P.J. MAY, F.A. NICE, G.H. PHILLIPPS, W.F. WALL: "Action of Some Steroids on the Central Nervous System of the Mouse. I. Synthetic Methods", JOURNAL OF MEDICINAL CHEMISTRY, AMERICAN CHEMICAL SOCIETY, US, vol. 8, no. 4, 1 July 1965 (1965-07-01), US, pages 417 - 425, XP002619548, ISSN: 0022-2623, DOI: 10.1021/jm00328a003

## Description

### Field of the invention

The present invention relates to the field of pharmaceutical chemistry; in particular, the present invention relates to the 3-hydroxyl-5-pregnane-20-one derivative of formula (I) and the use thereof for the manufacture of a medicament for preventing or treating a disorder of the central nervous system.

### Background

Neuroactive steroids are active steroids in the nervous tissue, and the neuroactive steroids playing a key role in regulating the human body. Neuroactive steroids mainly comprise progesterone, pregnenolone, allopregnanolone, etc. Progesterone, pregnenolone and allopregnanolone are all produced by cholesterol through different metabolism pathways. Cholesterol transfers, under the mediation of a 18 kDa translin, from the outer membrane of the mitochondria to the inner membrane, produces pregnenolone through the metabolism of cytochrome P450 cholesterol side-chain cleavage enzyme, then produces progesterone through the metabolism of 3β-hydroxysteroid dehydrogenase, and continues to produce allopregnanolone through the metabolism of a series of enzyme reactions mediated by 5α-reductase and 3α-hydroxysteroid dehydrogenase. Neuroactive steroids can be used as anesthetics, tranquillizers, hypnotics, antianxietics, antidepressants and anticonvulsants.

Allopregnanolone has been a hotspot of studies in recent years, and it has been pointed out as early as in 1986 that allopregnanolone is a positive modulator of the GABA_{A} receptor. However, it was not until the year 2006 when people finally found that allopregnanolone may essentially bind to the α and β subunits of the GABA_{A} receptor, increase the open frequency of chloride ion channel in the receptor, and decrease the nerve excitability so as to produce stabilizing and anxiolytic effects.

It has been reported in the literature that during different periods of the menstrual cycle, the levels of progesterone and the metabolites thereof *in vivo* are different. Before the beginning of the menstrual cycle, the levels of progesterone and the metabolite thereof decrease, which can induce premenstrual syndrome (PMS), that is to say, before the beginning of the menstrual cycle, some symptoms would repeatedly appear but they would disappear after the menstrual cycle, such as stress, anxiety and migraine. Postpartum depression would also be related to abnormal levels of progesterone and the metabolite thereof, and with the development of pregnancy, the concentration of allopregnanolone in the plasma of a healthy pregnant woman increases, and after delivery, the concentration of allopregnanolone would drastically decrease.

Researches have suggested that the decrease of the content of allopregnanolone is considered to be closely related to the occurrence and development of many mental disorders such as anxiety, depression and tremor, and the exogenous administration of allopregnanolone can significantly improve the above-mentioned psychiatric symptoms.

However, allopregnanolone has a low water solubility and a poor oral availability, with a human plasma half-life period being about 45 minutes, and can be rapidly metabolized. The marketed Zulresso is a water-soluble, sulfobutyl β-cyclodextrin-based preparation of allopregnanolone that is injected intravenously to produce a stable physiological concentration of allopregnenolone. However, brexanolone requires an intravenous infusion of up to 60 hours, and the compliance of a patient is poor.

CN-A-108 517 001 discloses allopregnanolone esters with amino acids as prodrugs thereof.

Therefore, there is a clinical need for the solution of improved solubility, reducing the duration of administration, and maintainning a stable physiological concentration of allopregnanolone in vivo for a long time.

### Summary of the Invention

An object of the present invention is to provide a derivative of 3-hydroxyl-5-pregnane-20-one, which can be used for the manufacture of a medicament for preventing or treating a disorder of the central nervous system. The derivative has improved solubility, stable storage, convenient administration, and high patient compliance at the time of administration.

In the first respect, the present invention provides a compound of formula I, or a pharmaceutically acceptable salt thereof: 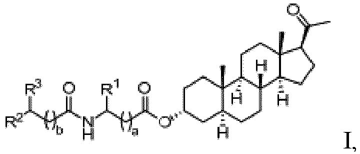 wherein,
each R¹ and R² is independently H, a substituted or unsubstituted C₁-C₁₀ alkyl, or a substituted or unsubstituted cycloalkyl; R³ is H, halogen, hydroxyl, amino, nitryl, or sulfydryl; or R² and R³ are linked to form a 5- to 6-membered saturated or unsaturated heterocyclic ring; and
each a and b is independently an integer of 0 to 3.

In a specific embodiment, R³ is an amino or H.

In a specific embodiment, the compound of formula I is the compound of formula I-1, I-2, I-3 or 1-4: 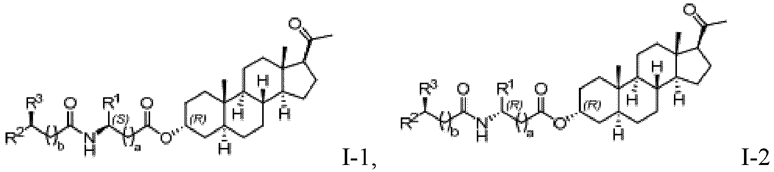 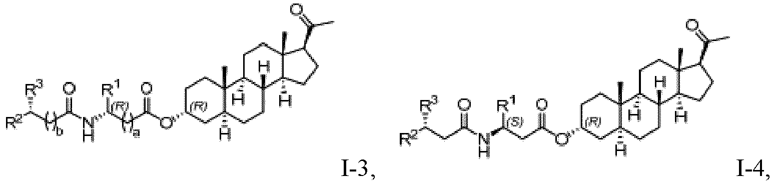 wherein R¹, R², R³, a, and b are as described above.

In a preferred embodiment, each R¹ and R² is independently H, or a substituted or unsubstituted C₁-C₈ alkyl; wherein a substituent of the alkyl is selected from C₁-C₆ alkyl, aryl, hydroxyl-substituted aryl, amino-substituted aryl, halogen-substituted aryl, carboxyl-substituted aryl, heteroaryl, hydroxyl-substituted heteroaryl, amino-substituted heteroaryl, halogen-substituted heteroaryl, carboxyl-substituted heteroaryl, amino, methylamino, dimethylamino, hydroxyl, sulfydryl, methylthio, acylamino, guanidyl or carboxyl.

In a preferred embodiment, each R¹ and R² is independently H, a substituted or unsubstituted C₁ alkyl, a substituted or unsubstituted C₂ alkyl, a substituted or unsubstituted C₃ alkyl, a substituted or unsubstituted C₄ alkyl, a substituted or unsubstituted C₅ alkyl, a substituted or unsubstituted C₆ alkyl, a substituted or unsubstituted C₇ alkyl, or a substituted or unsubstituted C₈ alkyl; wherein a substituent of the alkyl is selected from methyl, ethyl, propyl, isobutyl, butyl, isopropyl, tert-butyl, phenyl, hydroxyl-substituted phenyl, indolyl, imidazolyl, amino, hydroxyl, sulfydryl, methylthio, acylamino, guanidyl, methylamino, dimethylamino or carboxyl.

In a preferred embodiment, each R¹ and R² is independently H, a substituted or unsubstituted methyl, a substituted or unsubstituted ethyl, a substituted or unsubstituted propyl, a substituted or unsubstituted isopropyl, a substituted or unsubstituted n-butyl, a substituted or unsubstituted tert-butyl, a substituted or unsubstituted 2-isobutyl, or a substituted or unsubstituted 1-isobutyl; wherein the substituent is selected from methyl, ethyl, propyl, butyl, isopropyl, isobutyl, tert-butyl, phenyl, 2-hydroxyphenyl, 3-hydroxyphenyl, 4-hydroxyphenyl, indolyl, imidazolyl, amino, hydroxyl, sulfydryl, methylthio, acylamino, methylamino, dimethylamino, guanidyl or carboxyl.

In a preferred embodiment, each R¹ and R² is independently H, a substituted or unsubstituted methyl, a substituted or unsubstituted ethyl, a substituted or unsubstituted propyl, a substituted or unsubstituted isopropyl, a substituted or unsubstituted n-butyl, a substituted or unsubstituted tert-butyl, a substituted or unsubstituted 2-isobutyl, or a substituted or unsubstituted 1-isobutyl; wherein the substituent is selected from methyl, ethyl, propyl, butyl, isopropyl, isobutyl, tert-butyl, amino, hydroxyl, sulfydryl, methylthio, acylamino, methylamino, dimethylamino, guanidyl or carboxyl.

In a preferred embodiment, each R¹ and R² is independently H, methyl, ethyl, propyl, isopropyl, butyl, tert-butyl, isobutyl, amide-substituted methyl, phenyl-substituted methyl, hydroxyl-substituted methyl, carboxyl-substituted methyl, sulfydryl-substituted methyl, imidazole-substituted methyl, indole-substituted methyl, p-hydroxyphenyl-substituted methyl, methylthio-substituted methyl, guanidyl-substituted methyl, amino-substituted methyl, amide-substituted ethyl, hydroxyl-substituted ethyl, carboxyl-substituted ethyl, sulfydryl-substituted ethyl, imidazole-substituted ethyl, indole-substituted ethyl, p-hydroxyphenyl-substituted ethyl, methylthio-substituted ethyl, guanidyl-substituted ethyl, amino-substituted ethyl, amide-substituted propyl, hydroxyl-substituted propyl, carboxyl-substituted propyl, sulfydryl-substituted propyl, imidazole-substituted propyl, indole-substituted propyl, p-hydroxyphenyl-substituted propyl, methylthio-substituted propyl, guanidyl-substituted propyl, amino-substituted propyl, dimethylamino-substituted propyl, methyl-substituted propyl, amide-substituted butyl, hydroxyl-substituted butyl, carboxyl-substituted butyl, sulfydryl-substituted butyl, imidazole-substituted butyl, indole-substituted butyl, p-hydroxyphenyl-substituted butyl, methylthio-substituted butyl, guanidyl-substituted butyl, or amino-substituted butyl.

In a preferred embodiment, each R¹ and R² is independently H, methyl, ethyl, propyl, isopropyl, butyl, tert-butyl, isobutyl, amide-substituted methyl, hydroxyl-substituted methyl, carboxyl-substituted methyl, sulfydryl-substituted methyl, methylthio-substituted methyl, guanidyl-substituted methyl, amino-substituted methyl, amide-substituted ethyl, hydroxyl-substituted ethyl, carboxyl-substituted ethyl, sulfydryl-substituted ethyl, methylthio-substituted ethyl, guanidyl-substituted ethyl, amino-substituted ethyl, amide-substituted propyl, hydroxyl-substituted propyl, carboxyl-substituted propyl, sulfydryl-substituted propyl, methylthio-substituted propyl, guanidyl-substituted propyl, amino-substituted propyl, dimethylamino-substituted propyl, methyl-substituted propyl, amide-substituted butyl, hydroxyl-substituted butyl, carboxyl-substituted butyl, sulfydryl-substituted butyl, methylthio-substituted butyl, guanidyl-substituted butyl, or amino-substituted butyl.

In a specific embodiment, at least one of R¹ and R² is an isopropyl.

In a preferred embodiment, each R¹ and R² is independently H, methyl, ethyl, propyl, isopropyl, butyl, tert-butyl, isobutyl, amide-substituted methyl, phenyl-substituted methyl, hydroxyl-substituted methyl, carboxyl-substituted methyl, sulfydryl-substituted methyl, imidazole-substituted methyl, indole-substituted methyl, p-hydroxyphenyl-substituted methyl, methylthio-substituted methyl, guanidyl-substituted methyl, amino-substituted methyl, amide-substituted ethyl, hydroxyl-substituted ethyl, carboxyl-substituted ethyl, sulfydryl-substituted ethyl, imidazole-substituted ethyl, indole-substituted ethyl, p-hydroxyphenyl-substituted ethyl, methylthio-substituted ethyl, guanidyl-substituted ethyl, amino-substituted ethyl, amide-substituted propyl, hydroxyl-substituted propyl, carboxyl-substituted propyl, sulfydryl-substituted propyl, imidazole-substituted propyl, indole-substituted propyl, p-hydroxyphenyl-substituted propyl, methylthio-substituted propyl, guanidyl-substituted propyl, amino-substituted propyl, dimethylamino-substituted propyl, methyl-substituted propyl, amide-substituted butyl, hydroxyl-substituted butyl, carboxyl-substituted butyl, sulfydryl-substituted butyl, imidazole-substituted butyl, indole-substituted butyl, p-hydroxyphenyl-substituted butyl, methylthio-substituted butyl, guanidyl-substituted butyl, or amino-substituted butyl; and at least one of R¹ and R² is an isopropyl; R³ is H, halogen, hydroxyl, amino, nitryl, or sulfydryl; and each a and b is independently selected from 0, 1, 2, or 3.

In a specific embodiment, R¹ is an isopropyl.

In a preferred embodiment, R² is selected from H, methyl, ethyl, propyl, isopropyl, butyl, tert-butyl, isobutyl, amide-substituted methyl, phenyl-substituted methyl, hydroxyl-substituted methyl, carboxyl-substituted methyl, sulfydryl-substituted methyl, imidazole-substituted methyl, indole-substituted methyl, p-hydroxyphenyl-substituted methyl, methylthio-substituted methyl, 2-amidoethyl, 1-hydroxyethyl, 2-hydroxyethyl, 2-carboxyethyl, sulfydryl-substituted ethyl, imidazole-substituted ethyl, indole-substituted ethyl, p-hydroxyphenyl-substituted ethyl, 2-methylthioethyl, guanidyl-substituted ethyl, 2-aminoethyl, amide-substituted propyl, hydroxyl-substituted propyl, carboxyl-substituted propyl, sulfydryl-substituted propyl, imidazole-substituted propyl, indole-substituted propyl, p-hydroxyphenyl-substituted propyl, methylthio-substituted propyl, 1-guanidinopropyl, 2-guanidinopropyl, 3-guanidinopropyl, 1-aminopropyl, 2-aminopropyl, 3-aminopropyl, 1-dimethylaminopropyl, 2-dimethylaminopropyl, 3-dimethylaminopropyl, 1-methylpropyl, 2-methylpropyl, amide-substituted butyl, hydroxyl-substituted butyl, carboxyl-substituted butyl, sulfydryl-substituted butyl, imidazole-substituted butyl, indole-substituted butyl, p-hydroxyphenyl-substituted butyl, methylthio-substituted butyl, guanidyl-substituted butyl, 1-aminobutyl, 2-aminobutyl, 3-aminobutyl or 4-aminobutyl.

In a preferred embodiment, R² is selected from H, methyl, ethyl, propyl, isopropyl, butyl, tert-butyl, isobutyl, amide-substituted methyl, phenyl-substituted methyl, hydroxyl-substituted methyl, carboxyl-substituted methyl, sulfydryl-substituted methyl, imidazole-substituted methyl, indole-substituted methyl, p-hydroxyphenyl-substituted methyl, methylthio-substituted methyl, 2-amidoethyl, 1-hydroxyethyl, 2-carboxyethyl, 2-methylthioethyl, 3-guanidinopropyl, 3-dimethylaminopropyl, 1-methylpropyl, 2-methylpropyl or 4-aminobutyl.

In a preferred embodiment, R³ is selected from H or amino.

In a preferred embodiment, R² and R³ are linked to form a 5- to 6-membered saturated or unsaturated heterocyclic ring; preferably a 5-membered saturated or unsaturated heterocyclic ring containing one heteroatom; more preferably a 5-membered saturated heterocyclic ring containing one heteroatom; and most preferably pyrrolidinyl.

In a preferred embodiment, each a and b is independently selected from 0, 1, 2, or 3.

In a preferred embodiment, a is selected from 0, b is selected from 0 or 1.

In a preferred embodiment, R¹ is an isopropyl; R² is selected from H, methyl, ethyl, propyl, isopropyl, butyl, tert-butyl, isobutyl, amide-substituted methyl, phenyl-substituted methyl, hydroxyl-substituted methyl, carboxyl-substituted methyl, sulfydryl-substituted methyl, imidazole-substituted methyl, indole-substituted methyl, p-hydroxyphenyl-substituted methyl, methylthio-substituted methyl, 2-amidoethyl, 1-hydroxyethyl, 2-hydroxyethyl, 2-carboxyethyl, sulfydryl-substituted ethyl, imidazole-substituted ethyl, indole-substituted ethyl, p-hydroxyphenyl-substituted ethyl, 2-methylthioethyl, guanidyl-substituted ethyl, 2-aminoethyl, amide-substituted propyl, hydroxyl-substituted propyl, carboxyl-substituted propyl, sulfydryl-substituted propyl, imidazole-substituted propyl, indole-substituted propyl, p-hydroxyphenyl-substituted propyl, methylthio-substituted propyl, 1-guanidinopropyl, 2-guanidinopropyl, 3-guanidinopropyl, 1-aminopropyl, 2-aminopropyl, 3-aminopropyl, 1-dimethylaminopropyl, 2-dimethylaminopropyl, 3-dimethylaminopropyl, 1-methylpropyl, 2-methylpropyl, amide-substituted butyl, hydroxyl-substituted butyl, carboxyl-substituted butyl, sulfydryl-substituted butyl, imidazole-substituted butyl, indole-substituted butyl, p-hydroxyphenyl-substituted butyl, methylthio-substituted butyl, guanidyl-substituted butyl, 1-aminobutyl, 2-aminobutyl, 3-aminobutyl, or 4-aminobutyl; R³ is selected from H, or amino; and each a and b is independently selected from 0, 1, 2 or 3.

In a preferred embodiment, R¹ is an isopropyl; R² is selected from H, methyl, ethyl, propyl, isopropyl, butyl, tert-butyl, isobutyl, amide-substituted methyl, phenyl-substituted methyl, hydroxyl-substituted methyl, carboxyl-substituted methyl, sulfydryl-substituted methyl, imidazole-substituted methyl, indole-substituted methyl, p-hydroxyphenyl-substituted methyl, methylthio-substituted methyl, 2-amidoethyl, 1-hydroxyethyl, 2-carboxyethyl, 2-methylthioethyl, 3-guanidinopropyl, 3-dimethylaminopropyl, 1-methylpropyl, 2-methylpropyl or 4-aminobutyl; R³ is selected from H or amino; a is selected from 0; and b is selected from 0 or 1.

In a preferred embodiment, R¹ is an isopropyl; R² and R³ are linked to form a 5- to 6-membered saturated or unsaturated heterocyclic ring; and each a and b is independently selected from 0, 1, 2 or 3.

In a preferred embodiment, R² and R³ are linked to form a 5-membered saturated or unsaturated heterocyclic ring containing one heteroatom; preferably a 5-membered saturated heterocyclic ring containing one heteroatom; more preferably pyrrolidinyl; a is selected from 0; and b is selected from 0 or 1.

In the second respect, the present invention provides a compound or a pharmaceutically acceptable salt thereof selected from the group consisting of:

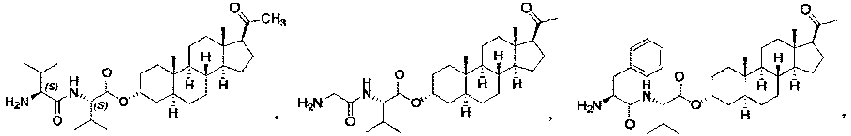

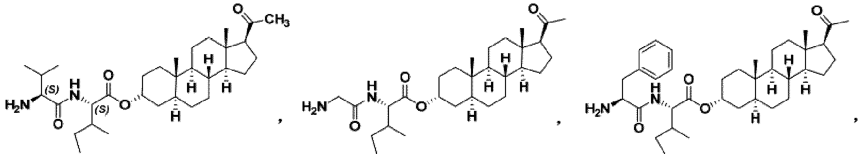

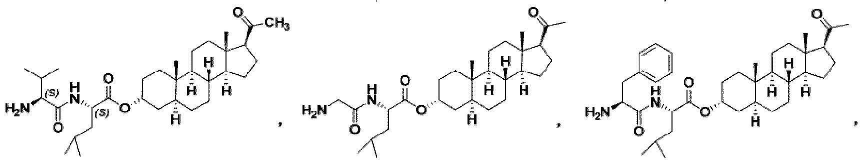

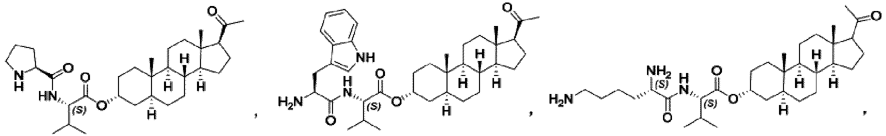

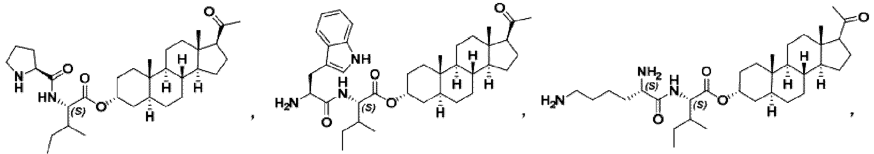

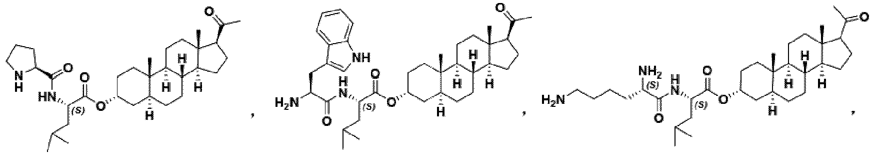

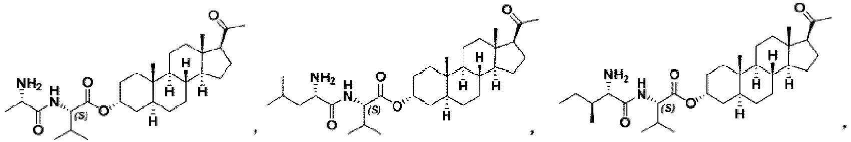

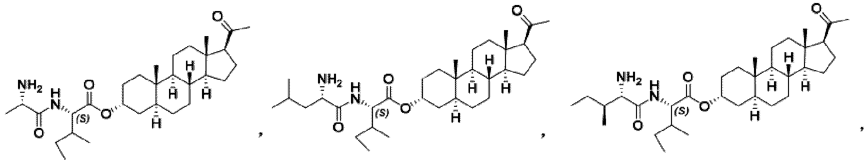

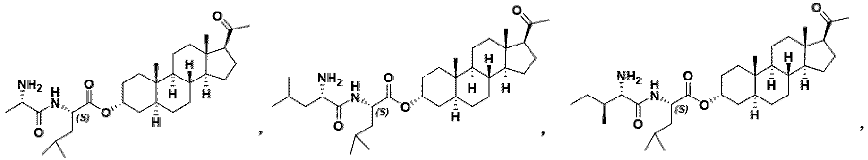

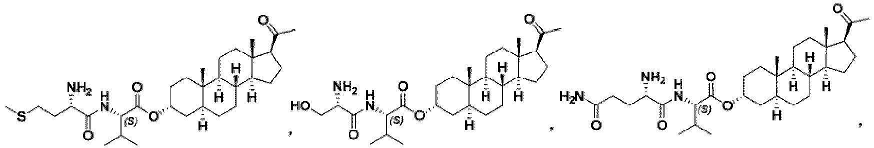

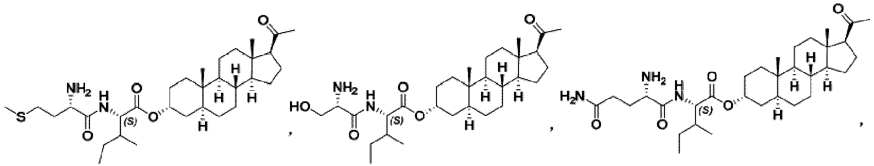

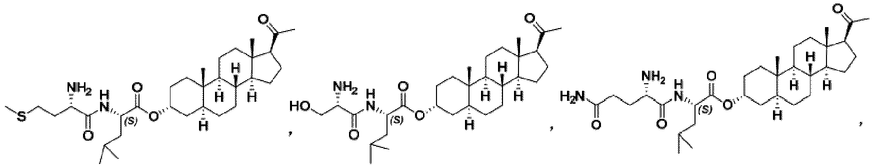

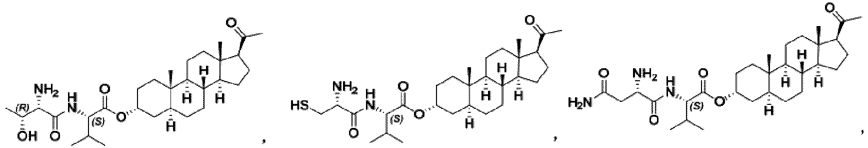

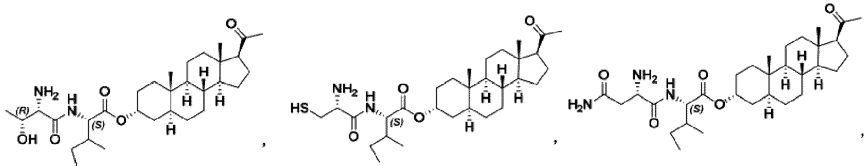

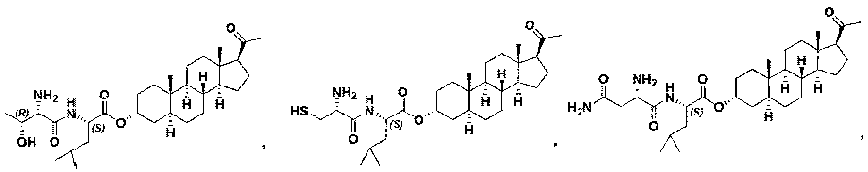

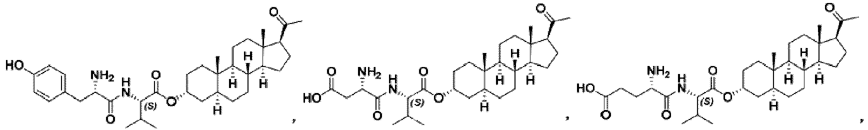

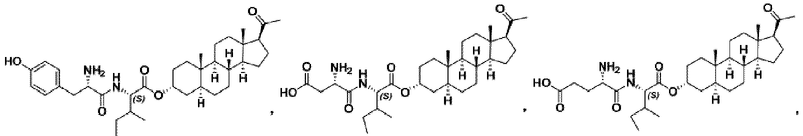

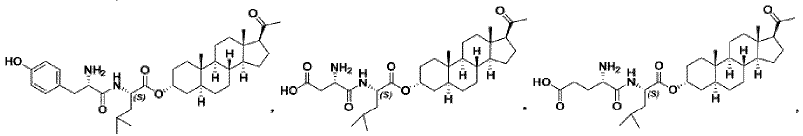

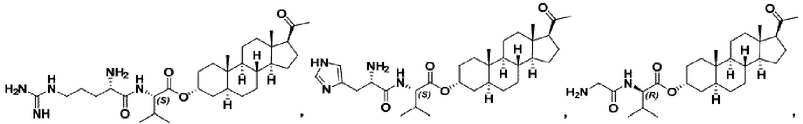

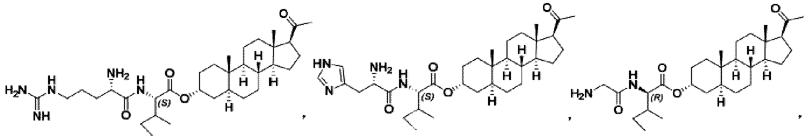

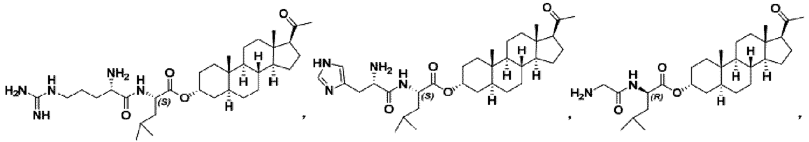

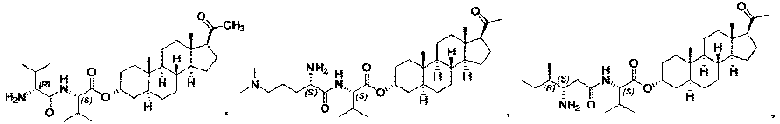

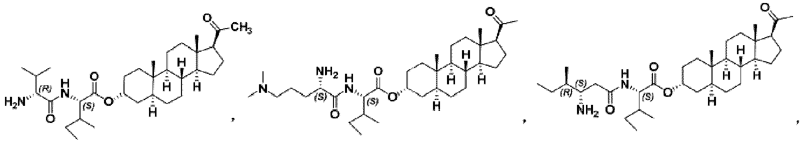

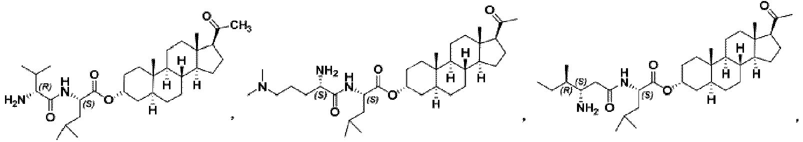

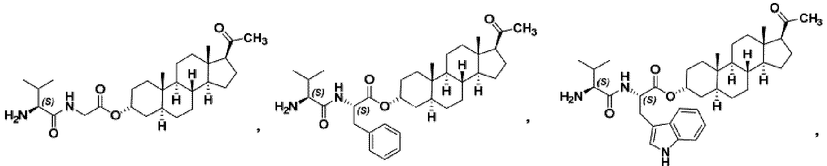

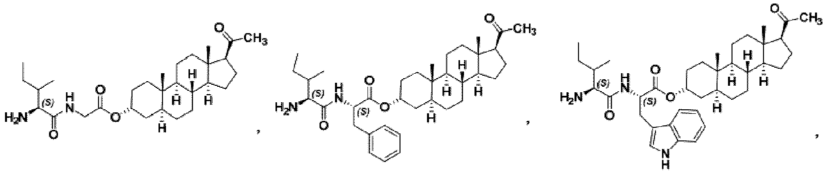

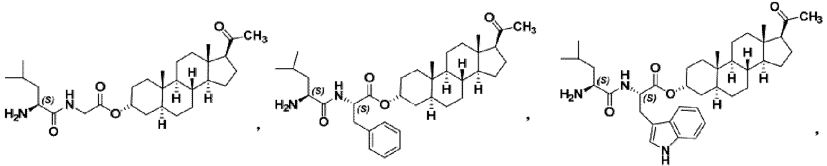

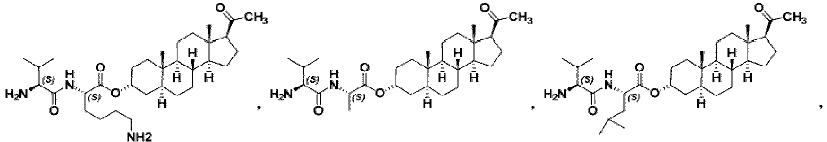

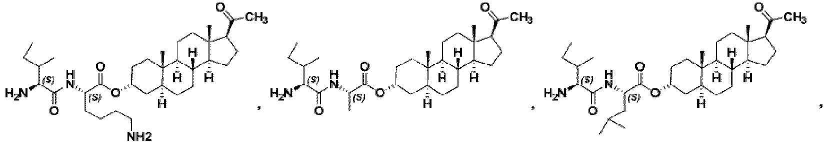

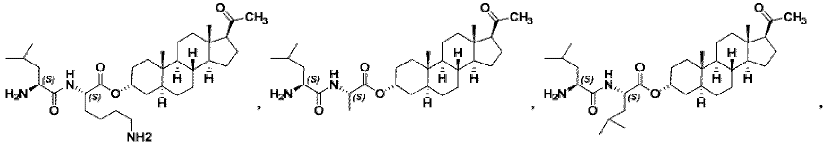

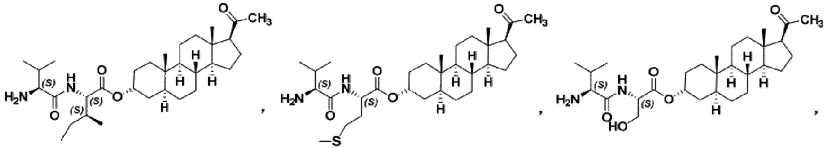

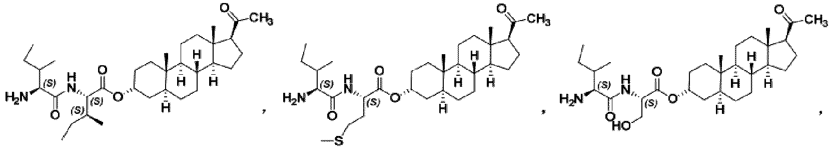

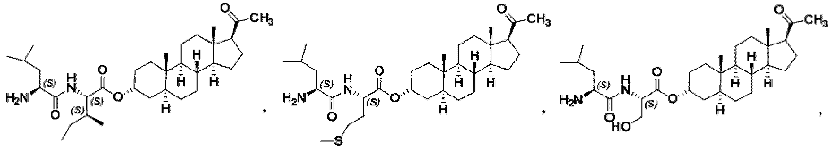

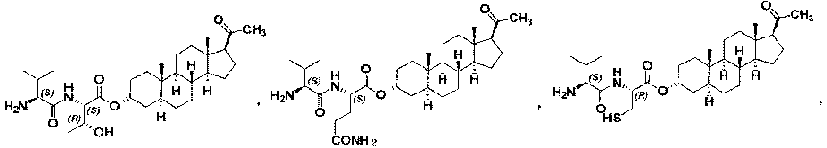

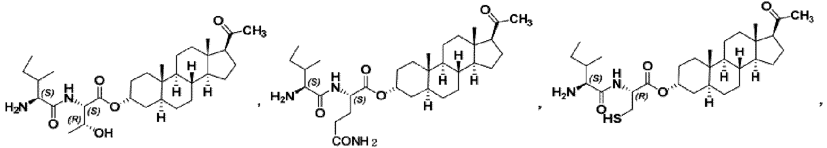

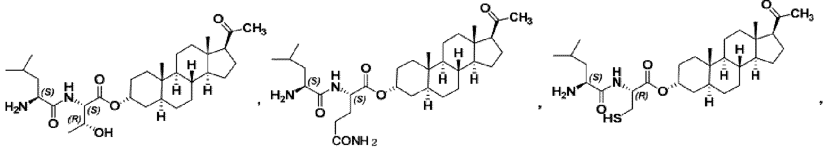

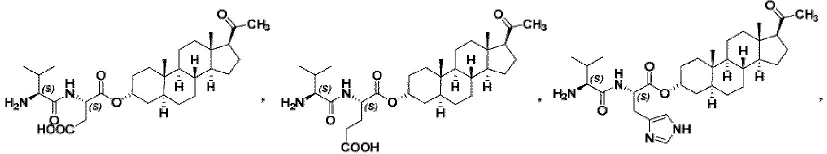

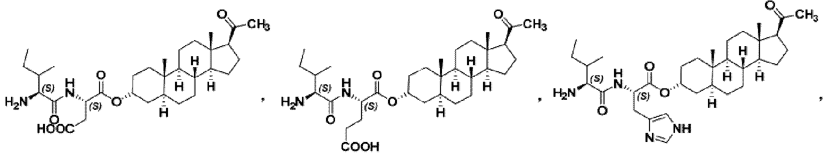

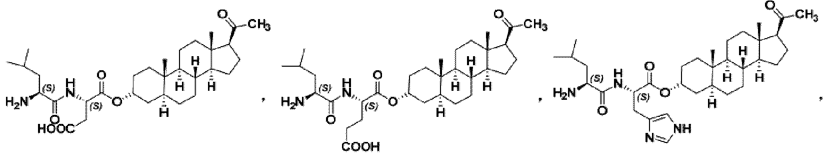

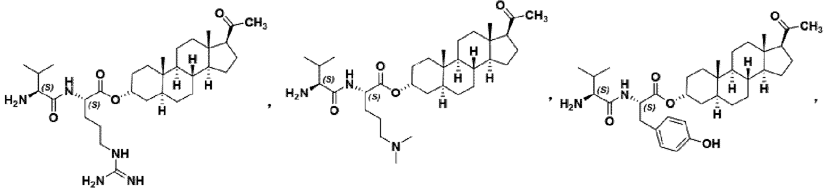

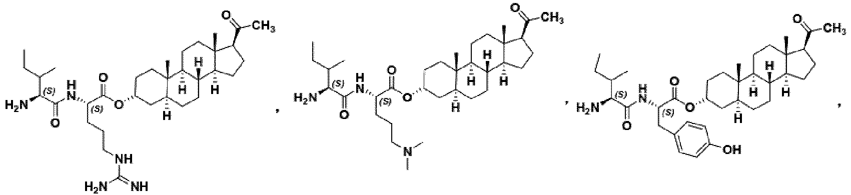

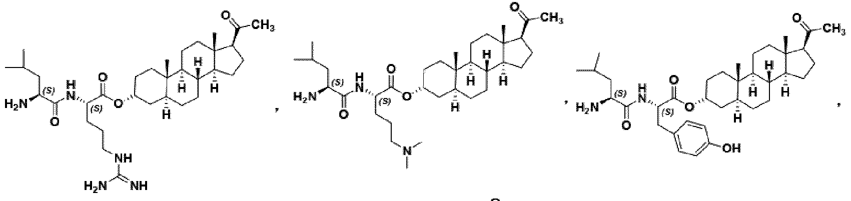

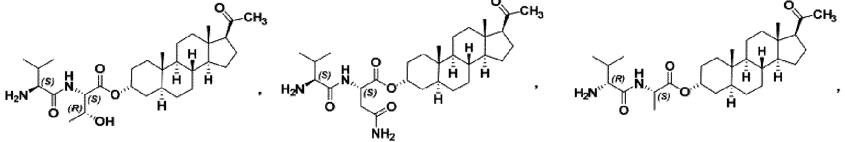

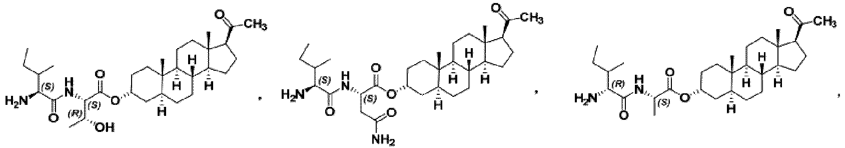

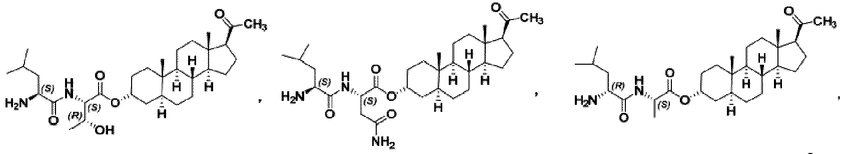

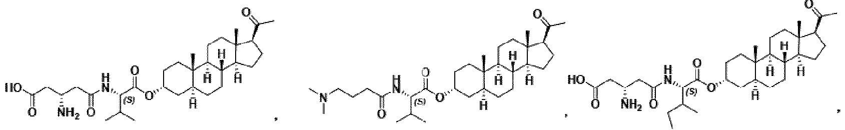

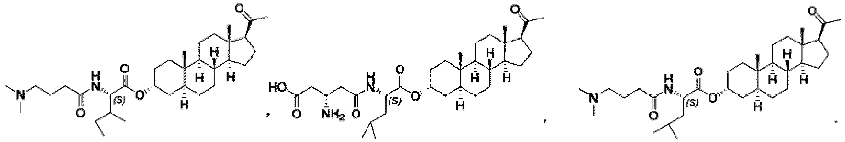

In the third respect, the present invention provides a compound or a pharmaceutically acceptable salt thereof selected from the group consisting of:

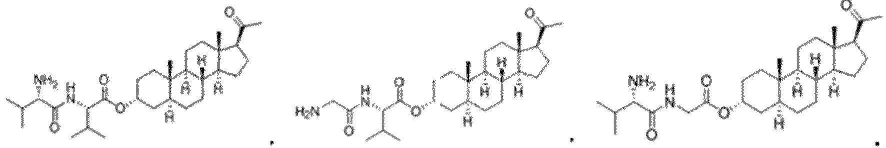

In the fourth respect, the present invention provides a pharmaceutical composition comprising a therapeutically effective dose of the compound or the pharmaceutically acceptable salt thereof of the first to the third respects, and a pharmaceutically acceptable carrier and/or excipient.

In a preferred embodiment, the pharmaceutical composition is a pharmaceutical composition for preventing or treating a disorder of the central nervous system.

In a preferred embodiment, the disorder of the central nervous system includes, but is not limited to, tremor, a sleep disorder, depression, a depressive disorder, a bipolar disorder, an anxiety disorder, a stress response, a post-traumatic stress disorder, an obsessive-compulsive disorder, schizophrenia, a schizoaffective disorder, epilepsy, epileptic seizure, memory impairment and/or cognitive impairment, dementia, dyskinesia, a personality disorder, autism, single-cause autism, pain, a traumatic brain injury, a vascular disease, a substance abuse disorder and/or withdrawal syndrome or tinnitus; or
the disorder of the central nervous system includes, but is not limited to, essential tremor, epilepsy, clinical depression, postnatal or postpartum depression, atypical depression, psychotic major depression, catatonic depression, a seasonal affective disorder, dysthymia, double depression, a depressive personality disorder, recurrent brief depression, a mild depressive disorder, a bipolar disorder or a manic-depressive disorder, a post-traumatic stress disorder, depression due to chronic medical conditions, treatment-resistant depression, refractory depression, suicidal tendency, suicidal ideation, a suicidal behavior, a traumatic brain injury, a generalized anxiety disorder, a social anxiety disorder, an attention deficit hyperactivity disorder, dementia, Huntington's disease, Parkinson's disease, neuropathic pain, an injury-related pain syndrome, acute pain, long-term pain, stroke, ischemia, vascular malformation, addiction to opioids, cocaine and/or alcohol, or insomnia.

In a specific embodiment, the disorder of the central nervous system includes, but is not limited to, tremor, a sleep disorder, depression, a depressive disorder, a bipolar disorder, an anxiety disorder, a stress response, a post-traumatic stress disorder, an obsessive-compulsive disorder, schizophrenia, a schizoaffective disorder, epilepsy, epileptic seizure, memory impairment and/or cognitive impairment, dementia, dyskinesia, a personality disorder, autism, single-cause autism, pain, a traumatic brain injury, a vascular disease, a substance abuse disorder and/or withdrawal syndrome or tinnitus; or
the disorder of the central nervous system includes, but is not limited to, essential tremor, epilepsy, clinical depression, postnatal or postpartum depression, atypical depression, psychotic major depression, catatonic depression, a seasonal affective disorder, dysthymia, double depression, a depressive personality disorder, recurrent brief depression, a mild depressive disorder, a bipolar disorder or a manic-depressive disorder, a post-traumatic stress disorder, depression due to chronic medical conditions, treatment-resistant depression, refractory depression, suicidal tendency, suicidal ideation, a suicidal behavior, a traumatic brain injury, a generalized anxiety disorder, a social anxiety disorder, an attention deficit hyperactivity disorder, dementia, Huntington's disease, Parkinson's disease, neuropathic pain, an injury-related pain syndrome, acute pain, long-term pain, stroke, ischemia, vascular malformation, addiction to opioids, cocaine and/or alcohol, or insomnia.

In the sixth respect, the present invention provides the compound or the pharmaceutically acceptable salt thereof of the first to the third respects, or the pharmaceutical composition of the second respect, which are used as a medicament for preventing or treating a disorder of the central nervous system.

In the seventh respect, the present invention provides a method for preventing or treating a disorder of the central nervous system, comprising administering a therapeutically effective amount of the compound or the pharmaceutically acceptable salt thereof of the first to the third respects, or the pharmaceutical composition of the fourth respect to an subject in need thereof.

In a preferred embodiment, the subject is a mammal, preferably a human.

It should be understood that, within the scope of the present invention, each of the above-mentioned technical features of the present invention and each of the technical features specifically described hereinafter (e.g., embodiments) may be combined with each other to constitute a new or preferred technical solution. Due to space limitations, it will not be repeated herein.

### Brief description of the Drawings

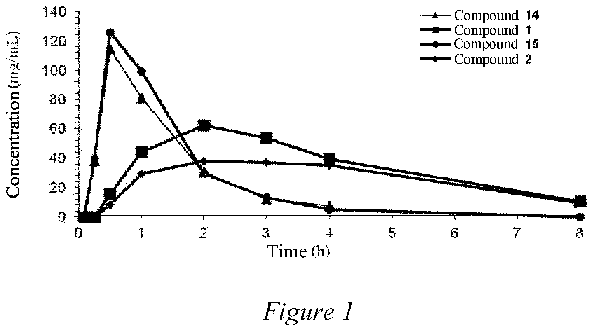

Figure 1 shows the pharmacokinetic curve of male rats after oral administration of a compound of the present invention.

### Detailed Description of invention

Through study, the present inventors have found that allopregnanolone has poor water solubility, needs to be prepared into cyclodextrin aqueous formulation clinically, and requires long-time injection to be effective. After extensive and intensive study, the present inventors have unexpectedly found that the allopregnanolone into a specific derivative can significantly improve the water solubility of allopregnanolone, has a certain storage stability in an aqueous solution, and can be formulated into a long-acting, and sustained-release preparation with little individual difference after administration. The preparation formulated by a derivative of the present invention can maintain an effective physiological concentration of allopregnanolone in vivo for a relatively long time and is convenient to be administered, thus having the advantages of improving the patient's compliance. The present invention has been completed on this basis.

### Definitions

Unless stated to the contrary, the terms used in the description and claims have the following meanings.

The carbon, hydrogen, oxygen, sulfur, nitrogen or halogen involved in the groups and compound of the present invention all comprises their isotopes, and the carbon, hydrogen, oxygen, sulfur, nitrogen or halogen involved in the groups and compound of the present invention is optionally further substituted by one or more of their corresponding isotopes, wherein the isotopes of carbon comprise ¹²C, ¹³C and ¹⁴C, the isotopes of hydrogen comprise protium (H), deuterium (D, also known as heavy hydrogen), tritium (T, also known as superheavy hydrogen), the isotopes of oxygen comprise ¹⁶O, ¹⁷O and ¹⁸O, the isotopes of sulfur comprise ³²S, ³³S, ³⁴S and ³⁶S, the isotopes of nitrogen comprise ¹⁴N and ¹⁵N, the isotopes of fluorine comprise ¹⁹F, the isotopes of chlorine comprise ³⁵Cl and ³⁷Cl, and the isotopes of bromine comprise ⁷⁹Br and ⁸¹Br.

The term "alkyl" as used herein has the meaning as commonly understood by those skilled in the art, and specifically refers to a straight or branched saturated group made up of carbon and hydrogen having the specified number of carbon atoms. For embodiment, C₁-C₁₀ alkyl as used herein refers to a straight or branched alkyl having 1 to 10 carbon atoms; including, but not limited to: n-methyl, n-ethyl, n-propyl, isopropyl, n-butyl, tert-butyl, isobutyl, sec-butyl, n-pentyl, n-hexyl, and n-heptyl, etc.

The term "cycloalkyl" as used herein refers to a monocyclic, fused, spiro or bridged ring which is all carbon, including, but not limited to cyclopropane, cyclobutane, cyclopentane, spiro[3.4]octane, and bicyclo[3.1.1]hexane, etc.

The term "5- to 6-membered saturated or unsaturated heterocyclic ring" as used herein refers to a substituted or unsubstituted saturated or unsaturated non-aromatic ring system containing at least 1 or 2 atoms or groups selected from N, O, S, S(=O) or S(=O)2, and the non-aromatic ring system comprises 5 or 6 ring atoms; and non-limiting embodiments comprise azacyclopentyl, azacyclohexyl, 1,3-dioxocyclopentyl, 1,4-dioxocyclopentyl, 1,3-dioxocyclopentyl, 1,3-dioxocyclohexyl, 1,3-dithiocyclohexyl, morpholinyl, piperazinyl, pyridyl, furyl, thienyl, pyrrolyl, pyranyl, N-alkylpyrrolyl, pyrimidinyl, pyrazinyl, pyridazinyl, imidazolyl, piperidyl, thiomorpholinyl, dihydropyran, thiadiazolyl, oxazolyl, oxadiazolyl, pyrazolyl, 1,4-dioxocyclohexadienyl, 2H-1,2-oxazinyl or 2,5-dihydrothienyl, etc.

The term "halogen" as used herein refers to F, Cl, Br, or I.

The term "pharmaceutically acceptable salt" as used herein refers to those salts that are suitable for the contact with the tissues of human and lower animals but show no excessive toxicity, irritation, and allergic reaction, etc. within a reliable medical judgement range, and are proportional to the reasonable benefit/danger. The pharmaceutically acceptable salts are well-known in the art, for embodiment, the pharmaceutically acceptable salt described in detail by Berge et al., J. Pharmaceutical Sciences (1977) 66: 1-19. The pharmaceutically acceptable salt of the compound of the present invention comprises a salt derived from suitable inorganic and organic acids and bases. embodiments of pharmaceutically acceptable non-toxic acid addition salts are a salt formed by amino group and an inorganic acid, such as hydrochloric acid, hydrobromic acid, phosphoric acid, sulfuric acid and perchloric acid, or a salt formed by amino group and an organic acid, such as acetic acid, oxalic acid, maleic acid, tartaric acid, citric acid, succinic acid or malonic acid, or a salt formed using the method used in the art, such as the ion exchange method. Other pharmaceutically acceptable salts comprise: adipate, alginate, ascorbate, aspartate, benzene sulfonate, benzoate, bisulfate, borate, butyrate, camphorate, camphorsulfonate salt, citrate, cypionate, digluconate, lauryl sulfate, ethyl sulfonate, formate, fumarate, gluconate, glycerophosphate, gluconate, hemisulphate, enanthate, caproate, hydriodate, 2-hydroxyl-ethyl sulfonate, lactobionate, lactate, laurate, lauryl sulfate, malate, maleate, malonate, mesylate, 2-naphthalene sulfonate, nicotinate, nitrate, oleate, oxalate, palmitate, pamoate, pectate, persulfate, 3-phenpropionate, phosphate, picrate, pivalate, propionate, stearate, succinate, sulfate, tartrate, thiocyanate, tosilate, undecanoate, and valerate, etc. Pharmaceutically acceptable salts derived from suitable bases comprise an alkali metal salt, an alkaline-earth metal salt, an ammonium salt and N+(C1-4alkyl)4 salt. Representative alkali metal salt or alkaline-earth metal salt comprises sodium salt, lithium salt, potassium salt, calcium salt, and magnesium salt, etc. If appropriate, further pharmaceutically acceptable salts comprise non-toxic ammonium salts, quaternary ammonium salt and amine cation formed using counterions, wherein the counterions are such as halide anion, hydroxy radical, carboxylate radical, sulfate radical, phosphate radical, nitrate radical, low alkyl sulfonate and aryl sulfonate.

### 3-hydroxyl-5-pregnane-20-one derivative of the present invention and preparation method thereof

In order to realize the object of the present invention, the present inventors derivate an allopregnanolone compound, and 3-hydroxyl-5-pregnane-20-one, so as to obtain the 3-hydroxyl-5-pregnane-20-one derivative of formula I:

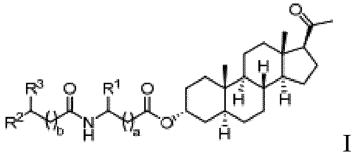

Wherein each R¹ and R² is independently H, a substituted or unsubstituted C₁-C₁₀ alkyl, or a substituted or unsubstituted cycloalkyl; R³ is H, halogen, hydroxyl, amino, nitryl, or sulfydryl; or R² and R³ are linked to form a 5- to 6-membered saturated or unsaturated heterocyclic ring; and each a and b is independently an integer of 0 to 3.

Based on the teachings of the present invention and conventional technical means in the art, those skilled in the art will know that the compound of formula I above can be further resolved into the compound of formula I-1 or I-2 or I-3 or I-4:

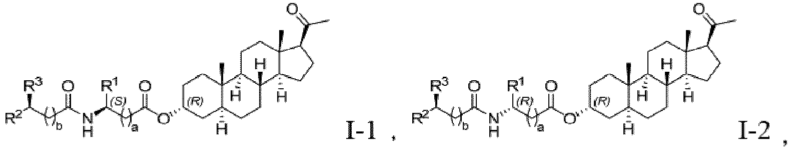

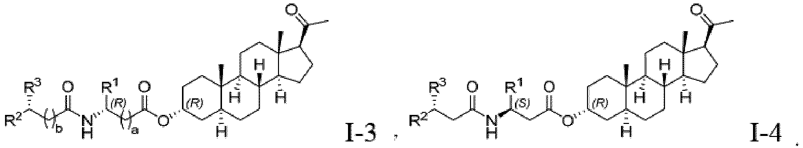

The compound described in the present invention can comprise one or more asymmetric centres, and therefore can have multiple isomer forms, such as the forms of an enantiomer and/or a diastereoisomer. For embodiment, the compound described herein may be in the form of an enantiomer, a diastereomer or a geometric isomer alone, or may be in the form of a stereoisomeric mixture, including a racemic mixture and a mixture rich in one or more stereoisomers. The isomers can be separated from mixtures by methods known to those skilled in the art, including: chiral high performance liquid chromatography (HPLC) and formation and crystallization of a chiral salt; or preferred isomers can be prepared by asymmetric synthesis. The present invention further includes the compound described herein as an isomer alone that is substantially free of other isomers, or as a mixture of multiple isomers.

The present inventors have unexpectedly found that the 3-hydroxyl-5-pregnane-20-one derivative of the present invention has improved water solubility and can maintain a certain stability when stored in an aqueous glucose solution; particularly when R¹ or R² in the 3-hydroxyl-5-pregnane-20-one derivative of formula I of the present invention is isopropyl, the water solubility is significantly improved. After further study, the present inventors have found that the 3-hydroxyl-5-pregnane-20-one derivative of the present invention has excellent pharmacokinetic properties; particularly when R¹ in the 3-hydroxyl-5-pregnane-20-one derivative of formula I of the present invention is isopropyl, the pharmacokinetic properties of the derivative are superior to those of a prototype drug and other derivatives with a similar structure.

It is known to those skilled in the art that although ester formation is one of the commonly used modification methods in the design of prodrugs, however, a major problem faced by ester prodrugs is that it is difficult to predict the pharmacokinetic distribution of ester prodrugs, and since the biotransformation of a substituted or unsubstituted alkyl ester in human blood is relatively slow and incomplete, the bioavailability of these ester prodrugs is often lower than expected; in other words, how an ester is formed and whether the resulting prodrugs thereafter will have the desired properties are unpredictable. Therefore, it is very unexpected that the 3-hydroxyl-5-pregnane-20-one derivative of the present invention has excellent pharmacokinetic properties.

Based on the teachings of the present invention and the common general knowledge in the field of chemical synthesis, those skilled in the art know how to obtain the 3-hydroxyl-5-pregnane-20-one derivative of the present invention. For embodiment, the 3-hydroxyl-5-pregnane-20-one derivative is prepared by reacting the allopregnanolone compound of the following formula with a corresponding organic acid.

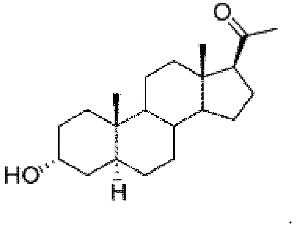

An allopregnanolone used for preparing the compound of the present invention may be commercially available or may be prepared according to a known method.

### Pharmaceutical Composition of the Present Invention

The present inventors have found that the 3-hydroxyl-5-pregnane-20-one derivative of the present invention, after being administered to a subject, can be hydrolyzed under suitable conditions, e.g., hydrolysis by esterase in vivo, thereby releasing an active allopregnanolone. Therefore, the 3-hydroxyl-5-pregnane-20-one derivative of the present invention or a composition comprising same can be used for preparing a medicament for a disease caused by abnormality of the central nervous system. A disease caused by abnormality of the central nervous system includes, but is not limited to, tremor, epilepsy, depression or an anxiety disorder. In more detail, the disorder of the central nervous system includes, but is not limited to, essential tremor, epilepsy, clinical depression, postnatal or postpartum depression, atypical depression, psychotic major depression, catatonic depression, a seasonal affective disorder, dysthymia, double depression, a depressive personality disorder, recurrent brief depression, a mild depressive disorder, a bipolar disorder or a manic-depressive disorder, a post-traumatic stress disorder, depression due to chronic medical conditions, treatment-resistant depression, refractory depression, suicidal tendency, suicidal ideation or a suicidal behavior.

In view of this, the present invention further provides a pharmaceutical composition comprising the above-mentioned 3-hydroxyl-5-pregnane-20 ketone derivative of the present invention and an optional pharmaceutically acceptable carrier. As used herein, the term "composition" is intended to encompass a product comprising a particular amount of a particular ingredient, as well as any product, directly or indirectly, resulting from a combination of a particular amount of a particular ingredient; while a pharmaceutically acceptable carrier refers to a carrier, a diluent or an excipient which does not cause significant irritation to an organism and does not interfere with the biological activity and properties of the compound administered; that is, the carrier, diluent or excipient must be compatible with other ingredients of the preparation and not deleterious to the subject thereof.

The pharmaceutical composition of the present invention may be prepared by a method well known to those skilled in the art. For embodiment, the compound of the present invention may be mixed with a pharmaceutically acceptable carrier, a diluent or an excipient to prepare the corresponding pharmaceutical composition. Further, the compound or pharmaceutical composition of the present invention can be formulated into various suitable dosage forms by those skilled in the art, including, but not limited to the form suitable for rectal administration, transdermal administration, intradermal administration, intrathecal administration, subcutaneous administration, intravenous administration, intramuscular administration, articular cavity administration, oral mucosa administration, vaginal administration, and intranasal administration, etc. Depending on the dosage form required, those skilled in the art can also select the corresponding pharmaceutically acceptable carrier, diluent or excipient.

### Compounds for use in a Method for Preventing and Treating Disease

As described above, whereas the 3-hydroxyl-5-pregnane-20-one derivative of the present invention can release an active allopregnanolone under suitable conditions after being administered to a subject. It is understood by those skilled in the art that the 3-hydroxyl-5-pregnane-20-one derivative of the present invention is provided for use in a method for preventing or treating a disorder of the central nervous system, including, but not limited to the above-mentioned disorders of the central nervous system.

The method for preventing or treating a disorder of the central nervous system of the present invention comprises administering a therapeutically effective amount of the above-mentioned compound and pharmaceutical composition to an subject in need thereof. The subject includes, but is not limited to human.

### Advantages of the Present Invention:

1. The 3-hydroxyl-5-pregnane-20-one derivative of the present invention has an improved solubility;
2. the 3-hydroxyl-5-pregnane-20-one derivative of the present invention has a certain storage stability in a glucose solution;
3. the 3-hydroxyl-5-pregnane-20-one derivative of the present invention can be formulated into a long-acting, and sustained-release preparation with a little individual difference after administration;
4. the preparation of the present invention is convenient to be administered; and
5. the preparation of the present invention has a high patient compliance at the time of administration.

The present invention is further described below in conjunction with specific embodiments. It is to be understood that these embodiments serve only to illustrate the present invention and are not limiting the scope of the present invention, the invention in its broadest scope being as defined in the claims. In the following embodiments, experimental methods without specifying specific conditions are generally performed under conventional conditions or following the manufacturer's recommended conditions. Percentages and parts are by weight unless otherwise specified.

Reagents and raw materials used in the present invention are all commercially available.

### Example 1. Synthesis of Compound 1 Hydrochloride

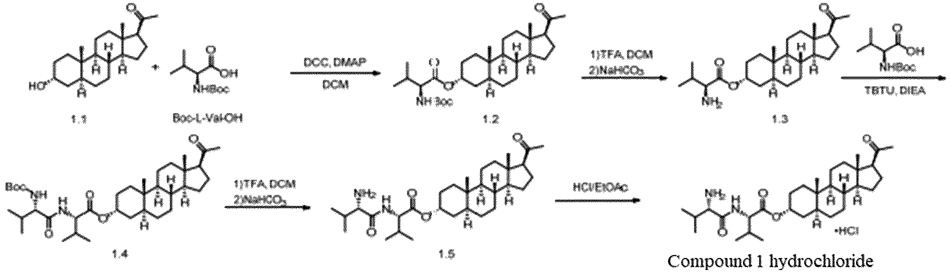

### Preparation of Intermediate 1.2:

Compound 1.1 (50.0 g, 157.0 mmol, 1.0 eq), Boc-L-Val-OH (40.9 g, 188.2 mmol), DMAP (1.9 g, 15.5 mmol) and 500 mL of dichloromethane were added into a 1,000 mL three-neck round-bottom reaction flask, and magnetically stirred, and the reaction system was cooled to -5 to 10°C under nitrogen protection. A dichloromethane solution (80 mL) of DCC (38.9 g, 188.5 mmol) was added dropwise, then reacted at this temperature for 3 hours. The reaction was detected to be complete by TLC then quenched. The reaction solution was filtered, and the cake was washed with dichloromethane (100 mL). The filtrate was concentrated under a reduced pressure, and mixed with 100- to 200-mesh silica gel, and the crude product was subjected to column chromatography (petroleum ether (60-90) petroleum ether (60-90)/ethyl acetate 20 : 1-10 : 1) to obtain an off-white waxy solid (78.2 g, yield 96.2%).

### Preparation of Intermediate 1.3:

Compound 1.2 (78 g, 150.6 mmol, 1.0 eq), and dichloromethane (320 mL) were added into a 1,000 mL three-neck round-bottom reaction flask. The system was cooled to 0 to 10°C under nitrogen protection and magnetically stirred. Trifluoroacetic acid (171.8 g, 1,510 mmol) was added dropwise rapidly, then reacted at 15 to 25°C for 3 hours, then the reaction was quenched. The reaction solution was poured into a solution (with 780 mL of water) of sodium bicarbonate (164.5 g, 1,958 mmol) and quenched to maintain a pH of about 8. Dichloromethane (700 mL) was added and stirred,and liquid separation was conducted to obtain an organic phase. The organic phase was then washed with 500 mL of pure water and dried over anhydrous sodium sulfate, which was filtered and concentrated to obtain an off-white solid (59.5 g, yield 94.6%).

### Preparation of Intermediate 1.4:

Compound Boc-L-Val-OH (3.4 g, 15.8 mmol) and dichloromethane (60 mL) were added into a 250 mL single-neck reaction flask, and magnetically stirred. Then, N,N-diisopropylethylamine (2.0 g, 15.8 mmol), TBTU (5.1 g, 15.8 mmol), and compound 1.3 (6.0 g, 14.4 mmol, 1.0 eq) were added, then reacted at room temperature for about 1 hour, then the reaction was quenched. The reaction solution was concentrated under a reduced pressure, and the crude product was subjected to column chromatography (petroleum ether (60-90) petroleum ether (60-90)/ethyl acetate 50:1-10 : 1) to obtain an off-white solid (7.9 g, yield 96.2%).

### Preparation of Intermediate 1.5:

Compound 1.4 (7.9 g, 12.8 mmol, 1.0 eq), and dichloromethane (32 mL) were added into a 250 mL three-neck reaction flask. Under nitrogen protection and magnetic stirring, trifluoroacetic acid (14.6 g, 128.0 mmol) was added at 0 to 10°C, then reacted at room temperature for 3 hours, then the reaction was quenched. The reaction solution was concentrated under a reduced pressure, dichloromethane (70 mL) was added, a saturated aqueous solution of sodium bicarbonate was used for washing, and an aqueous phase was extracted with dichloromethane (30 mL). The organic phase was combined, then washed with 100 mL of pure water and the organic phase was dried over anhydrous sodium sulfate. It was filtered and concentrated to obtain an off-white solid (6.5 g, yield 98.2%).

### Preparation of Compound 1 Hydrochloride:

Compound 1.5 (6.5 g, 12.6 mmol, 1.0 eq), and ethyl acetate (32 mL) were added into a 250 mL single-neck reaction flask. Under nitrogen protection and magnetic stirring, a solution of hydrogen chloride in ethyl acetate (3 M, 5.0 mL, 15.0 mmol) was added at room temperature, and after the addition was completed, stirring was conducted for another 1 hour. A white colloid was precipitated out, and the reaction solution was evaporated to remove the solvent. Isopropanol (60 mL) was added to dissolve the obtained residue by heating, then stirred at room temperature for 50 minutes, filtered, and washed with isopropanol (7 mL) to obtain an off-white solid (3.2 g, yield 46.0%).

¹H NMR (400 MHz, CDCl₃) δ 8.34 (bs, 3H), 7.80 - 7.59 (m, 1H), 5.21 - 5.01 (m, 1H), 4.54 - 4.41 (m, 1H), 4.36 - 4.20 (m, 1H), 2.53 (t, *J =* 8.8 Hz, 1H), 2.45 - 2.10 (m, 3H), 2.12 (s, 3H),2.05 - 1.97 (m, 1H), 1.78 - 0.90 (m, 19H),1.16 (d, *J =* 6.9 Hz, 3H), 1.11 (d, *J =* 6.8 Hz, 3H), 1.05 (d, *J =* 3.3 Hz, 3H), 1.03 (d, *J =* 3.2 Hz, 3H),0.86 - 0.73 (m, 1H), 0.79 (s, 3H), 0.61 (s, 3H).

MS: m/z [M + H]⁺ 517. 6

### Example 2. Synthesis of Compound 2 Hydrochloride

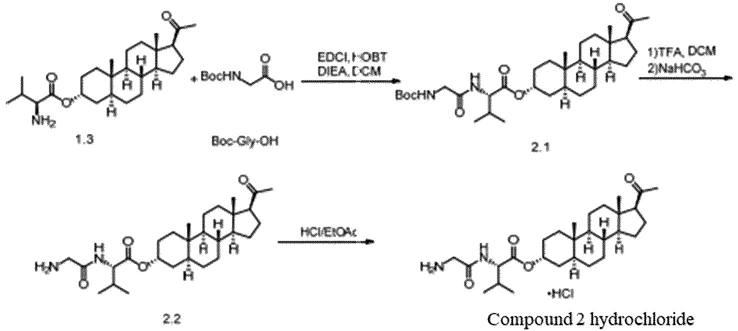

### Preparation of Intermediate 2.1:

Compound 1.3 (5.0 g, 12.0 mmol), Boc-Gly-OH (2.5 g, 14.3 mmol), and dichloromethane (50 mL) were added into a 250 mL single-neck reaction flask, and magnetically stirred. Then, N,N-diisopropylethylamine (3.1 g, 24.0 mmol), HOBT (342 mg, 2.4 mmol), and EDCI (2.8 g, 14.6 mmol) were added. The mixture was reacted at room temperature for 4 hours, and the reaction solution was washed with H₂O (50 mL), 1N HCl (50 mL), a saturated aqueous solution of NaHCO₃, and pure water. The reaction solution was concentrated under a reduced pressure, and the crude product was subjected to column chromatography (petroleum ether (60-90)/ethyl acetate 10 : 1-3 : 1) to obtain an off-white solid (5.7 g, yield 82.8%).

### Preparation of Intermediate 2.2:

Compound 1.4 (5.5 g, 9.63 mmol, 1.0 eq), and dichloromethane (22 mL) were added into a 250 mL three-neck reaction flask. Under nitrogen protection and magnetic stirring, trifluoroacetic acid (10.9 g, 95.7 mmol) was added at 0°C, then reacted at room temperature for 3 hours. The mixture was concentrated under a reduced pressure, and evaporated to remove the solvent. Dichloromethane (50 mL) was added, a saturated aqueous solution of sodium bicarbonate was used for washing, and an aqueous phase was extracted with dichloromethane (30 mL). The organic phase was combined, then washed with 50 mL of pure water and dried over anhydrous sodium sulfate. It was filtered and concentrated under a reduced pressure, and vacuum-dried using an oil pump to obtain an off-white solid (4.5 g, yield 99.0%).

### Preparation of Compound 2 Hydrochloride:

Compound 1.5 (4.5 g, 9.5 mmol, 1.0 eq), and ethyl acetate (27 mL) were added into a 250 mL single-neck reaction flask. Under nitrogen protection and magnetic stirring, a solution of hydrogen chloride in ethyl acetate (3 M, 3.8 mL, 11.4 mmol) was added at room temperature, and stirred for another 1 hour. The mixture was concentrated under a reduced pressure, and evaporated to remove the solvent. Acetonitrile (70 mL) was added and stirred at room temperature for 2 hours. After filtration, the solid was washed with acetonitrile (15 mL). An oil pump was used for vacuum drying at 40°C for 2 hours to obtain a white solid (3.5 g, yield 72.2%).

¹H NMR (400 MHz, CDCl₃) δ 8.28 (d, *J=* 8.5 Hz, 1H), 8.16 (bs, 3H), 5.14 - 5.03 (m, 1H), 4.55 (d, *J =* 4.1 Hz, 1H), 4.27 (d, *J =* 16.1 Hz, 1H), 4.09 (d, *J =* 16.0 Hz, 1H), 2.52 (t, *J =* 8.7 Hz, 1H), 2.40 - 2.07 (m, 2H), 2.11 (s, 3H), 2.05 - 1.96 (m, 1H), 1.82 - 1.08 (m, 18H), 1.08 -0.89 (m, 7H), 0.85 - 0.71 (m, 1H), 0.79 (s, 3H), 0.61 (s, 3H).

MS: m/z [M + H]⁺ 475.3.

### Example 3. Synthesis of Compound 3 Hydrochloride

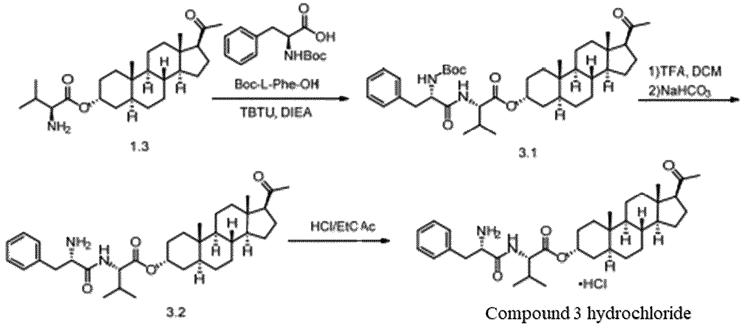

### Preparation of Intermediate 3.1:

Compound Boc-L-Phe-OH (0.96 g, 3.6 mmol), dichloromethane (12 mL), N,N-diisopropylethylamine (0.44 g, 3.6 mmol), TBTU (1.16 g, 3.6 mmol), and 1.3 (1.25 g, 3.0 mmol, 1.0 eq) were added into a 100 mL single-neck reaction flask, and magnetically stirred, then the reaction was continued for 40 minutes. The reaction solution was concentrated under a reduced pressure, and the crude product was subjected to column chromatography (petroleum ether (60-90)/ethyl acetate 10 : 1-4 : 1) to obtain a light yellow solid (1.5 g, yield 75.3%).

### Preparation of Intermediate 3.2:

Compound 3.1 (1.5 g, 2.3 mmol, 1.0 eq), and dichloromethane (7.5 mL) were added into a 100 mL three-neck reaction flask. Under nitrogen protection and magnetic stirring, trifluoroacetic acid (2.63 g, 23 mmol) was added at 0°C, then reacted at room temperature for 3 hours. The mixture was concentrated under a reduced pressure, and evaporated to remove the solvent. Dichloromethane (50 mL) was added, a saturated aqueous solution of sodium bicarbonate was used for washing, and an aqueous phase was extracted with dichloromethane (20 mL). The organic phases were combined, then washed with 50 mL of pure water and dried over anhydrous sodium sulfate. It was filtered and concentrated to obtain an off-white solid (1.1 g, yield 86.3%).

### Preparation of Compound 3 Hydrochloride:

Compound 3.2 (1.1 g, 1.95 mmol, 1.0 eq), and ethyl acetate (11 mL) were added into a 100 mL single-neck reaction flask. Under nitrogen protection and magnetic stirring, a solution of hydrogen chloride in ethyl acetate (3 M, 0.8 mL, 2.4 mmol) was added at room temperature, and stirred for another 1 hour. The mixture was concentrated to dryness under a reduced pressure, and crystallized with ethanol/water (2:1, 15 mL). After filtration, an oil pump was used for vacuum drying at 50°C for 4 hours to obtain a light yellow solid (620 mg, yield 52.9%).

¹H NMR (400 MHz, CDCl₃) δ 7.88 (d, *J =* 9.2 Hz, 1H), 7.38 - 7.17 (m, 5H), 5.15 - 5.05 (m, 1H), 4.57 (dd, *J =* 9.2, 4.6 Hz, 1H), 3.67 (dd, *J =* 9.5, 3.8 Hz, 1H), 3.30 (dd, *J =* 13.7, 3.8 Hz, 1H), 2.72 (dd, *J =* 13.7, 9.5 Hz, 1h), 2.53 (t, *J =* 8.8 Hz, 1H), 2.28 - 2.07 (m, 2H), 2.12 (s, 3H), 2.05 - 1.97 (m, 1H), 1.78 - 1.10 (m, 18h), 1.00 - 0.85 (m, 1H), 0.95 (d, *J* = 6.9 Hz, 3H), 0.92 (d, *J =* 6.8 Hz, 3H), 0.83 - 0.72 (m, 1H), 0.80 (s, 3H), 0.61 (s, 3H).

MS: [M + H]⁺ 565. 40.

### Example 4. Synthesis of Compound 4 Hydrochloride

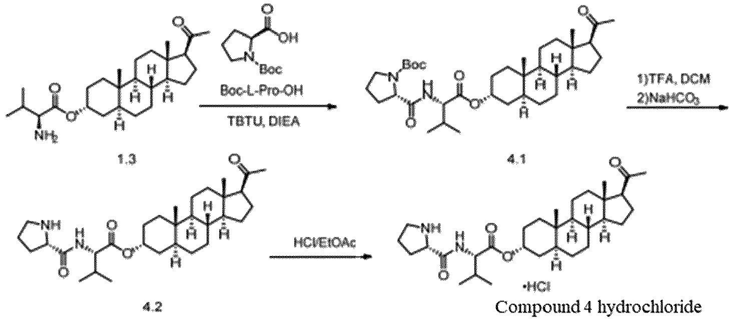

### Preparation of Intermediate 4.1:

Compound Boc-L-Pro-OH (0.77 g, 3.6 mmol), dichloromethane (12 mL), N,N-diisopropylethylamine (0.44 g, 3.6 mmol), TBTU (1.16 g, 3.6 mmol), and compound 1.3 (1.25 g, 3.0 mmol, 1.0 eq) were added into a 100 mL single-neck reaction flask, and magnetically stirred, then the reaction was continued at room temperature for 40 minutes. The reaction solution was concentrated under a reduced pressure, and the crude product was subjected to column chromatography (petroleum ether (60-90)/ethyl acetate 10 : 1-3 : 1) to obtain an off-white solid (1.6 g, yield 86.9%).

### Preparation of Intermediate 4.2:

Compound 4.1 (1.41 g, 2.3 mmol, 1.0 eq), and dichloromethane (7 mL) were added into a 100 mL three-neck reaction flask. Under nitrogen protection and magnetic stirring, trifluoroacetic acid (2.63 g, 23 mmol) was added at 0°C, then reacted at room temperature for 3 hours. The mixture was concentrated under a reduced pressure, and evaporated to remove the solvent. Dichloromethane (50 mL) was added, a saturated aqueous solution of sodium bicarbonate was used for washing, and an aqueous phase was extracted with dichloromethane (20 mL). The organic phases were combined, then washed with 50 mL of pure water and dried over anhydrous sodium sulfate. It was filtered and concentrated to obtain an off-white solid (1.0 g, yield 84.7%).

### Preparation of Compound 4 Hydrochloride:

Compound 3.2 (1.0 g, 1.94 mmol, 1.0 eq), and ethyl acetate (11 mL) were added into a 100 mL single-neck reaction flask. Under nitrogen protection and magnetic stirring, a solution of hydrogen chloride in ethyl acetate (3 M, 0.8 mL, 2.4 mmol) was added at room temperature, and after the addition is completed, stirring was continued for 17 hours. A white solid was precipitated out, filtered, and washed with ethyl acetate (3 mL). An oil pump was used for vacuum drying at 40°C for 3 hours to obtain a white solid (560 mg, yield 52.0%).

¹H NMR (400 MHz, CDCl₃) δ 11.34 (bs, 1H), 8.01 (d, *J =* 7.7 Hz, 1H), 7.69 - 7.34 (m, 1H), 5.16 - 5.03 (m, 1H), 5.01 - 4.89 (m, 1H), 4.43 (dd, *J =* 7.7, 4.6 Hz, 1H), 3.62 - 3.49 (m, 1H), 3.48 - 3.37 (m, 1H), 2.72 - 2.58 (m, 1H), 2.53 (t, *J =* 8.9 Hz, 1H), 2.38 - 2.26 (m, 1H), 2.25 - 2.09 (m, 3H), 2.12 (s, 3H), 2.07 - 1.96 (m, 2H), 1.88 - 1.10 (m, 18H),1.05 (d, *J =* 2.1 Hz, 3H), 1.09 - 0.88 (m, 1H), 1.03 (d, *J =* 2.2 Hz, 3H), 0.85 - 0.72 (m, 1H), 0.80 (s, 3H), 0.61 (s, 3H).

MS: [M + H]⁺ 515. 4.

### Example 5. Synthesis of Compound 5 Hydrochloride

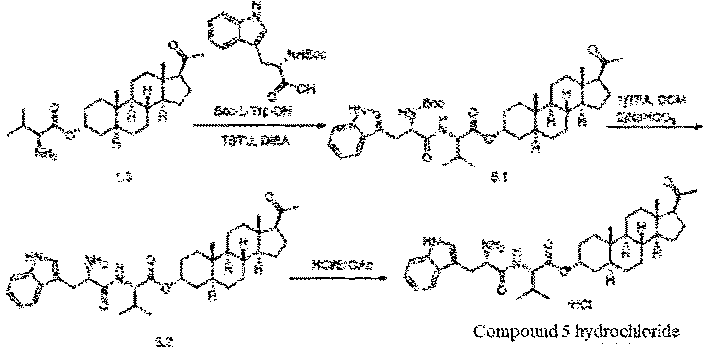

### Preparation of Intermediate 5.1:

Compound Boc-L-Trp-OH (1.10 g, 3.6 mmol), dichloromethane (12 mL), N,N-diisopropylethylamine (0.44 g, 3.6 mmol), and TBTU (1.16 g, 3.6 mmol) were added into a 100 mL single-neck reaction flask, and stirred at room temperature for 6 minutes. Afterwards, compound 1.3 (1.25 g, 3.0 mmol, 1.0 eq) was added, and magnetically stirred, then the reaction was continued at room temperature for 40 minutes. The reaction solution was concentrated under a reduced pressure, and the crude product was subjected to column chromatography (petroleum ether (60-90)/ethyl acetate 10 : 1-3 : 1) to obtain an off-white solid (1.7 g, yield 80.7%).

### Preparation of Intermediate 5.2:

Compound 5.1 (1.58 g, 2.3 mmol, 1.0 eq), and dichloromethane (8 mL) were added into a 100 mL three-neck reaction flask. Under nitrogen protection and magnetic stirring, trifluoroacetic acid (2.63 g, 23 mmol) was added at 0°C, then reacted at room temperature for 3 hours. The mixture was concentrated under a reduced pressure, and evaporated to remove the solvent. Dichloromethane (50 mL) was added, a saturated aqueous solution of sodium bicarbonate was used for washing, and an aqueous phase was extracted with dichloromethane (20 mL). The organic phases were combined, then washed with 50 mL of pure water and dried over anhydrous sodium sulfate. It was filtered and concentrated to obtain an off-white solid (1.20 g, yield 88.5%).

### Preparation of Compound 5 Hydrochloride:

Compound 3.2 (1.15 g, 1.95 mmol, 1.0 eq), and ethyl acetate (11 mL) were added into a 100 mL single-neck reaction flask. Under nitrogen protection and magnetic stirring, a solution of hydrogen chloride in ethyl acetate (3 M, 0.8 mL, 2.4 mmol) was added at room temperature, and after the addition is completed, stirring was continued for 1 hour. The mixture was concentrated to dryness under a reduced pressure, and stirred and pulped with petroleum ether/ethyl acetate (5 : 1, 30 mL). After filtration, an oil pump was used for vacuum drying at 40°C for 3 hours to obtain an off-white solid (690 mg, yield 56.7%).

¹H NMR (400 MHz, CDCl3)δ 9.35 (s, 1H), 7.96 (bs, 3H), 7.74 - 7.41 (m, 2H),7.38 - 7.16 (m, 2H), 7.13 - 6.74 (m, 2H), 5.13 - 4.96 (m, 1H), 4.60 - 4.20 (m, 2H), 3.54 - 3.20 (m, 2H), 2.35 - 2.22 (m, 1H), 2.17 - 1.96 (m, 2H), 2.05 (s, 3H), 1.92 - 1.80 (m, 1H), 1.79 - 0.99 (m, 18H), 1.00 - 0.63 (m, 8H), 0.75 (s, 3H), 0.53 (s, 3H).

MS:[M+H]+ 604.4.

### Example 6. Synthesis of Compound 6 Hydrochloride

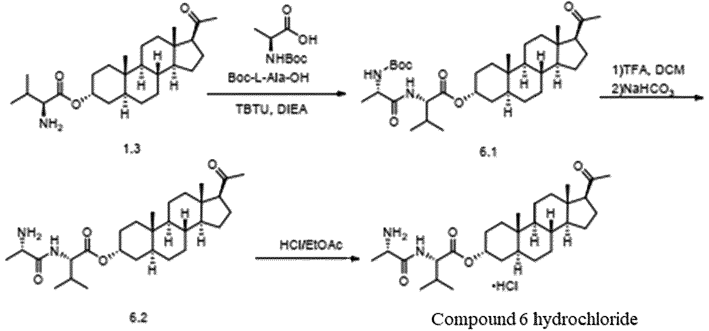

### Preparation of Intermediate 6.1:

Compound 1.3 (6.26 g, 15.0 mmol), Boc-L-Ala-OH (3.41 g, 18 mmol), dichloromethane (60 mL), triethylamine (3.04 g, 30 mmol), HOBT (0.41 g, 3 mmol), and EDCI (3.45 g, 18 mmol) were added into a 250 mL single-neck reaction flask, and magnetically stirred. The mixture was reacted at room temperature for 4 hours, and the reaction solution was washed with H₂O (50 mL), 1N HCl (50 mL), and a saturated aqueous solution of NaHCO₃. The reaction solution was concentrated under a reduced pressure, and the crude product was subjected to column chromatography (petroleum ether (60-90)/ethyl acetate 10 : 1-3 : 1) to obtain an off-white solid (8.20 g, yield 92.9%).

### Preparation of Intermediate 6.2:

Compound 6.1 (7.9 g, 13 mmol, 1.0 eq), and dichloromethane (40 mL) were added into a 100 mL three-neck reaction flask. Under nitrogen protection and magnetic stirring, trifluoroacetic acid (14.8 g, 130 mmol) was added at 0°C, then reacted at room temperature for 3 hours. The mixture was concentrated under a reduced pressure, and evaporated to remove the solvent. Dichloromethane (100 mL) was added, a saturated aqueous solution of sodium bicarbonate was used for washing, and an aqueous phase was extracted with dichloromethane (50 mL). The organic phases were combined, then washed with 100 mL of pure water and dried over anhydrous sodium sulfate. It was filtered and concentrated under a reduced pressure, and vacuum-dried using an oil pump to obtain an off-white solid (6.25 g, yield 95.3%).

### Preparation of Compound 6 Hydrochloride:

Compound 6.2 (6.0 g, 12.3 mmol, 1.0 eq), and ethyl acetate (60 mL) were added into a 100 mL single-neck reaction flask. Under nitrogen protection and magnetic stirring, a solution of hydrogen chloride in ethyl acetate (3 M, 5 mL, 15 mmol) was added at room temperature, and after the addition is completed, stirring was continued for 6 hours. A white solid was precipitated out, filtered, and washed with ethyl acetate (15 mL). An oil pump was used for vacuum drying at 40°C for 3 hours to obtain a white solid (4.7 g, yield 72.9%).

¹H NMR (400 MHz, CDCl₃) δ 8.33 (bs, 3H), 7.96 - 7.67 (m, 1H), 5.20 - 5.00 (m, 1H), 4.76 - 4.36 (m, 2H), 2.53 (t, *J =* 8.6 Hz, 1H), 2.39 - 2.07 (m, 2H), 2.11 (s, 3H), 2.05 - 1.96 (m, 1H), 1.80 - 0.89 (m, 28H), 0.88 - 0.71 (m, 1H), 0.79 (s, 3H), 0.61 (s, 3h).

MS: [M+H]⁺ 489. 4.

### Example 7. Synthesis of Compound 7 Hydrochloride

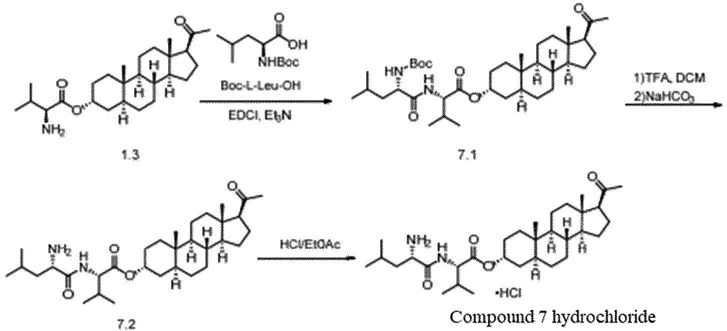

### Preparation of intermediate 7.1:

Compound 1.3 (2.09 g, 5.0 mmol), Boc-L-Leu-OH (1.50 g, 6.0 mmol), dichloromethane (20 mL), triethylamine (0.76 g, 7.5 mmol), HOBT (0.14 g, 1 mmol), and EDCI (1.15 g, 6 mmol) were added into a 250 mL single-neck reaction flask, and magnetically stirred. The mixture was reacted at room temperature for 4 hours, and the reaction solution was washed with H₂O (50 mL), 1N HCl (50 mL), and a saturated aqueous solution of NaHCO₃. The reaction solution was concentrated under a reduced pressure, and the crude product was subjected to column chromatography (petroleum ether (60-90)/ethyl acetate 10 : 1-3 : 1) to obtain an off-white solid (3.0 g, yield 95.0%).

### Preparation of intermediate 7.2:

Compound 7.1 (3.0 g, 4.7 mmol, 1.0 eq), and dichloromethane (12 mL) were added into a 100 mL three-neck reaction flask. Under nitrogen protection and magnetic stirring, trifluoroacetic acid (5.42 g, 47 mmol) was added at 0°C, stirred for 20 minutes, then reacted at room temperature for 3 hours. The mixture was concentrated under a reduced pressure, and evaporated to remove the solvent. Dichloromethane (30 mL) was added, a saturated aqueous solution of sodium bicarbonate was used for washing, and an aqueous phase was extracted with dichloromethane (10 mL). The organic phases were combined, then washed with 30 mL of pure water and dried with anhydrous sodium sulfate. It was filtered and concentrated under a reduced pressure, and vacuum-dried using an oil pump to obtain an off-white solid (1.30 g, yield 51.5%).

### Preparation of Compound 7 Hydrochloride:

Compound 7.2 (1.30 g, 2.5 mmol, 1.0 eq), and ethyl acetate (13 mL) were added into a 50 mL single-neck reaction flask. Under nitrogen protection and magnetic stirring, a solution of hydrogen chloride in ethyl acetate (3 M, 5 mL, 15 mmol) was added at room temperature, and after the addition is completed, stirring was continued for 6 hours. A white solid was precipitated out, filtered, and washed with ethyl acetate (15 mL). An oil pump was used for vacuum drying at 40°C for 3 hours to obtain a white solid (0.9 g, yield 64.8%).

¹H NMR (400 MHz, CDCl₃) δ 8.40 (s, 3H), 7.55 (d, *J =* 7.9 Hz, 1H), 5.17 - 5.05 (m, 1H), 4.57 - 4.44 (m, 1H), 4.36 - 4.21 (m, 1H), 2.52 (t, *J =* 8.8 Hz, 1H), 2.35 - 1.97 (m, 3H), 2.11 (s, 3H), 1.96 - 0.73 (m, 35H), 0.80 (s, 3H), 0.61 (s, 3H).

MS: m/z [M+H]⁺ 531. 4.

### Example 8. Combination of Compound 8 Hydrochloride

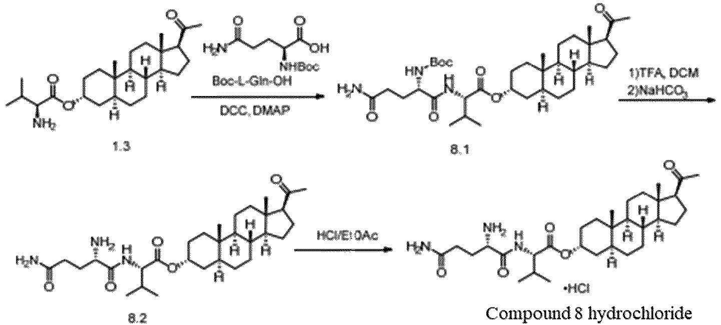

### Preparation of Intermediate 8.1:

Compound 1.3 (5.00 g, 11.98 mmol, 1.0 eq), Boc-L-Gln-OH (3.54 g, 14.38 mmol), DMAP (0.15 g, 1.20 mmol), and dichloromethane (40 mL) were added into a 250 mL three-neck reaction flask. Under nitrogen protection and magnetic stirring, the mixture was cooled in an ice-water bath. A solution of DCC (2.97 g, 14.38 mmol) in dichloromethane (50 mL) was added, and after the addition is completed, reacted at room temperature for 3 hours. The reaction solution was washed with 1N HCl (50 mL), and a saturated aqueous solution of NaHCO₃. The reaction solution was dried over anhydrous Na₂SO₄, and filtered. It was concentrated under a reduced pressure, and vacuum-dried using an oil pump to obtain a white solid (4.42 g, yield 57.2%).

### Preparation of Intermediate 8.2:

Compound 8.1 (4.42 g, 6.84 mmol, 1.0 eq), and dichloromethane (22 mL) were added into a 100 mL three-neck reaction flask. Under nitrogen protection and magnetic stirring, trifluoroacetic acid (7.80 g, 68.43 mmol) was added at 0°C, then reacted at room temperature for 3 hours. The mixture was concentrated under a reduced pressure, dichloromethane (100 mL) was added, a saturated aqueous solution of sodium bicarbonate was used for washing, and an aqueous phase was extracted with dichloromethane (50 mL). The organic phases were combined, then washed with 100 mL of pure water and dried over anhydrous sodium sulfate. It was filtered and concentrated to obtain an off-white solid (3.0 g, yield 80.3%).

### Preparation of Compound 8 Hydrochloride:

Compound 8.2 (1.10 g, 2.02 mmol, 1.0 eq), and dichloromethane (12 mL) were added into a 50 mL single-neck reaction flask. Under nitrogen protection and magnetic stirring, a solution of hydrogen chloride in ethyl acetate (3 M, 0.8 mL, 2.4 mmol) was added at room temperature, and after the addition is completed, stirring was continued for 1 hour. The mixture was concentrated to remove dichloromethane under a reduced pressure, and stirred and pulped with added methyl tert-butyl ether/isopropanol (4 : 1, 20 mL) for 2 hours. After filtration, methyl tert-butyl ether/isopropanol (4 : 1, 5 mL) was pre-cooled for washing. An oil pump was used for vacuum drying at 40°C for 3 hours to obtain a white solid (0.85 g, yield 72.4%).

¹H NMR (400 MHz, CDCl₃) δ 8.67 - 8.59 (m, 1H), 8.36 (bs, 3H), 7.63 (bs, 1H), 6.92 (bs, 1H), 5.30 - 4.96 (m, 1H), 4.80 - 4.31 (m, 2H), 2.80 - 1.94 (m, 6H), 2.52 (t, *J =* 8.8 Hz, 1H), 2.11 (s, 3H), 1.89 - 0.70 (m, 27H), 0.79 (s, 3H), 0.60 (s, 3H).

MS: m/z [M+H]⁺ 546. 4

### Example 9. Synthesis of Compound 9 Hydrochloride

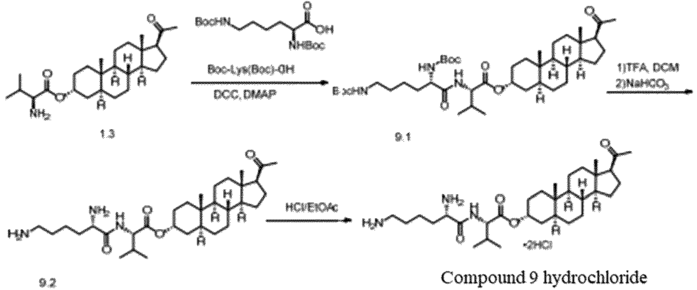

### Preparation of Intermediate 9.1:

Compound 1.3 (9.60 g, 23.1 mmol, 1.0 eq), Boc-Lys(Boc)-OH (8.00 g, 23.1 mmol), DMAP (0.28 g, 2.3 mmol), and dichloromethane (100 mL) were added into a 250 mL three-neck reaction flask. Under nitrogen protection and magnetic stirring, the mixture was cooled in an ice-water bath. A solution of DCC (5.20 g, 25.4 mmol) in dichloromethane (20 mL) was added dropwise while inner temperature was controlled at 3 to 5°C, and after the addition is completed, reacted at room temperature for 17 hours. The reaction solution was concentrated under a reduced pressure, and the crude product was subjected to column chromatography (petroleum ether (60-90)/ethyl acetate 10 : 1-3 : 1) to obtain a white foam solid (10.0 g, yield 61.0%).

### Preparation of Intermediate 9.2:

Compound 9.1 (10.5 g, 14.1 mmol, 1.0 eq), and dichloromethane (50 mL) were added into a 100 mL three-neck reaction flask. Under nitrogen protection and magnetic stirring, trifluoroacetic acid (16.0 g, 141.0 mmol) was added at 0°C, then reacted at room temperature for 3 hours. The reaction solution was added dropwise to a solution of NaHCO₃ (28 g) in H₂O (100 mL) with stirring, and dichloromethane (150 mL) was added. The liquid-separation was conducted, and an aqueous phase was extracted with dichloromethane (100 mL). The organic phases were combined, then washed with 100 mL of pure water and dried over anhydrous sodium sulfate. It was filtered and concentrated to obtain a light yellow foam solid (7.1 g, yield 92%).

### Preparation of Compound 9 Hydrochloride:

Compound 9.2 (7.0 g, 12.8 mmol, 1.0 eq), and ethyl acetate (20 mL) were added into a 50 mL single-neck reaction flask. Under nitrogen protection and magnetic stirring, a solution of hydrogen chloride in ethyl acetate (3 M, 5.1 mL, 15.3 mmol) was added at room temperature, and after the addition is completed, stirring was continued for 1 hour. The mixture was concentrated to remove ethyl acetate under a reduced pressure, and stirred and pulped with added methyl tert-butyl ether (100 mL) for 2 hours. After filtration, methyl tert-butyl ether (20 mL) was used for washing. An oil pump was used for vacuum drying at 45°C for 3 hours to obtain a white solid (7.2 g, yield 90.7%).

¹H NMR (400 MHz, CDCl₃) δ 8.30 (bs, 3H), 8.13 - 7.98 (m, 1H), 7.89 (bs, 3H), 5.20 - 4.96 (m, 1H), 4.60 - 4.19 (m, 2H), 3.17 - 2.85 (m, 2H), 2.51 (t, *J* = 9.1 Hz, 1H), 2.46 - 0.70 (m, 35H), 2.11 (s, 3H), 0.79 (s, 3H), 0.61 (s, 3H).

MS: m/z [M+H]⁺ 546. 8.

### Example 10. Synthesis of Compound 10 Hydrochloride

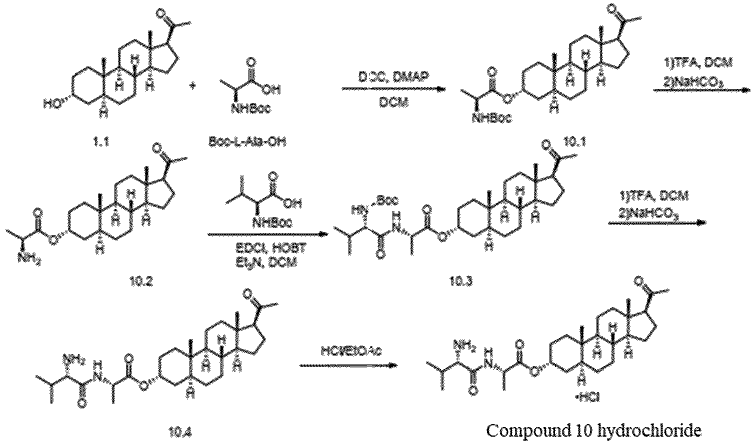

### Preparation of Intermediate 10.1:

Compound 1.1 (20.0 g, 62.8 mmol, 1.0 eq), Boc-L-Ala-OH (14.3 g, 75.4 mmol), DMAP (0.8 g, 6.3 mmol), and dichloromethane (150 mL) were added into a 1,000 mL three-neck reaction flask. Under nitrogen protection and magnetic stirring, a solution of DCC (15.5 g, 75.4 mmol) in dichloromethane (50 mL) was added at 0°C, then reacted at room temperature for 6 hours. The reaction solution was filtered, and the cake was washed with dichloromethane (100 mL). The filtrate was concentrated, and the crude product was subjected to column chromatography (petroleum ether (60-90)/ethyl acetate 10 : 1-4 : 1) to obtain a white solid (27.3 g, yield 88.7%).

### Preparation of Intermediate 10.2:

Compound 10.1 (27.0 g, 55.1 mmol, 1.0 eq), and dichloromethane (135 mL) were added into a 500 mL three-neck reaction flask. Under nitrogen protection and magnetic stirring, trifluoroacetic acid (62.8 g, 551 mmol) was added at 0°C, then reacted at room temperature for 3 hours. The mixture was concentrated under a reduced pressure, and evaporated to remove the solvent. Dichloromethane (300 mL) was added, and a saturated aqueous solution of sodium bicarbonate was used for washing. Dichloromethane (150 mL) was used for extracting the aqueous phase. The organic phases were combined, then washed with 300 mL of pure water and dried over anhydrous sodium sulfate. It was filtered and concentrated to obtain an off-white solid (20.9 g, yield 97.3%).

### Preparation of Intermediate 10.3:

Compound 10.2 (5.0 g, 12.8 mmol), Boc-L-Val-OH (3.3 g, 15.4 mmol), dichloromethane (50 mL), triethylamine (1.6 g, 15.4 mmol), HOBT (0.7 g, 5.1 mmol), and EDCI (3.0 g, 15.4 mmol) were added into a 100 mL single-neck reaction flask, and magnetically stirred. The mixture was reacted at room temperature for 4 hours, and the reaction solution was washed with H₂O (50 mL), 1N HCl (50 mL), and a saturated aqueous solution of NaHCO₃. The reaction solution was concentrated under a reduced pressure, and the crude product was subjected to column chromatography (petroleum ether (60-90)/ethyl acetate 10 : 1-5 : 1) to obtain a white solid (7.2 g, yield 95.6%).

### Preparation of Intermediate 10.4:

Compound 10.3 (6.0 g, 10.2 mmol, 1.0 eq), and dichloromethane (30 mL) were added into a 100 mL three-neck reaction flask. Under nitrogen protection and magnetic stirring, trifluoroacetic acid (11.6 g, 102 mmol) was added at 0°C, then reacted at room temperature for 3 hours. The mixture was concentrated under a reduced pressure, dichloromethane (100 mL) was added, a saturated aqueous solution of sodium bicarbonate was used for washing, and an aqueous phase was extracted with dichloromethane (50 mL). The organic phases were combined, then washed with 100 mL of pure water and dried with anhydrous sodium sulfate. It was filtered and concentrated to obtain an off-white solid (4.6 g, yield 92.3%).

### Preparation of Compound 10 Hydrochloride:

Compound 10.3 (4.6 g, 9.4 mmol, 1.0 eq), and ethyl acetate (50 mL) were added into a 250 mL single-neck reaction flask. Under nitrogen protection and magnetic stirring, a solution of hydrogen chloride in ethyl acetate (3 M, 3.8 mL, 11.4 mmol) was added at room temperature, and after the addition is completed, stirring was continued for 1 hour. A white solid was precipitated out, filtered, and washed with ethyl acetate (20 mL). An oil pump was used for vacuum drying at 40°C for 3 hours to obtain a white solid (4.5 g, yield 86.4%).

¹H NMR (400 MHz, CDCl₃) δ 8.28 (bs, 3H), 8.11 (d, *J =* 6.2 Hz, 1H), 5.06 (d, *J* = 3.4 Hz, 1H), 4.51 (p, *J =* 7.0 Hz, 1H), 4.23 (d, *J =* 5.6 Hz, 1H), 2.53 (t, *J =* 8.8 Hz, 1H), 2.40 (q, *J* = 6.6 Hz, 1H), 2.12 (s, 3H), 2.25-2.09 (m,2H), 2.08-1.06 (m, 18H),1.54-1.50 (m, 3H), 1.22-1.12 (m, 6H)1.05-0.73 (m, 2H) , 0.79 (s, 3H), 0.61 (s, 3H).

MS: m/z [M+H]⁺ 489. 4.

### Example 11. Synthesis of Compound 11 Hydrochloride

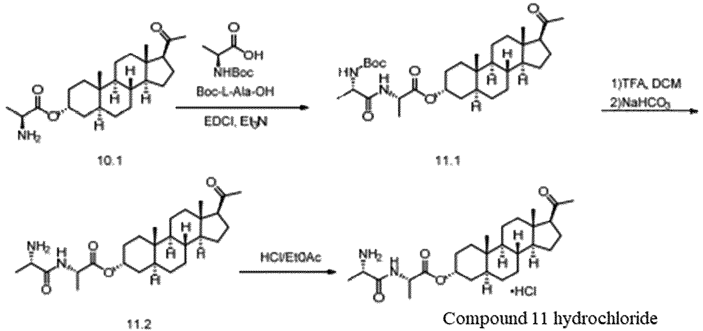

### Preparation of Intermediate 11.1:

Compound 10.1 (5.0 g, 12.8 mmol), Boc-L-Ala-OH (2.9 g, 15.4 mmol), dichloromethane (50 mL), triethylamine (1.6 g, 15.4 mmol), HOBT (0.7 g, 5.1 mmol), and EDCI (3.0 g, 15.4 mmol) were added into a 250 mL single-neck reaction flask, and magnetically stirred. The mixture was reacted at room temperature for 4 hours, and the reaction solution was washed with H₂O (50 mL), 1N HCl (50 mL), and a saturated aqueous solution of NaHCO₃. The reaction solution was concentrated under a reduced pressure, and the crude product was subjected to column chromatography (petroleum ether (60-90)/ethyl acetate 10 : 1-3 : 1) to obtain a white solid (6.7 g, yield 93.5%).

### Preparation of Intermediate 11.2:

Compound 11.1 (5.8 g, 10.3 mmol, 1.0 eq), and dichloromethane (30 mL) were added into a 100 mL three-neck reaction flask. Under nitrogen protection and magnetic stirring, trifluoroacetic acid (11.8 g, 103 mmol) was added at 0°C, then reacted at room temperature for 3 hours. The mixture was concentrated under a reduced pressure, dichloromethane (100 mL) was added, a saturated aqueous solution of sodium bicarbonate was used for washing, and an aqueous phase was extracted with dichloromethane (50 mL). The organic phases were combined, then washed with 100 mL of pure water and dried over anhydrous sodium sulfate. It was filtered and concentrated to obtain an off-white solid (4.5 g, yield 94.4%).

### Preparation of Compound 11 Hydrochloride:

Compound 11.2 (4.5 g, 9.8 mmol, 1.0 eq), and ethyl acetate (50 mL) were added into a 250 mL single-neck reaction flask. Under nitrogen protection and magnetic stirring, a solution of hydrogen chloride in ethyl acetate (3 M, 3.9 mL, 11.7 mmol) was added at room temperature, and after the addition is completed, stirring was continued for 1 hour. A white solid was precipitated out, filtered, and washed with ethyl acetate (20 mL). An oil pump was used for vacuum drying at 40°C for 3 hours to obtain a white solid (4.2 g, yield 86.5%).

¹H NMR (400 MHz, CDCl₃) δ 8.14 (m, 4H), 5.05 (m, 1H), 4.47 (dt, *J =* 21.0, 6.9 Hz, 1H), 2.53 (t, *J* = 8.8 Hz, 1H), 2.12 (s, 3H), 2.24-1.97 (m, 2H), 1.94 - 1.07 (m, 19H), 1.69-1.65 (m, 3H), 1.52-1.47 (m, 3H), 0.79 (s,3H), 1.05-0.73 (m, 2H), 0.61(s, 3H).

MS: m/z [M+H]⁺ 461. 3.

### Example 12. Synthesis of Compound 12 Hydrochloride

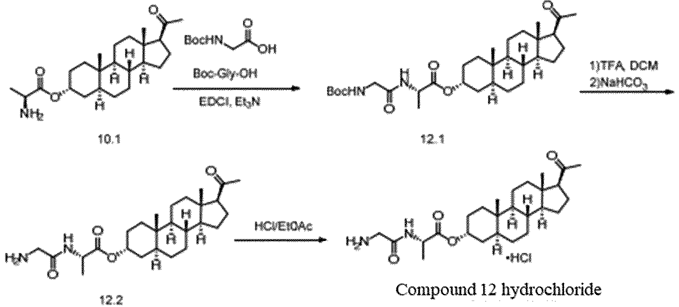

### Preparation of intermediate 12.1:

Compound 10.1 (5.0 g, 12.8 mmol), Boc-Gly-OH (2.7 g, 15.4 mmol), dichloromethane (50 mL), triethylamine (1.6 g, 15.4 mmol), HOBT (0.7 g, 5.1 mmol), and EDCI (3.0 g, 15.4 mmol) were added into a 250 mL single-neck reaction flask. The mixture was reacted at room temperature for 4 hours, and the reaction solution was washed with H₂O (50 mL), 1N HCl (50 mL), and a saturated aqueous solution of NaHCO₃. The reaction solution was concentrated under a reduced pressure, and the crude product was subjected to column chromatography (petroleum ether (60-90)/ethyl acetate 10 : 1-3 : 1) to obtain a white solid (6.5 g, yield 93.1%).

### Preparation of intermediate 12.2:

Compound 11.1 (5.8 g, 10.6 mmol, 1.0 eq), and dichloromethane (30 mL) were added into a 100 mL three-neck reaction flask. Under nitrogen protection and magnetic stirring, trifluoroacetic acid (12.1 g, 106 mmol) was added at 0°C, then reacted at room temperature for 3 hours. The mixture was concentrated under a reduced pressure, dichloromethane (100 mL) was added, a saturated aqueous solution of sodium bicarbonate was used for washing, and an aqueous phase was extracted with dichloromethane (50 mL). The organic phases were combined, then washed with 100 mL of pure water and dried over anhydrous sodium sulfate. It was filtered and concentrated to obtain a white solid (4.5 g, yield 95.0%).

### Preparation of Compound 12 Hydrochloride:

Compound 11.2 (4.5 g, 10.1 mmol, 1.0 eq), and ethyl acetate (50 mL) were added into a 250 mL single-neck reaction flask. Under nitrogen protection and magnetic stirring, a solution of hydrogen chloride in ethyl acetate (3 M, 4.0 mL, 12.0 mmol) was added at room temperature, and after the addition is completed, stirring was continued for 1 hour. A white solid was precipitated out, filtered, and washed with ethyl acetate (20 mL). An oil pump was used for vacuum drying at 40°C for 3 hours to obtain a white solid (4.6 g, yield 94.5%).

¹H NMR (400 MHz, CDCl₃) δ 8.49 (d, *J* = 7.2 Hz, 1H), 8.08 (bs, 3H), 5.06 (m, 1H), 4.55 (p, *J* = 7.1 Hz, 1H), 4.35-3.94 (m, 2H), 2.51 (t, *J* = 8.7 Hz, 1H), 2.11 (s, 3H), 2.17-1.95 (m, 2H) 1.94 - 0.88 (m, 19H), 1.51-1.45(m,3H),0.87-0.72 (m, 1H), 0.79 (s, 3H), 0.61 (s, 3H).

MS: m/z [M+H]⁺ 447. 3.

### Example 13. Synthesis of Compound 13 Hydrochloride

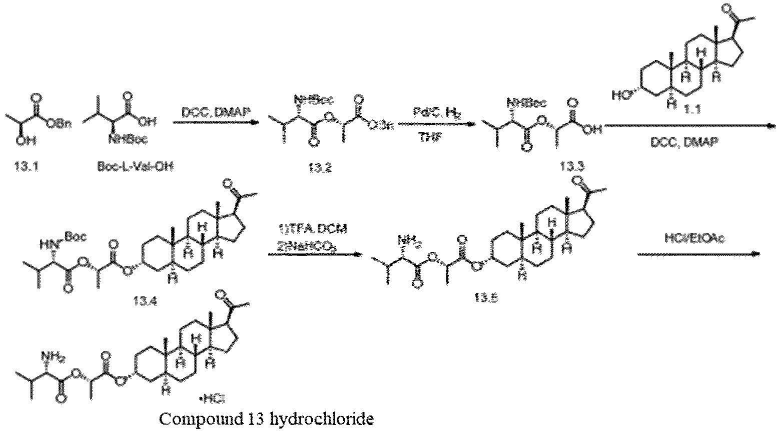

### Preparation of Intermediate 13.2:

Compound 13.1 (10.0 g, 55.5 mmol, 1.0 eq), Boc-L-Val-OH (12.0 g, 55.5 mmol), DMAP (1.4 g, 11.5 mmol), and dichloromethane (100 mL) were added into a 500 mL three-neck reaction flask. Under nitrogen protection and magnetic stirring, a solution of DCC (13.7 g, 66.6 mmol) in dichloromethane (50 mL) was added to a at 0°C, then reacted at room temperature for 6 hours. The reaction solution was filtered, and the cake was washed with dichloromethane (100 mL). The filtrate was concentrated, and the crude product was subjected to column chromatography (petroleum ether (60-90)/ethyl acetate 20 : 1-6:1) to obtain a colorless oil (20.5 g, yield 97.3%).

### Preparation of Intermediate 13.3:

Compound 13.2 (20.0 g, 52.7 mmol), and THF (300 mL) were added into a 1,000 mL single-neck reaction flask. 10% Pd/C (2 g) was added after nitrogen replacement. After hydrogen replacement, it was hydrogenated at room temperature for 6 hours under normal pressure. After nitrogen replacement, it was filtered and washed with THF (50 mL). The filtrate was concentrated, evaporated to remove the solvent, and vacuum-dried using an oil pump at room temperature to obtain a white solid (14.9 g, yield 97.7%).

### Preparation of Intermediate 13.4:

Compound 1.1 (5.0 g, 15.7 mmol, 1.0 eq), Compound 13.3 (5.4 g, 18.7 mmol), DMAP (0.2 g, 1.6 mmol), and dichloromethane (50 mL) were added into a 250 mL three-neck reaction flask. Under nitrogen protection and magnetic stirring, a solution of DCC (3.9 g, 18.9 mmol) in dichloromethane (15 mL) was added at 0°C, then reacted at room temperature for 2 hours. The reaction solution was filtered, and the cake was washed with dichloromethane (20 mL). The filtrate was concentrated, and the crude product was subjected to column chromatography (petroleum ether (60-90)/ethyl acetate 20 : 1-3 : 1) to obtain a white solid (8.7 g, yield 93.9%).

### Preparation of Intermediate 13.5:

Compound 13.4 (8.0 g, 13.6 mmol, 1.0 eq), and dichloromethane (40 mL) were added into a 250 mL three-neck reaction flask. Under nitrogen protection, trifluoroacetic acid (15.5 g, 136 mmol) was added at 0°C, then reacted at room temperature for 3 hours. The mixture was concentrated under a reduced pressure, dichloromethane (150 mL) was added, a saturated aqueous solution of sodium bicarbonate was used for washing, and an aqueous phase was extracted with dichloromethane (50 mL). The organic phases were combined, then washed with 100 mL of pure water and dried with anhydrous sodium sulfate. It was filtered and concentrated to obtain a white solid (6.5 g, yield 97.8%).

### Preparation of Compound 13 Hydrochloride:

Compound 13.4 (6.0 g, 12.3 mmol, 1.0 eq), and ethyl acetate (60 mL) were added into a 250 mL single-neck reaction flask. Under nitrogen protection and magnetic stirring, a solution of hydrogen chloride in ethyl acetate (3 M, 4.9 mL, 14.7 mmol) was added at room temperature, and stirring was continued for 1 hour. A white solid was precipitated out, filtered, and washed with ethyl acetate (20 mL). An oil pump was used for vacuum drying at 40°C for 3 hours to obtain a white solid (5.2 g, yield 80.6%).

¹H NMR (400 MHz, CDCl₃) δ 8.76 (s, 3H), 5.25 (q, *J* = 7.0 Hz, 1H), 5.08 (q, *J* = 2.8 Hz, 1H), 4.02 (d, *J =* 3.9 Hz, 1H), 2.62 - 2.45 (m, 2H), 2.12 (s, 3H), 2.01 (dt, *J =* 11.9, 3.3 Hz, 1H), 1.78 - 1.63 (m, 5H), 1.63 - 1.58 (m, 1H), 1.56 (d, *J =* 7.1 Hz, 4H), 1.50 (t, *J =* 5.1 Hz, 3H), 1.45 -1.32 (m, 3H), 1.28 (dd, *J =* 13.2, 3.9 Hz, 2H), 1.22 (dd, *J =* 7.0, 2.7 Hz, 7H), 1.20 - 1.09 (m, 4H), 0.96 (dd, *J* = 12.3, 4.6 Hz, 1H), 0.79 (s, 3H), 0.61 (s, 3H).

MS: m/z [M+H]⁺490. 3.

### Example 14. Synthesis of Compound 14 Hydrochloride

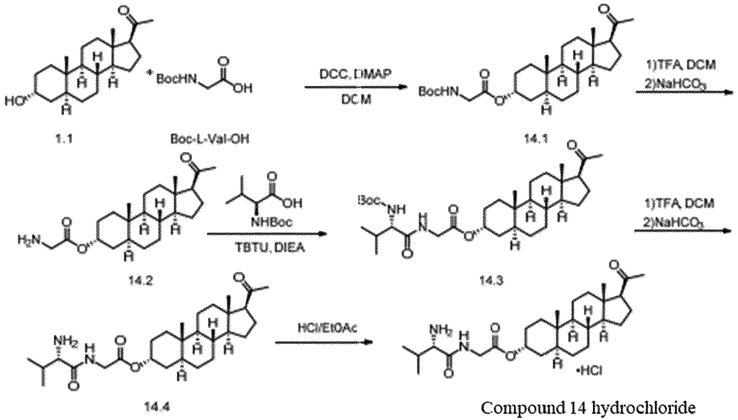

### Preparation of intermediate 14.1:

Compound 1.1 (50.0 g, 157.0 mmol, 1.0 eq), Boc-Gly-OH (33.0 g, 188.2 mmol), DMAP (1.9 g, 15.5 mmol), and dichloromethane (500 mL) were added into a 1,000 mL three-neck reaction flask. Under nitrogen protection and magnetic stirring, a solution of DCC (38.9 g, 188.5 mmol) in dichloromethane (80 mL) was added at 0°C, then reacted at room temperature for 2 hours, then the reaction was stopped. The reaction solution was filtered, and the cake was washed with dichloromethane (100 mL). The filtrate was concentrated, and the crude product was subjected to column chromatography (petroleum ether (60-90)/ethyl acetate 20 : 1-4 : 1) to obtain a white solid (71.8 g, yield 95.8%).

### Preparation of Intermediate 14.2:

Compound 1.2 (30 g, 63.1 mmol, 1.0 eq), and dichloromethane (1200 mL) were added into a 1,000 mL three-neck reaction flask. Under nitrogen protection and magnetic stirring, trifluoroacetic acid (71.9 g, 631 mmol) was added at 0°C, then reacted at room temperature for 3 hours. The mixture was concentrated under a reduced pressure, and evaporated to remove the solvent. Dichloromethane (500 mL) and isopropanol (50 mL) were added, and a saturated aqueous solution of sodium bicarbonate was used for washing. The organic phase was then washed with 500 mL of pure water and dried with anhydrous sodium sulfate. It was filtered and concentrated to obtain an off-white solid (21.7 g, yield 91.6%).

### Preparation of Intermediate 14.3:

Compound Boc-L-Val-OH (3.4 g, 15.8 mmol), dichloromethane (60 mL), N,N-diisopropylethylamine (2.0 g, 15.8 mmol), TBTU (5.1 g, 15.8 mmol), and compound 14.2 (5.4 g, 14.4 mmol, 1.0 eq) were added into a 250 mL single-neck reaction flask, then reacted at room temperature for 40 minutes. The reaction solution was concentrated under a reduced pressure, and the crude product was subjected to column chromatography (petroleum ether (60-90)/ethyl acetate 20 : 1-5 : 1) to obtain an off-white solid (7.8 g, yield 92.6%).

### Preparation of Intermediate 14.4:

Compound 1.4 (7.8 g, 13.6 mmol, 1.0 eq), and dichloromethane (32 mL) were added into a 250 mL three-neck reaction flask. Under nitrogen protection and magnetic stirring, trifluoroacetic acid (15.5 g, 135.9 mmol) was added at 0°C, then reacted at room temperature for 3 hours. The mixture was concentrated under a reduced pressure, and evaporated to remove the solvent. Dichloromethane (100 mL) was added, a saturated aqueous solution of sodium bicarbonate was used for washing, and an aqueous phase was extracted with dichloromethane (50 mL). The organic phases were combined, then washed with 100 mL of pure water and dried over anhydrous sodium sulfate. It was filtered and concentrated to obtain an off-white solid (6.2 g, yield 96.2%).

### Preparation of Compound 14 Hydrochloride:

Compound 1.5 (6.0 g, 12.6 mmol, 1.0 eq), and ethyl acetate (42 mL) were added into a 250 mL single-neck reaction flask. Under nitrogen protection and magnetic stirring, a solution of hydrogen chloride in ethyl acetate (3 M, 5.0 mL, 15.0 mmol) and methyl tert-butyl ether (42 mL) were added at room temperature. The mixture was stirred at room temperature for 50 minutes, and stirred at 0°C for 1 hour. After filtration, ethyl acetate (10 mL) was pre-cooled for washing to obtain an off-white solid (5.3 g, yield 82.0%).

¹H NMR (400 MHz, CDCl₃) δ 8.54 (s, 1H), 8.38 - 8.02 (s, 3H), 5.18 - 4.96 (m, 1H), 4.33 (d, *J* = 5.3 Hz, 1H), 4.07 (s, 2H), 2.52 (t, *J =* 8.9 Hz, 1H), 2.43 (q, *J* = 6.5 Hz, 1H), 2.12 (s, 3H), 2.02 (dd, *J =* 12.3, 3.4 Hz, 1H), 1.82 - 1.56 (m, 6H), 1.56 - 1.32 (m, 6H), 1.32 - 1.20 (m, 4h), 1.16 (d, *J =* 6.8 Hz, 6H), 1.12 (d, *J =* 6.7 Hz, 3H), 1.03 - 0.81 (m, 2H), 0.79 (s, 3H), 0.61 (s, 3H).

MS: m/z [M+H]⁺ 475. 3.

### Example 15. Synthesis of Compound 15 Hydrochloride

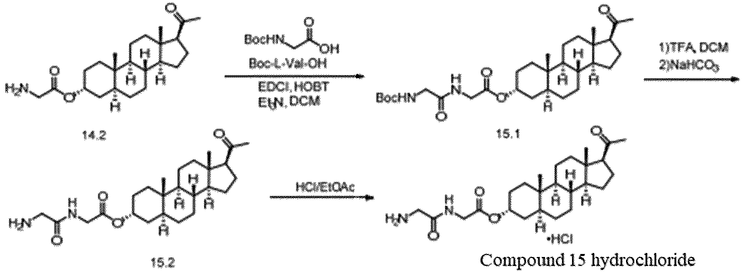

### Preparation of intermediate 15.1:

Compound 14.2 (5.0 g, 13.3 mmol), Boc-Gly-OH (2.8 g, 16.0 mmol), dichloromethane (50 mL), triethylamine (1.6 g, 16.0 mmol), HOBt (0.7 g, 5.1 mmol), and EDCI (3.1 g, 16.2 mmol) were added into a 250 mL single-neck reaction flask. The mixture was reacted at room temperature for 4 hours, and the reaction solution was washed with H₂O (50 mL), 1N HCl (50 mL), and a saturated aqueous solution of NaHCO₃. The reaction solution was concentrated under a reduced pressure, and the crude product was subjected to column chromatography (petroleum ether (60-90)/ethyl acetate 10 : 1-2:1) to obtain a white solid (6.2 g, yield 87.4%).

### Preparation of Intermediate 15.2:

Compound 15.1 (3.0 g, 5.6 mmol, 1.0 eq), and dichloromethane (15 mL) were added into a 250 mL three-neck reaction flask. Under nitrogen protection and magnetic stirring, trifluoroacetic acid (6.4 g, 56 mmol) was added at 0°C, then reacted at room temperature for 3 hours. The mixture was concentrated under a reduced pressure, dichloromethane (100 mL) was added, 100 mL of saturated aqueous solution of sodium bicarbonate was used for washing, and an aqueous phase was extracted with dichloromethane (50 mL). The organic phases were combined, then washed with 100 mL of pure water and dried over anhydrous sodium sulfate. It was filtered and concentrated to obtain a white solid (2.0 g, yield 82.1%).

### Preparation of Compound 15 Hydrochloride:

Compound 15.2 (2.0 g, 4.6 mmol, 1.0 eq), and ethyl acetate (20 mL) were added into a 250 mL single-neck reaction flask. Under nitrogen protection and magnetic stirring, a solution of hydrogen chloride in ethyl acetate (3 M, 1.9 mL, 5.7 mmol) was added at room temperature, and after the addition is completed, stirring was continued for 1 hour. A white solid was precipitated out, and filtered. The solid was dissolved by reflux heating with MeCN/H₂O (23 mL), stirred at room temperature for 1 hour, and stirred at 0°C for 1 hour. After filtration, MeCN (5 mL) was used for washing. An oil pump was used for vacuum drying at 40°C for 3 hours to obtain a white solid (1.2 g, yield 55.3%).

¹H NMR (400 MHz, CD₃OD) δ 5.10 - 5.02 (m, 1H), 4.03 (s, 2H), 3.74 (s, 2H), 2.63 (t, *J* =9.0 Hz, 1H), 2.17 - 2.00 (m, 2H), 2.11 (s, 3H), 1.85 - 1.10 (m, 18H), 1.07 - 0.90 (m, 1H), 0.88 - 0.77 (m, 1H), 0.85 (s, 3H), 0.61 (s, 3H).

MS: m/z [M+H]⁺ 433. 5.

### Example 16. Synthesis of Compound 16 Hydrochloride

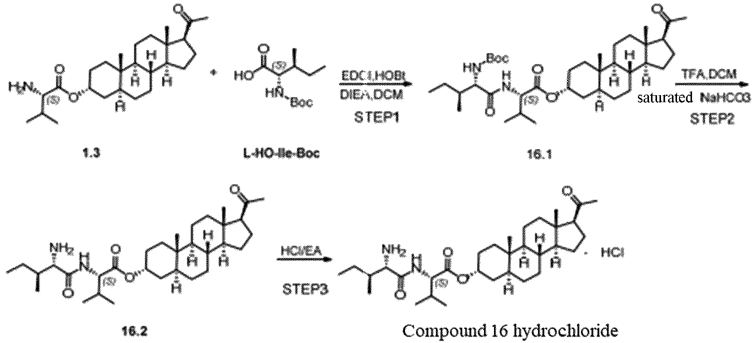

### Preparation of intermediate 16.1:

L-OH-IIe-Boc (2.00 g, 8.63 mmol, 1.2 eq), EDCI (1.65 g, 8.63 mmol, 1.2 eq), HOBt (0.2 g, 1.40 mmol, 1.2 eq) and N,N-diisopropylethylamine (1.86 g, 14.38 mmol, 2.0 eq) were added into a 100 mL eggplant-shaped flask. Dichloromethane (25 mL) was added, and stirred in an ice bath to a homogeneous phase. Then, **1.3** (3.00 g, 7.19 mmol, 1.0 eq) was slowly added into the reaction solution, stirred in an ice bath for 1 hour, and stirred at room temperature for 3 hours. The reaction was determined to be completed by TLC detection. The reaction solution was adjusted to neutral with 1.0 M hydrochloric acid, the liquid-separation was conducted, and the organic phases were washed with a saturated NaHCO₃, combined, and dried over anhydrous sodium sulfate for 2.0 hours; and the solvent was removed by evaporation to obtain a colorless colloid (4.12 g, yield 90.95%).

### Preparation of Intermediate 16.2:

**16.1** (4.12 g, 6.53 mmol, 1.0 eq), and dichloromethane (40 mL) were added into a 100 mL reaction flask, and stirred to a homogeneous phase. Trifluoroacetic acid (12 mL) was added, and the reaction solution gradually became light yellow, and stirred at room temperature for 1 hour. The reaction was detected to be completed by TLC. The solvent was removed by evaporation to obtain a colorless oil, which was dissolved with dichloromethane. The pH of the solution was adjusted to about 8 with a saturated NaHCO₃, and the liquid-separation was conducted to obtain an organic phase, which was dried over anhydrous sodium sulfate. The solvent was removed by evaporation to obtain a colorless oil (3.15 g, yield 90.78%).

### Preparation of Compound 16 Hydrochloride:

**16.2** (3.15 g, 5.94 mmol, 1.0 eq), and ethyl acetate (50 mL) were added into a 50 mL reaction flask, and stirred to a homogeneous phase. The pH of the reaction solution was adjusted to about 3 to 4 by adding a solution of hydrogen chloride in ethyl acetate. With the addition of HCl/ethyl acetate, a white solid was gradually precipitated out of the reaction solution; and stirred and crystallized at room temperature for 1.0 hour. It was filtered to obtain a white solid (1.82 g, yield 54.17%).

¹H NMR (400 MHz, CDCl₃) δ 8.39 (s, 3H), 7.40 (d, *J =* 7.6 Hz, 1H), 5.10 (s, 1H), 4.48 (t, *J =* 6.1 Hz, 1H), 4.25 (s, 1H), 2.53 (t, *J =* 8.8 Hz, 1H), 2.27 (d, *J =* 6.0 Hz, 1H), 2.12 (s, 3H), 2.01 (d, *J =* 118 Hz, 1H), 1.81 (s, 5H), 1.69 (d, *J =* 11.6 Hz, 6H), 1.49 (s, 2H), 1.41 (d, *J =* 10.3 Hz, 3H), 1.31 - 1.24 (m, 1H), 1.24 - 1.20 (m, 3H), 1.19 (s, 3H), 1.07 (d, *J* = 6.8 Hz, 3H), 1.06 - 1.00 (m, 6H), 0.98 (t, *J* = 7.2 Hz, 3H), 0.80 (s, 4H), 0.61 (s, 3H).

MS: m/z [M+H]⁺ 531. 39.

### Example 17. Synthesis of Compound 17 Hydrochloride

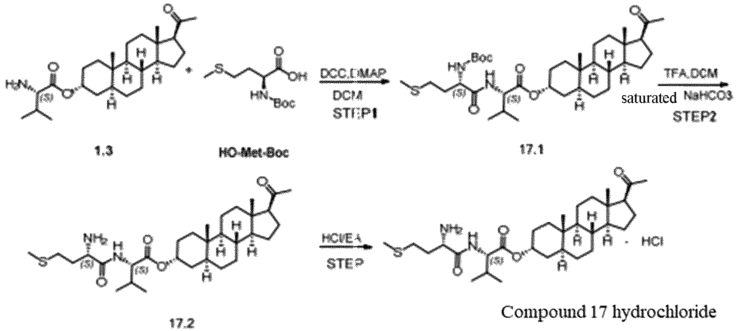

### Preparation of Intermediate 17.1:

**1.3** (3.00 g, 7.19 mmol, 1.0 eq), L-HO-Met-Boc (2.15 g, 8.63 mmol, 1.2 eq), DMAP (0.10 g, 0.72 mmol, 0.1 eq), and dichloromethane (20 mL) were added into a 100 mL reaction flask, and stirred in an ice bath to a homogeneous phase. DCC (1.78 g, 8.63 mmol, 1.2 eq) was dissolved in 10 mL of dichloromethane, added to the reaction solution, then stirred at room temperature for 3 hours. The reaction was detected to be completed by TLC. A white solid DCU was removed by filtration, and the organic phases were washed with a saturated NaHCO₃, combined, and dried over anhydrous sodium sulfate; the solvent was removed by evaporation to obtain a white solid mass, and was purified by a silica gel column (petroleum ether (60-90)/ethyl acetate = 10 : 1) to finally obtain a white solid (3.4 g, yield 72.96%).

### Preparation of Intermediate 17.2:

**17.1** (3.4 g, 4.32 mmol, 1.0 eq), and dichloromethane (30 mL) were added into a 100 mL reaction flask, and stirred to a homogeneous phase. Trifluoroacetic acid (9 mL) was added, and the reaction solution gradually became light yellow, and stirred at room temperature for 1 hour. The reaction was detected to be completed by TLC. The solvent was removed by evaporation to obtain a colorless oil, which was dissolved with 10 mL of dichloromethane. The pH of the solution was adjusted to about 8 with a saturated NaHCO₃, the liquid was separated, and the organic phases were combined, and dried over anhydrous sodium sulfate. The solvent was removed by evaporation to obtain a colorless oil (1.82 g, yield 63.41%).

### Preparation of Compound 17 Hydrochloride:

**17.2** (1.82 g, 3.33 mmol, 1.0 eq), and dichloromethane (5 mL) were added into a 25 mL reaction flask, and stirred to a homogeneous phase. The pH of the reaction solution was adjusted to about 3 to 4 by adding a solution of hydrogen chloride in ethyl acetate. The solvent was removed by evaporation to obtain a colorless oil liquid. Ethyl acetate (10 mL) was added for stirring and pulping. The reaction solution gradually became a white suspension. After pulping for 3 hours, it was filtered to obtain a white solid (1.2 g, yield 61.86%).

¹H NMR (400 MHz, CDCl₃) δ 8.56 (s, 3H), 7.62 (s, 1H), 5.10 (s, 1H), 4.53 (s, 2H), 2.77 (s, 2H), 2.53 (t, *J =* 8.8 Hz, 1H), 2.42 (s, 1H), 2.16 (s, 3H), 2.12 (s, 3H), 2.01 (d, *J =* 11.9 Hz, 2H), 1.79 (s, 1H), 1.69 (d, *J =* 11.9 Hz, 4H), 1.66 - 1.58 (m, 2H), 1.54 (d, *J =* 13.3 Hz, 2H), 1.49 (s, 3H), 1.41 (t, *J =* 13.0 Hz, 2H), 1.28 (d, *J =* 12.9 Hz, 4H), 1.19 (d, *J =* 1.0 Hz, 3H), 1.05 (d, *J* = 6.8 Hz, 3H), 1.01 (d, *J* = 6.6 Hz, 3H), 0.80 (s, 4H), 0.61 (s, 3H).

MS: m/z [M+H]⁺ 549. 4.

### Example 18. Synthesis of Compound 18 Hydrochloride

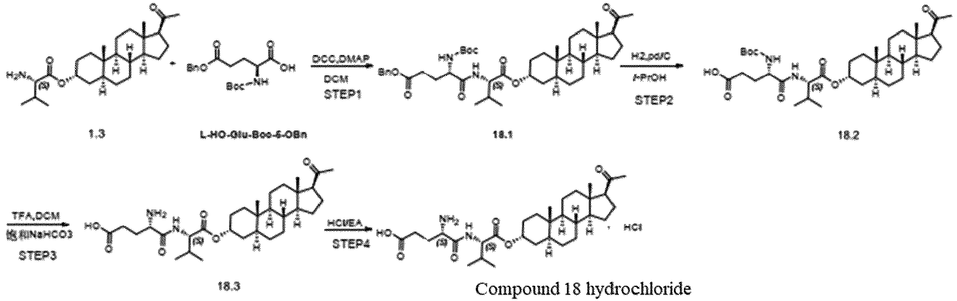

### Preparation of Intermediate 18.1:

**1.3** (3.00 g, 7.19 mmol, 1.0 eq), L-HO-Glu-Boc-5-OBn (2.91 g, 8.63 mmol, 1.2 eq), DMAP (0.10 g, 0.72 mmol, 0.1 eq), and dichloromethane (20 mL) were added into a 100 mL reaction flask, and stirred in an ice bath to a homogeneous phase. DCC (1.78 g, 8.63 mmol, 1.2 eq) was dissolved in 10 mL of dichloromethane, added to the reaction solution, then stirred at room temperature for 3 hours. The reaction was detected to be completed by TLC. A white solid DCU was removed by filtration, and the filtrate was washed with a saturated NaHCO₃. The organic phase was dried over anhydrous sodium sulfate, and concentrated under a reduced pressure. The crude product was subjected to column chromatography (petroleum ether (60-90)/ethyl acetate = 10 : 1) to obtain a white solid (4.7 g, yield 88.85%).

### Preparation of Intermediate 18.2:

A hydrogenation kettle was prepared, and configured with magnetic stirring device. **18.1** (4.7 g, 6.38 mmol, 1.0 eq), and isopropanol (40 mL) were added into a reaction flask, Pd/C (10%) was added, hydrogen replacement was carried out for 3 times, the reaction was carried out under a hydrogen pressure of about 1 MPa, and stirred at room temperature for 8 hours. The reaction was detected to be completed by TLC. The solvent was removed by evaporation to obtain a colorless oil, which was dissolved with dichloromethane and washed with water. The organic phases were combined, and dried over anhydrous sodium sulfate. The solvent was removed by evaporation to obtain a white solid (3.12 g, yield 75.54%).

### Preparation of Intermediate 18.3:

**18.2** (3.0 g, 4.64 mmol, 1.0 eq), and dichloromethane (30 mL) were added into a 100 mL reaction flask, and stirred to a homogeneous phase. Trifluoroacetic acid (9 mL) was added, and the reaction solution gradually became light yellow, and stirred at room temperature for 1 hour. The reaction was detected to be completed by TLC. The solvent was removed by evaporation to obtain a colorless oil, which was dissolved with dichloromethane. The pH of the solution was adjusted to about 8 with a saturated NaHCO₃, and the liquid was separated to obtain an organic phase, which was dried over anhydrous sodium sulfate. The solvent was removed by evaporation to obtain a colorless oil (1.82 g, yield 71.65%).

### Preparation of Compound 18 Hydrochloride:

Intermediate 3 (1.82 g, 3.33 mmol, 1.0 eq), and dichloromethane (5 mL) were added into a 25 mL reaction flask, and stirred to a homogeneous phase. HCl/ethyl acetate was added to adjust the pH of the reaction solution to about 3 to 4, and stirred for 10 minutes. The solvent was removed by evaporation to obtain a colorless oil liquid. Ethyl acetate (20 mL) was added and stirred without complete clarification. Methyl tert-butyl ether (20 mL) was added, and the reaction solution gradually became a white suspension. After pulping for 3 hours, it was filtered to obtain a white solid (1.33 g, yield 68.56%).

¹H NMR (400 MHz, CDCl₃) δ 8.39 - 8.23 (d, *J =* 7.8 Hz, 1H), 8.21 - 8.08 (s, 3H), 5.14 - 5.04 (s, 1H), 4.77 - 4.60 (s, 1H), 4.52 - 4.39 (dd, *J =* 8.0, 4.5 Hz, 1H), 2.84 - 2.64 (s, 2H), 2.58 -2.47 (t, *J =* 8.8 Hz, 1H), 2.44 - 2.33 (s, 1H), 2.33 - 2.21 (dd, *J =* 11.9, 6.0 Hz, 2H), 2.12 - 2.07 (s, 3H), 2.04 - 1.96 (d, *J =* 11.1 Hz, 1H), 1.81 - 1.56 (t, *J =* 15.4 Hz, 7H), 1.56 - 1.32 (m, 7H), 1.31 - 1.07 (m, 6H), 1.05 - 1.00 (d, *J =* 3.0 Hz, 3H), 1.00 - 0.93 (d, *J =* 4.7 Hz, 4H), 0.82 -0.75 (s, 4H), 0.65 - 0.55 (s, 3H).

MS: m/z[M+H]⁺ 546. 4.

### Example 19. Synthesis of Compound 19 Hydrochloride

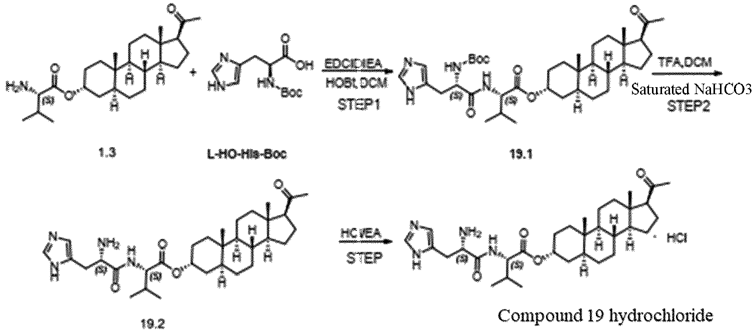

### Preparation of intermediate 19.1:

L-HO-His-Boc (2.20 g, 8.63 mmol, 1.2 eq), EDCI (1.65 g, 8.63 mmol, 1.2 eq), HOBt (0.2 g, 1.40 mmol, 1.2 eq), N,N-diisopropylethylamine (1.86 g, 14.38 mmol, 2.0 eq), and dichloromethane (3 mL) were added into a 100 mL reaction flask, and stirred in an ice bath to a homogeneous phase. Then, 1.3 (3.00 g, 7.19 mmol, 1.0 eq) was slowly added to the reaction solution, stirred in an ice bath for 1 hour, and stirred at room temperature for 3 hours. The reaction was determined to be completed by TLC detection. The reaction solution was adjusted to neutral with 1.0 M hydrochloric acid, the liquid was separated, and the organic phase was washed with a saturated NaHCO₃, and dried over anhydrous sodium sulfate. The solvent was removed by evaporation to obtain a light yellow oil (3.81 g, yield 96.38%).

### Preparation of intermediate 19.2:

**19.1** (3.81 g, 6.92 mmol, 1.0 eq), dichloromethane (60 mL), and trifluoroacetic acid (20 mL) were added into a 100 mL reaction flask. The reaction solution gradually became dark yellow, and stirred at room temperature for 1 hour. The reaction was detected to be completed by TLC. The solvent was removed by evaporation to obtain a colorless oil, which was dissolved with dichloromethane. The pH of the solution was adjusted to about 8 with a saturated NaHCO₃, the liquid-separation was conducted, and an organic phase was obtained, and dried over anhydrous sodium sulfate. The solvent was removed by evaporation to obtain a colorless oil (3.09 g, yield 95.96%).

### Preparation of Compound 19 Hydrochloride:

**19.2** (3.09 g, 5.57 mmol, 1.0 eq), and ethyl acetate (20 mL) were added into a 50 mL reaction flask, and stirred for dissolution. The pH of the reaction solution was adjusted to about 3 to 4 by adding a solution of hydrogen chloride in ethyl acetate. A white solid was gradually precipitated out of the reaction solution. After stirred and crystallized for 4 hours, it was filtered to obtain a white solid (2.11 g, yield 64.33%).

¹H NMR (400 MHz, CDCl₃) δ 13.88 (s, 1H), 8.88 (s, 1H), 8.58 (s, 3H), 7.56 (d, *J* = 24.9 Hz, 1H), 5.11 (s, 1H), 4.98 (s, 1H), 4.50 (s, 1H), 3.57 (s, 2H), 2.51 (s, 2H), 2.11 (s, 3H), 2.00 (s, 1H), 1.66 (s, 6H), 1.50 (s, 4H), 1.39 (s, 2H), 1.32 - 1.11 (m, 7H), 1.04 (s, 7H), 0.79 (s, 4H), 0.60 (s, 3H).

MS: m/z [M + H]⁺ 555. 4.

### Example 20. Synthesis of Compound 20 Hydrochloride

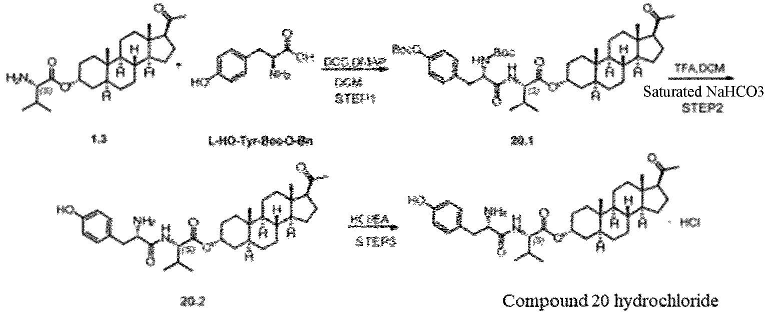

### Preparation of intermediate 20.1:

**1.3** (3.00 g, 7.19 mmol, 1.0 eq), L-HO-Tyr-Boc-O-Boc (2.74 g, 8.63 mmol, 1.2 eq), DMAP (0.10 g, 0.72 mmol, 0.1 eq), and dichloromethane (20 mL) were added into a 100 mL reaction flask, and stirred in an ice bath to a homogeneous phase. DCC (1.78 g, 8.63 mmol, 1.2 eq) was dissolved in 10 mL of dichloromethane, then is added to the reaction solution, then stirred at room temperature for 3 hours. The reaction was detected to be completed by TLC. A white solid was removed by filtration, and the organic phases were washed with a saturated NaHCO₃, combined, and dried over anhydrous sodium sulfate; the solvent was removed by evaporation to obtain a white solid mass, and was purified by a silica gel column (petroleum ether (60-90)/ethyl acetate = 10 : 1 to 5 : 1) to obtain a white solid (0.6 g, yield 10.69%).

### Preparation of intermediate 20.2:

**20.1** (0.60 g, 0.77 mmol, 1.0 eq), and dichloromethane (6.0 mL) were added into a 25 mL reaction flask, and stirred to a homogeneous phase. Trifluoroacetic acid (2.0 mL) was added, and stirred at room temperature for 1 hour. The reaction was detected to be completed by TLC. The solvent was removed by evaporation to obtain a colorless oil, which was dissolved with dichloromethane. A saturated aqueous solution of NaHCO₃ was used for washing, and the pH of an aqueous phase was 7 to 8. The liquid-separation was conducted, and the organic phase was dried over anhydrous sodium sulfate. The solvent was removed by evaporation to obtain a colorless oil (0.43 g, yield 95.56%).

### Preparation of Compound 20 Hydrochloride:

**20.2** (0.4 g, 0.68 mmol, 1.0 eq), and ethyl acetate (6.0 mL) were added into a 25 mL reaction flask, and stirred for dissolution. HCl/ethyl acetate was added to adjust the pH of the reaction solution to about 3 to 4. After methyl tert-butyl ether (1.5 mL) was added, stirring and crystallization were carried out for 1 hour. It was filtered and dried to obtain a white solid (0.41 g, yield 97.62%).

¹H NMR (400 MHz, CDCl₃) δ 8.11 (s, 5H), 7.19 (d, *J =* 7.7 Hz, 2H), 6.80 (d, *J* = 7.6 Hz, 2H), 5.09 (s, 1H), 4.57 (s, 1H), 4.43 (s, 1H), 3.30 (s, 1H), 3.06 (s, 1H), 2.43 (t*, J =* 8.3 Hz, 1H), 2.24 (s, 1H), 2.09 (s, 3H), 1.90 (s, 1H), 1.82 - 1.56 (m, 5H), 1.49 (s, 5H), 1.38 - 1.20 (m, 4H), 1.22 - 1.07 (m, 4H), 1.07 - 0.86 (m, 8H), 0.77 (s, 3H), 0.70 (s, 1H), 0.56 (s, 3H).

MS: m/z[M+H]⁺ 581. 4.

### Example 21. Synthesis of Compound 21 Hydrochloride

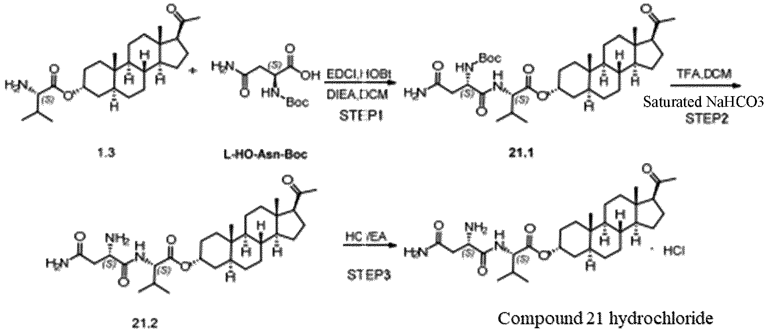

### Preparation of intermediate 21.1:

L-HO-Asn-Boc (2.00 g, 8.63 mmol, 1.2 eq), EDCI (1.65 g, 8.63 mmol, 1.2 eq), HOBt (0.2 g, 1.40 mmol, 1.2 eq), N,N-diisopropylethylamine (1.86 g, 14.38 mmol, 2.0 eq), and dichloromethane (30 mL) were added into a 100 mL reaction flask, and stirred in an ice bath to a homogeneous phase. Then, **1.3** (3.00 g, 7.19 mmol, 1.0 eq) was added to the reaction solution, then stirred at room temperature for 3 hours. The reaction was determined to be completed by TLC detection. The reaction solution was adjusted to neutral with 1.0 M hydrochloric acid, and the liquid-separation was conducted, washed with a saturated aqueous solution of NaHCO₃, and dried over anhydrous sodium sulfate. It was filtered and concentrated under a reduced pressure, and the solvent was evaporated to obtain a white mass solid (4.17 g, yield 91.85%).

### Preparation of intermediate 21.2:

**21.1** (4.17 g, 6.60 mmol, 1.0 eq), and dichloromethane (40 mL) were added into a 100 mL reaction flask, and stirred to a homogeneous phase. Trifluoroacetic acid (12 mL) was added, and stirred at room temperature for 1 hour. The reaction was detected to be completed by TLC. The solvent was removed by evaporation to obtain a colorless oil, which was dissolved with dichloromethane. The pH of the solution was adjusted to about 8 with a saturated NaHCO₃, the liquid-separation was conducted, and an organic phase was obtained, and dried over anhydrous sodium sulfate. The solvent was removed by evaporation to obtain a colorless oil (3.01 g, yield 86.00%).

### Preparation of Compound 21 Hydrochloride:

**21.2** (3.00 g, 6.57 mmol, 1.0 eq), and ethyl acetate (30 mL) were added into a 50 mL reaction flask, and stirred for dissolution. The pH of the reaction solution was adjusted to about 3 to 4 by adding a solution of hydrogen chloride in ethyl acetate. The reaction solution was clarified, methyl tert-butyl ether was added, and the mixture was stirred and crystallized. It was filtered to obtain a white solid (1.8 g, yield 56.25%).

¹H NMR (400 MHz, CDCl₃) δ 8.63 (d, *J* = 8.1 Hz, 1H), 8.23 (brs, 3H), 7.81 (s, 1H), 7.02 (s, 1H), 5.06 (s, 1H), 4.80 (s, 1H), 4.46 (dd, *J* = 8.3, 4.2 Hz, 1H), 3.11 (s, 2H), 2.52 (t, *J =* 8.8 Hz, 1H), 2.28 (d, *J =* 9.6 Hz, 1H), 2.11 (s, 3H), 2.01 (d, *J =* 10.8 Hz, 1H), 1.81 - 1.56 (m, 6H), 1.56 - 1.44 (m, 4H), 1.37 (d, *J* = 21.9 Hz, 1H), 1.26 (qd, *J* = 6.3, 5.9, 3.3 Hz, 2H), 1.19 (s, 6h), 0.99 (t, *J* = 7.3 Hz, 7H), 0.79 (s, 4H), 0.61 (s, 3H).

MS: m/z [M+H]⁺ 532. 4.

### Example 22. Synthesis of Compound 22 Hydrochloride

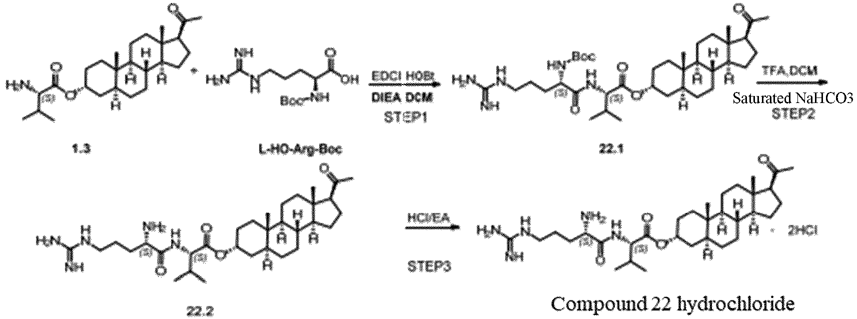

### Preparation of intermediate 22.1:

**L-HO-Ar g-Boc** (3.23 g, 8.63 mmol, 1.2 eq), **EDCI** (1.65 g, 8.63 mmol, 1.2 eq), **HOBt** (0.2 g, 1.40 mmol, 1.2 eq), **N,N-diisopropylethylamine** (1.86 g, 14.38 mmol, 2.0 eq), and dichloromethane (3 mL) were added into a 100 mL reaction flask, and stirred to a homogeneous phase in an ice bath. Then, **1.3** (3.00 g, 7.19 mmol, 1.0 eq) was added into the reaction solution, and stirred at room temperature for 3 hours. The reaction was determined to be completed by TLC detection. The pH of the reaction solution was adjusted to neutral with 1.0 M hydrochloric acid, and the liquid-separation was conducted. The organic phase was washed with a saturated NaHCO₃, and dried over anhydrous sodium sulfate. The organic phase was concentrated to obtain a light yellow oil (4.21 g, yield 86.98%).

### Preparation of intermediate 22.2:

**22.1** (4.21 g, 6.25 mmol, 1.0 eq), and dichloromethane (40 mL) were added into a 100 mL reaction flask, and stirred to a homogeneous phase. Trifluoroacetic acid (12 mL) was added, and stirred at room temperature for 1 hour. The reaction was detected to be completed by TLC. The solvent was removed by evaporation to obtain a colorless oil, which was dissolved with dichloromethane. a saturated aqueous solution of NaHCO₃ was used for washing, and the pH of an aqueous phase was 7 to 8. The liquid-separation was conducted, and the organic phase was dried over anhydrous sodium sulfate. It was filtered and concentrated to obtain a colorless oil (3.23 g, yield 90.23%).

### Preparation of Compound 22 Hydrochloride:

**22.2** (3.23 g, 5.63 mmol, 1.0 eq), and isopropanol (20 mL) were added into a 100 mL reaction flask, and stirred for dissolution. The pH of the reaction solution was adjusted to about 3 to 4 by adding a solution of hydrogen chloride in ethyl acetate, and stirring and crystallization were carried out for 1 hour. After filtration, an oil pump was used for vacuum drying at 40°C for 4 hours to obtain a white solid (0.84 g, yield 22.46%).

¹H NMR (400 MHz, CDCl₃) δ 8.53 (s, 1H), 8.28 (s, 3H), 7.54 (s, 1H), 6.96 (s, 4H), 5.07 (s, 1H), 4.53 (s, 1H), 4.45 (s, 1H), 3.35 (d, *J =* 28.0 Hz, 2H), 2.51 (s, 1H), 2.29 (s, 1H), 2.11 (s, 3H), 2.02 (s, 1H), 1.87 (s, 2H), 1.67 (s, 6H), 1.48 (s, 5H), 1.41 (s, 2H), 1.33 - 1.23 (m, 2H), 1.23 - 1.08 (m, 5H), 1.00 (d, *J* = 6.1 Hz, 7H), 0.91 (s, 1H), 0.79 (s, 4H), 0.61 (s, 3H).

MS: m/z [M + H]⁺ 574. 4.

### Example 23. Synthesis of Compound 23 Hydrochloride

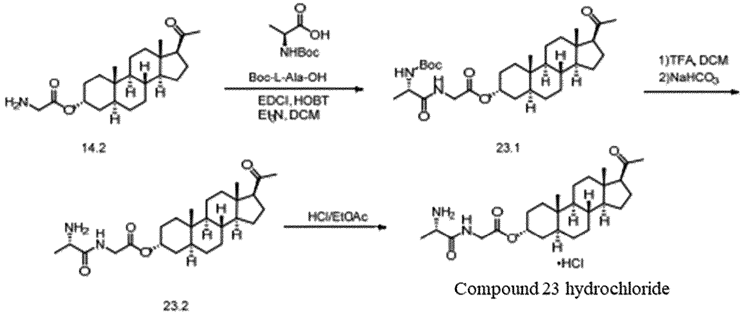

### Preparation of intermediate 23.1:

Compound 14.2 (5.0 g, 13.3 mmol), Boc-L-Ala-OH (3.0 g, 15.9 mmol), dichloromethane (50 mL), triethylamine (1.6 g, 16.0 mmol), HOBT (0.7 g, 5.1 mmol), and EDCI (3.1 g, 16.2 mmol) were added into a 250 mL single-neck reaction flask. The mixture was reacted at room temperature for 4 hours, and washed with a saturated aqueous solution of NaHCO₃. The reaction solution was concentrated under a reduced pressure, and the crude product was subjected to column chromatography (petroleum ether (60-90)/ethyl acetate 10 : 1-2 : 1) to obtain a white solid (6.5 g, yield 89.3%).

### Preparation of Intermediate 23.2:

Compound 23.1 (3.5 g, 6.4 mmol, 1.0 eq), and dichloromethane (18 mL) were added into a 250 mL three-neck reaction flask. Under nitrogen protection and magnetic stirring, trifluoroacetic acid (7.3 g, 64 mmol) was added at 0°C, then reacted at room temperature for 3 hours. The mixture was concentrated under a reduced pressure. Dichloromethane (100 mL) was added, and a saturated aqueous solution of sodium bicarbonate was used for washing. Organic phases were combined, and dried over anhydrous sodium sulfate. It was filtered and concentrated to obtain a white solid (2.6 g, yield 90.9%).

### Preparation of Compound 23 Hydrochloride:

Compound 23.2 (2.6 g, 5.85 mmol, 1.0 eq), and ethyl acetate (20 mL) were added into a 250 mL single-neck reaction flask. Under nitrogen protection and magnetic stirring, HCl/ethyl acetate (3 M, 2.4 mL, 7.2 mmol) was added at room temperature, and after the addition is completed, stirring was continued for 3 hours. A white solid was precipitated out, filtered, and washed with ethyl acetate (5 mL). An oil pump was used for vacuum drying at 40°C for 3 hours to obtain a white solid (2.1 g, yield 74.6%).

¹H NMR (400 MHz, CDCl₃) δ 8.66 - 8.46 (m, 1H), 8.16 (brs, 3H), 5.18 - 5.02 (m, 1H), 4.70 - 4.52 (m, 1H), 4.44 - 3.78 (m, 2H), 2.53 (d, *J =* 9.1 Hz, 1H), 2.28 - 1.96 (m, 2H),2.11 (s, 3H), 1.93 - 0.70 (m, 23H), 0.79 (s, 3H), 0.61 (s, 3H).

MS: m/z [M+H]⁺ 447. 6.

### Example 24. Solubility Test

### Test method

An appropriate amount of the compound of the present invention was weighed, prepared into solutions with different media, respectively, stirred in a constant temperature water bath magnetometer at 25°C for about 24 hours, and filtered through a 0.22 µm water filter membrane. An appropriate amount of filtrate was diluted to a certain concentration with methanol, and used as a test solution. The concentration of the test solution was determined by HPLC with a UV detector using methanol as a blank control solution.
(1) Blank solution: Methanol
(2) Reference solution: 5 mg of reference substance was weighed precisely, placed in a 10 mL measuring flask, dissolved with methanol and diluted to scale, and mixed well.
(3) Sample solution: An appropriate amount of sample solution was pipetted, diluted with methanol to about 0.5 mg/mL (determined by the concentration of each sample), and mixed well.

| | |
|---|---|
| Liquid chromatograph | Waters Acquity Arc |
| Detector | 2998 PDA detector |
| Chromatographic column | Waters Acquity UPLCBEH C18 3. 0*100 mm *1.7 µm |
| Mobile phase | 0.1% H₃PO₄ (pH was adjusted to 7.5 with ammonium hydroxide): acetonitrile = 2 : 8 |
| Flow rate | 0.3 mL/min |
| Injection volume | 10 µL |
| Column oven | 35°C |
| Sample tray | Uncontrolled temperature |
| Detection wavelength | 205 nm |

### Test result

| Medium | pH4.5 buffer solution | Water |
|---|---|---|
| Compound 14 hydrochloride (mg/mL (25°C)) | > 50 | > 10 |
| Compound 1 hydrochloride (mg/mL (25°C)) | > 50 | > 10 |
| Compound 13 hydrochloride (mg/mL (25°C)) | 0.01 | 0.37 |
| Compound 15 hydrochloride (mg/mL (25°C)) | 0.00 | 1.11 |
| Compound 23 hydrochloride (mg/mL (25°C)) | 0.97 | 0.64 |
| Compound 2 hydrochloride (mg/mL (25°C)) | > 10 | > 10 |
| Compound 12 hydrochloride (mg/mL (25°C)) | 1.91 | 2.62 |
| Compound 3 hydrochloride (mg/mL (25°C)) | / | 0.00 |
| Compound 4 hydrochloride (mg/mL (25°C)) | / | 0.19 |
| Compound 5 hydrochloride (mg/mL (25°C)) | / | 0.07 |
| Compound 6 hydrochloride (mg/mL (25°C)) | / | 5.68 |
| Compound 7 hydrochloride (mg/mL (25°C)) | / | 7.38 |

"j" means not tested; when the amount of dissolution is a specific value, it is expressed as "saturated solubility"; when it is "> 10", it means that the solubility is higher than 10 mg/mL.

### Example 25. Stability of Compound of the Present Invention in Glucose Solution

### Test method

An appropriate amount of the derivative of the present invention was weighed respectively, dissolved in a 5% glucose solution and prepared into a solution with a concentration of about 1 mg/g. After stirring in a constant temperature water bath magnetometer at 25°C for about 24 hours, the mixture was filtered through a 0.22 µm water filter membrane. A clear solution was diluted to a certain concentration with methanol, and used as a test solution. The sample was allowed to stand at room temperature for 0 hour, 1 hour, 3 hours, 5 hours, and 8 hours, and the solution stability of the test sample was determined by HPLC with a UV detector using methanol as a blank solvent.
(1) Blank solution: Methanol.
(2) Sample solution: A suitable solution was taken and filtered through a 0.22 µm filter membrane.

| | |
|---|---|
| Liquid chromatograph | Waters Acquity Arc |
| Detector | 2998 PDA detector |
| Chromatographic column | A gilent Pursuit 3 C18 4. 6*150 mm*3 µm |
| Mobile phase | A: 0.1% H₃PO₄ (pH was adjusted to 6.0 with ammonium hydroxide) |
| | B: Acetonitrile |
| Flow rate | 0.8 mL/min |
| Injection volume | 20 µL |
| Column oven | 35°C |
| Sample tray | Uncontrolled temperature |
| Detection wavelength | 205 nm |

### Test result

| Compound | Time | Purity/% |
|---|---|---|
| Compound 1 hydrochloride | 0 hour | 95.43 |
| | 1 hours | 95.49 |
| | 3 hours | 95.54 |
| | 5 hours | 95.20 |
| | 8 hours | 95.56 |
| Compound 10 hydrochloride | 0 hour | 99.12 |
| | 1 hours | 99.09 |
| | 3 hours | 99.04 |
| | 5 hours | 98.05 |
| | 8 hours | 97.89 |
| Compound 2 hydrochloride | 0 hour | 95.00 |
| | 1 hours | 94.59 |
| | 3 hours | 94.50 |
| | 5 hours | 94.05 |
| | 8 hours | 94.33 |

**Conclusion:** The above results indicate that the derivative of the present invention has a certain stability after being stored in an aqueous glucose solution for a certain period of time; particularly when R₁ in the 3-hydroxyl-5-pregnane-20-one derivative of formula I of the present invention is isopropyl, the purity of the derivative in the glucose solution remains substantially unchanged.

### Example 26. Pharmacokinetic Testing of the Compound of the Present Invention

The object of this experiment is to study the single oral administration of each compound solution of the present invention, and an allopregnanolone solution, to detect allopregnanolone, an active ingredient in plasma, and to evaluate its pharmacokinetic (PK) properties in SD rats. The solution was to dissolve each compound in an aqueous solution containing 5% Tween 20.

The male SD rats used in this embodiment, weighing 180-220 g, were purchased from Shanghai SLAC Laboratory Animal Co., Ltd. All animals were fasted overnight, until 4 hours after administration.

The experimental SD rats were grouped by randomized block design, and 5 rats in each group, namely allopregnanolone group, compound 1 hydrochloride group, compound 2 hydrochloride group, compound 14 hydrochloride group, and compound 15 hydrochloride group. Each group was administered by irrigation (i.g.) at a dose of 20 mg/kg (as measured by allopregnanolone).

0.0833h, 0.25h, 0.5h, 1h, 2h, 3h, 4h, 6h, 9h, 12h, 24 h after the administration, about 0.6 mL of blood sample was collected from peripheral veins and transferred to a commercial centrifuge tube containing 0.85 to 1.15 mg of K2 EDTA anticoagulant (Jiangsu Kangjian Medical Products Co., Ltd.). Within 30 minutes after blood collection, plasma was centrifuged (centrifugation at 3,000 g for 10 minutes at approximately 4°C). The plasma was placed in a labeled polypropylene tube, quick-frozen in dry ice, then stored in an ultra-low temperature freezer at -60°C or lower for testing. LC-MS/MS bioassay was used to detect the concentration of drug in SD rat plasma. Blood concentration-time data was analyzed using a non-compartment model and a WinNonlin software package (version6.3 or later), as shown in Table-3.

| Table -3 | Allopr egnano lone | Compound 1 hydrochloride | Compound 2 hydrochloride | Compound 14 hydrochloride | Compound 15 hydrochloride |
|---|---|---|---|---|---|
| Cmax (ng/mL) | 40.4 | 62.3 | 42.5 | 124.3 | 130.7 |
| Tmax (hours) | 0.5 | 2.0 | 2.8 | 0.8 | 0.8 |
| T1/2 (hours) | ND | 2.11 | 2.5 | 0.9 | 0.8 |
| AUC0-1a st (ng' hours/m L) | 24.9 | 244.9 | 196.0 | 150.2 | 166.3 |
| AUC0-in f (ng. hours/m L) | ND | 289.7 | 240.0 | 159.8 | 172.4 |

| | | | | | |
|---|---|---|---|---|---|
| "ND" indicates that it cannot be calculated | | | | | |

Conclusion: The above results indicate that the derivative of the present invention has significantly improved pharmacokinetic properties compared with the parent drug. After administration of the derivative of the present invention, both AUC and Cmax are significantly improved, and have long-acting and sustained-release properties. Particularly when R₁ in the 3-hydroxyl-5-pregnane-20-one derivative of formula I of the present invention is isopropyl, an effective physiological concentration of allopregnanolone can be maintained in vivo for a long time without sudden release, and the blood concentration curve has a little gentle fluctuation.

## Claims

1. A compound of formula I, or a pharmaceutically acceptable salt thereof: 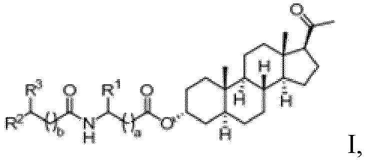 wherein,
each R¹ and R² is independently H, a substituted or unsubstituted C₁-C₁₀ alkyl, or a substituted or unsubstituted cycloalkyl; R³ is H, halogen, hydroxyl, amino, nitryl, or sulfydryl; or R² and R³ are linked to form a 5- to 6-membered saturated or unsaturated heterocyclic ring; and
each a and b is independently an integer of 0 to 3.

2. The compound or the pharmaceutically acceptable salt thereof of claim 1, wherein R³ is an amino or H.

3. The compound or the pharmaceutically acceptable salt thereof of claim 1 or 2, wherein the compound of formula I is the compound of formula I-1, I-2, I-3 or I-4: 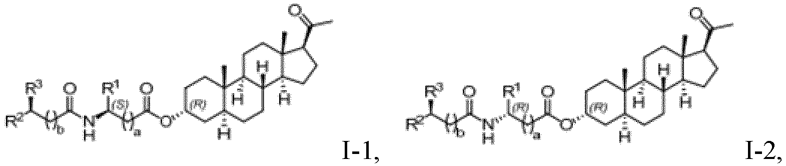 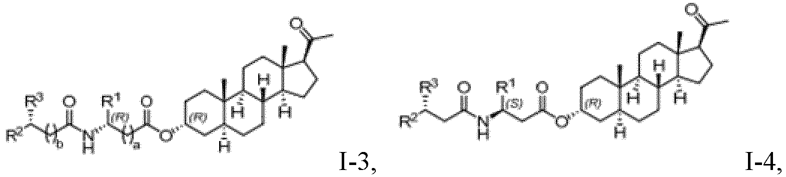 wherein R¹, R², R³, a, and b are as described in claim 1 or 2.

4. The compound or the pharmaceutically acceptable salt thereof of claim 1 or 2, wherein at least one of R¹ and R² is an isopropyl.

5. The compound or the pharmaceutically acceptable salt thereof of claim 4, wherein R¹ is an isopropyl.

6. The compound or a pharmaceutically acceptable salt thereof of claim 1, wherein the compound is selected from the group consisting of:

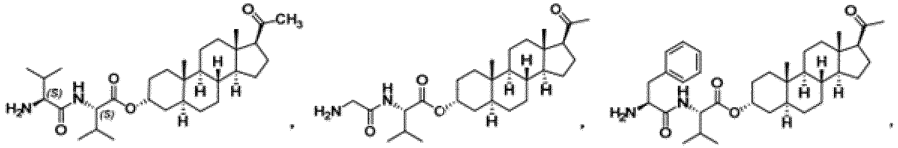

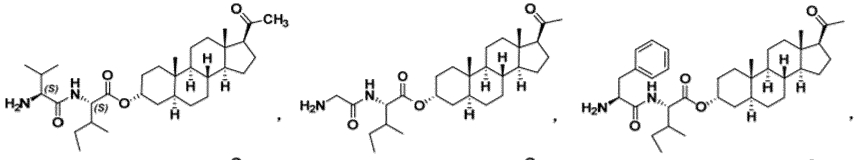

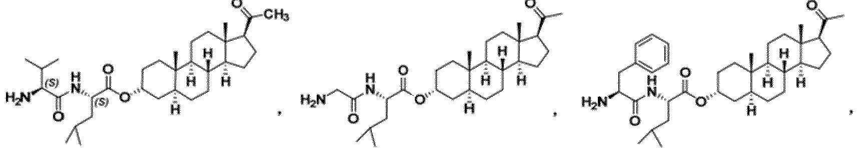

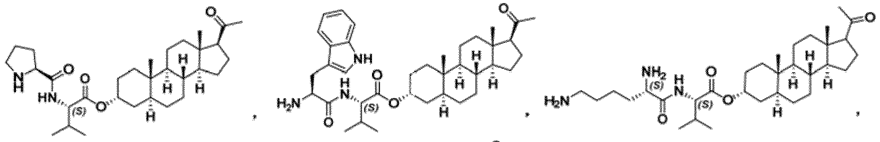

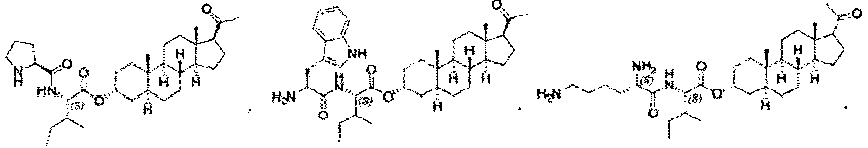

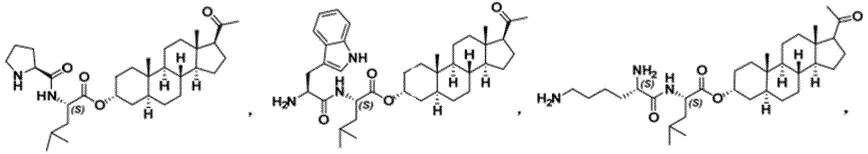

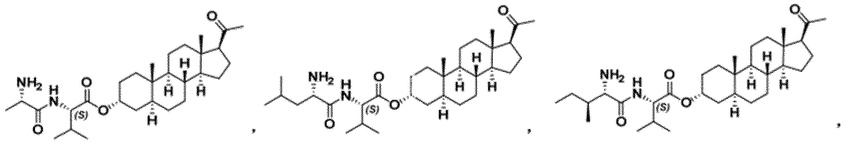

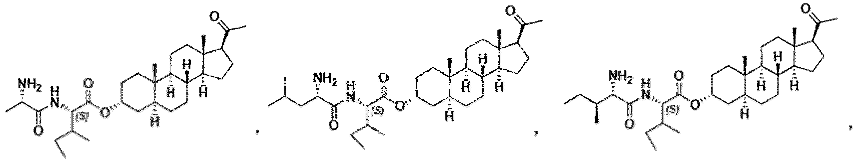

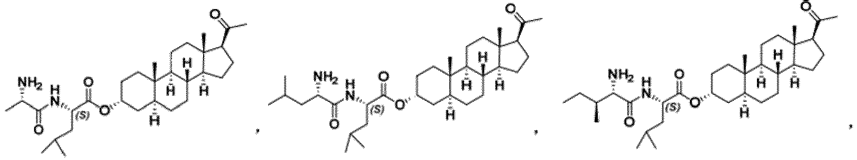

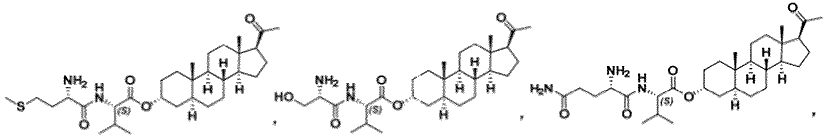

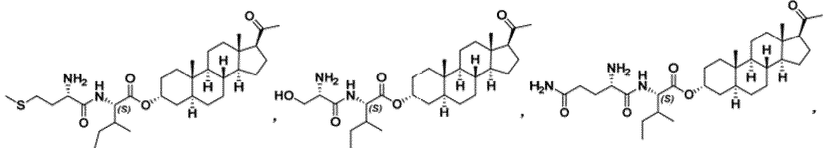

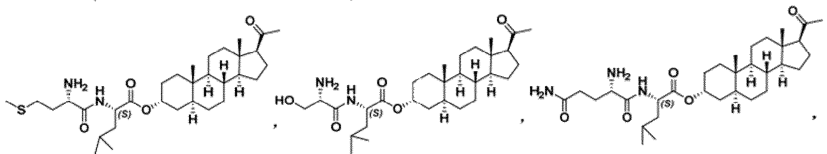

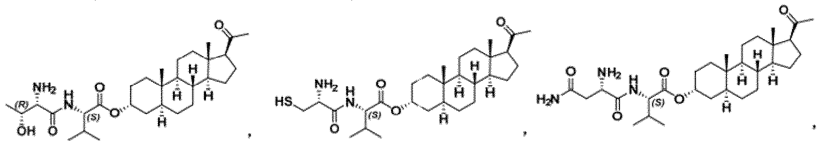

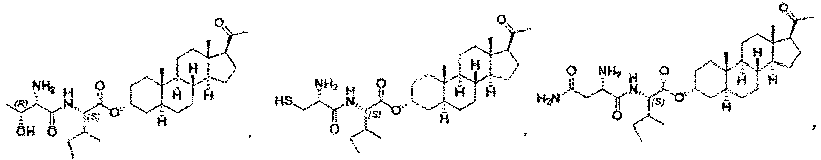

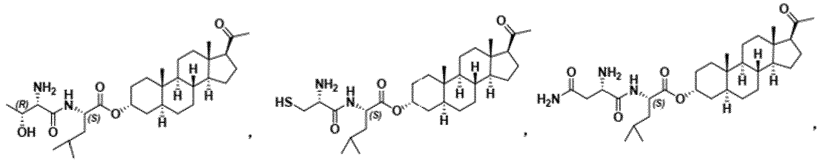

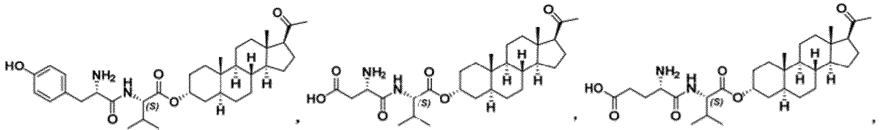

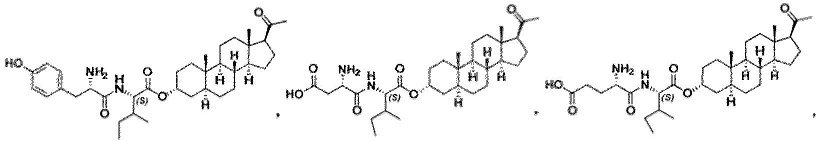

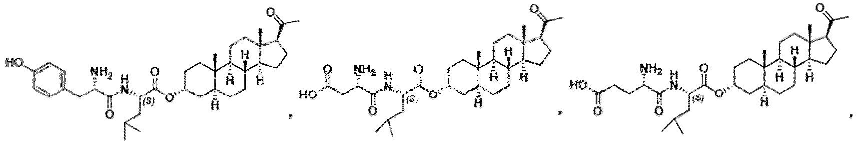

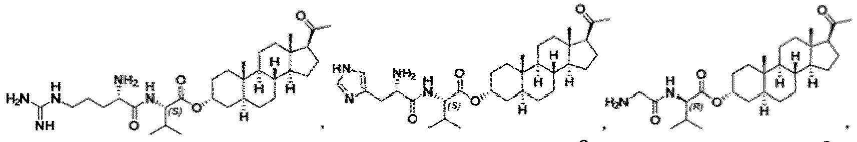

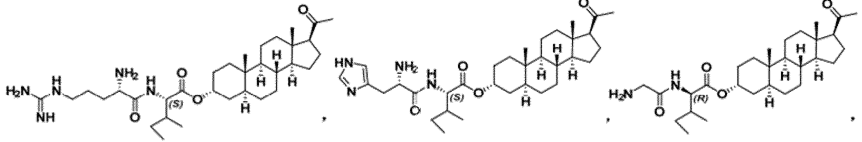

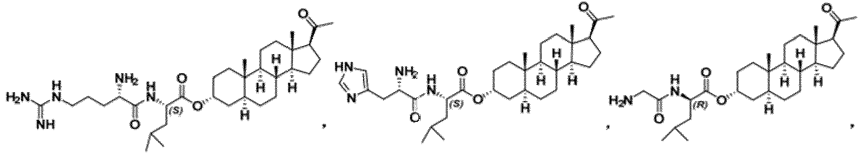

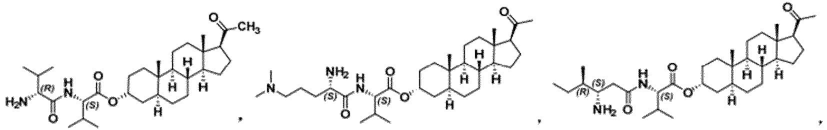

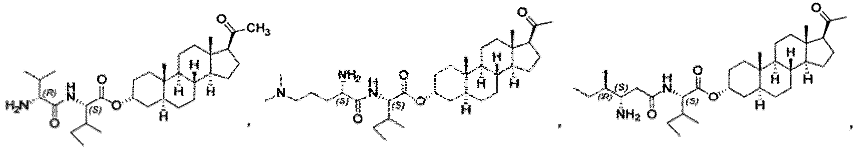

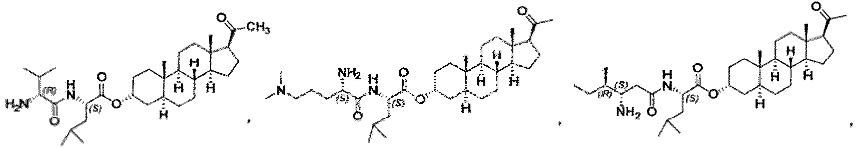

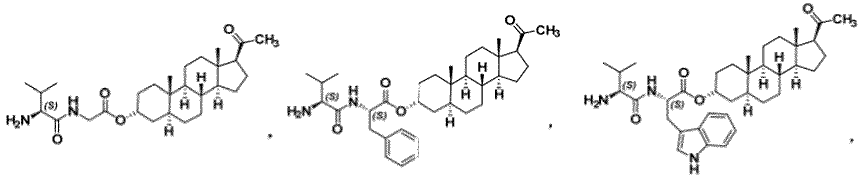

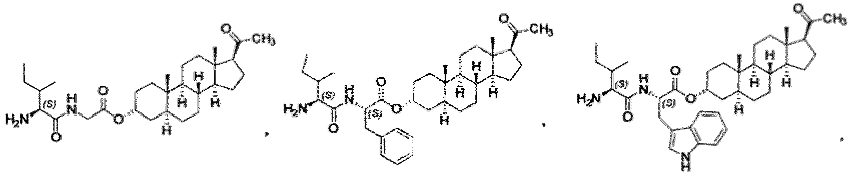

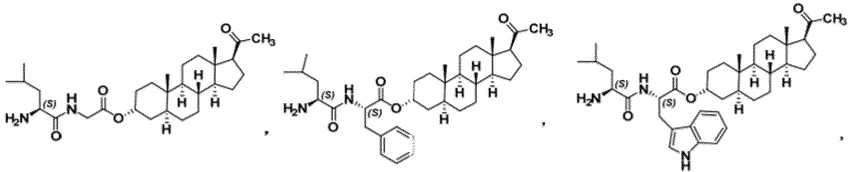

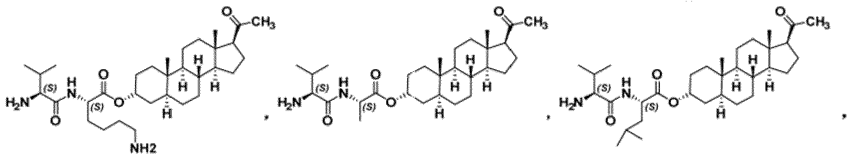

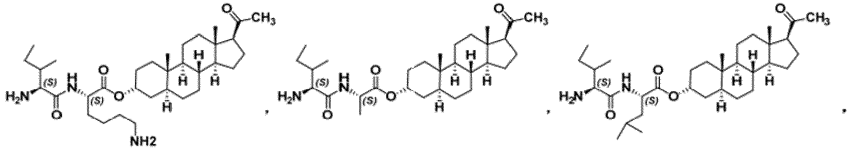

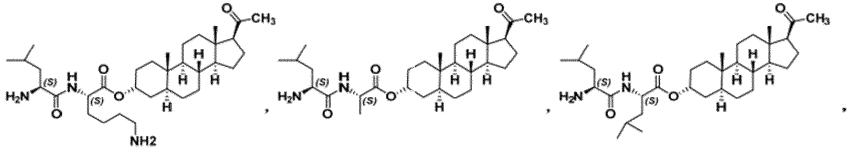

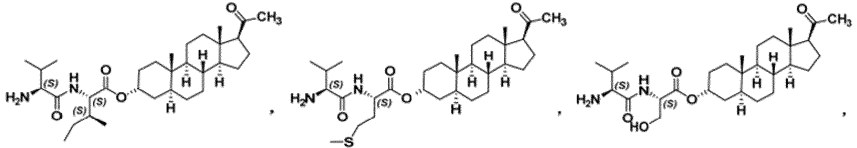

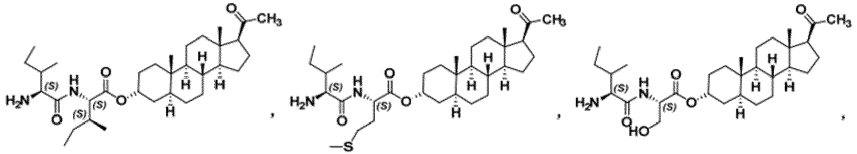

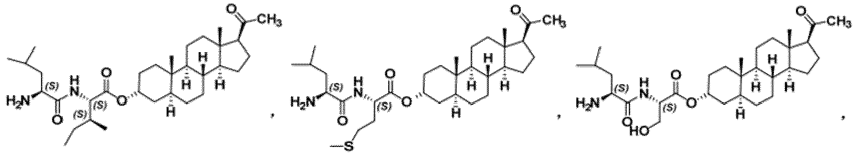

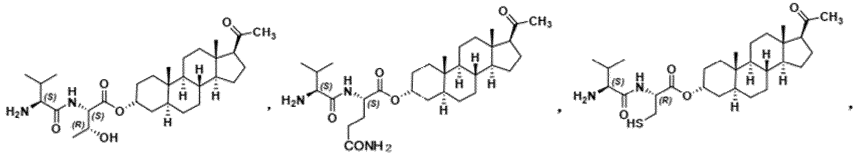

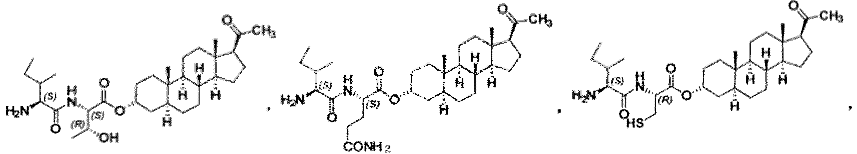

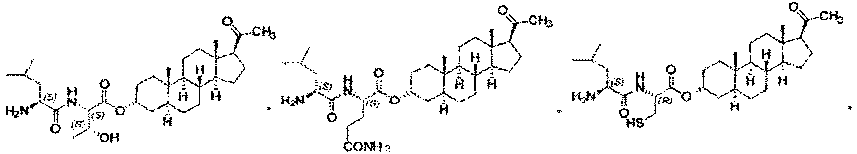

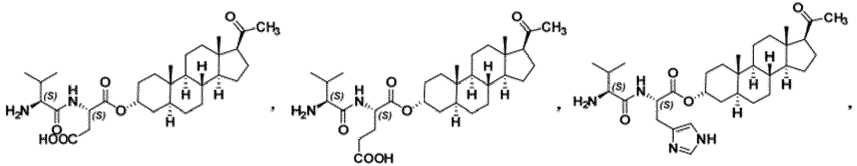

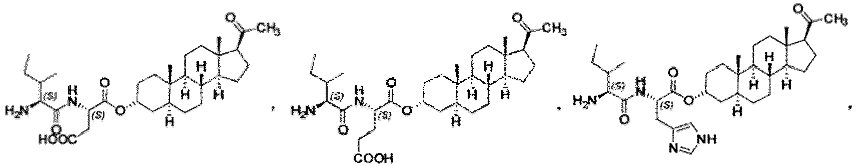

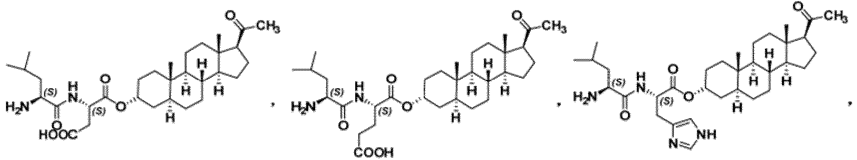

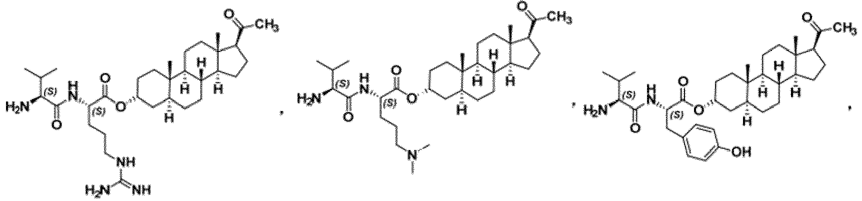

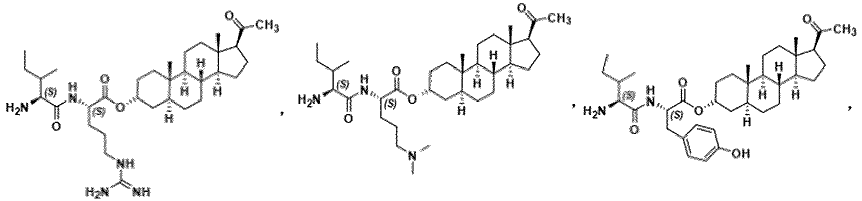

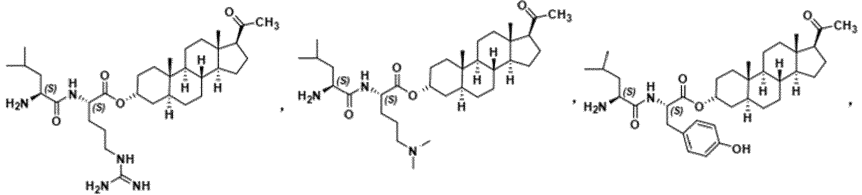

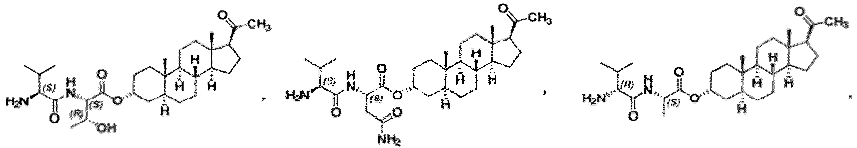

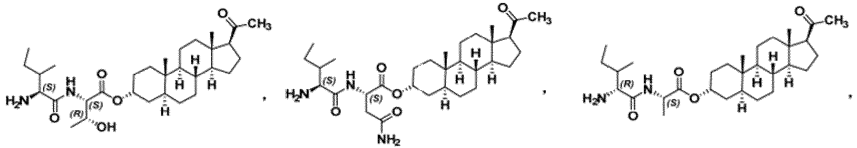

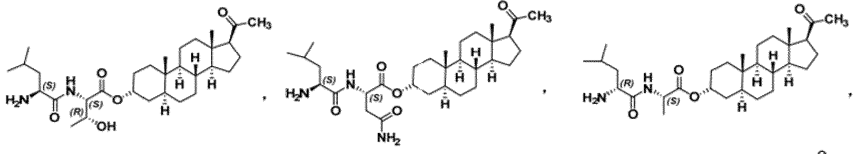

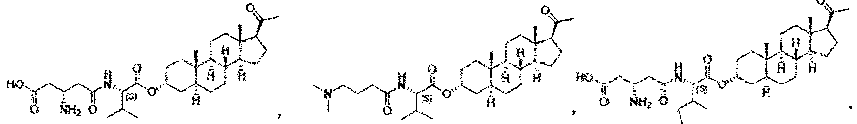

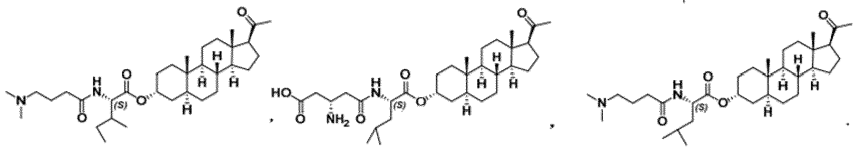

7. The compound or a pharmaceutically acceptable salt thereof of claim 6,
wherein the compound is selected from the group consisting of:

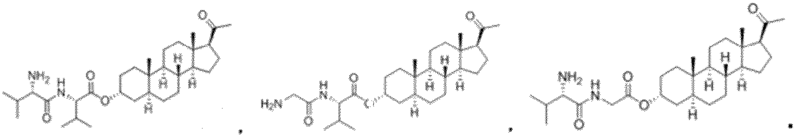

8. A pharmaceutical composition comprising a therapeutically effective dose of the compound or the pharmaceutically acceptable salt thereof of any one of claims 1-7, and a pharmaceutically acceptable carrier and/or excipient.

9. The compound or the pharmaceutically acceptable salt thereof of any one of claims 1-7, or the pharmaceutical composition of claim 8 for use as a medicament for preventing or treating a disorder of the central nervous system.

10. The compound or the pharmaceutically acceptable salt thereof or the pharmaceutical composition for use of claim 9, wherein the disorder of the central nervous system includes, but is not limited to, tremor, a sleep disorder, depression, a depressive disorder, a bipolar disorder, an anxiety disorder, a stress response, a post-traumatic stress disorder, an obsessive-compulsive disorder, schizophrenia, a schizoaffective disorder, epilepsy, epileptic seizure, memory impairment and/or cognitive impairment, dementia, dyskinesia, a personality disorder, autism, single-cause autism, pain, a traumatic brain injury, a vascular disease, a substance abuse disorder and/or withdrawal syndrome or tinnitus; or
the disorder of the central nervous system includes, but is not limited to, essential tremor, epilepsy, clinical depression, postnatal or postpartum depression, atypical depression, psychotic major depression, catatonic depression, a seasonal affective disorder, dysthymia, double depression, a depressive personality disorder, recurrent brief depression, a mild depressive disorder, a bipolar disorder or a manic-depressive disorder, a post-traumatic stress disorder, depression due to chronic medical conditions, treatment-resistant depression, refractory depression, suicidal tendency, suicidal ideation, a suicidal behavior, a traumatic brain injury, a generalized anxiety disorder, a social anxiety disorder, an attention deficit hyperactivity disorder, dementia, Huntington's disease, Parkinson's disease, neuropathic pain, an injury-related pain syndrome, acute pain, long-term pain, stroke, ischemia, vascular malformation, addiction to opioids, cocaine and/or alcohol, or insomnia.

## Patentansprüche

1. Verbindung der Formel I oder pharmazeutisch annehmbares Salz davon: 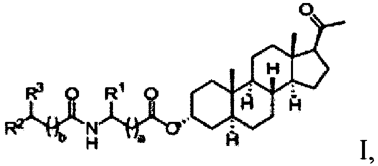 worin
die R¹ und R² jeweils unabhängig H, ein substituiertes oder unsubstituiertes C₁-C₁₀-Alkyl oder ein substituiertes oder unsubstituiertes Cycloalkyl sind; R³ H, Halogen, Hydroxyl, Amino, Nitryl oder Sulfydryl ist; oder R² und R³ miteinander zu einem 5- bis 6-gliedrigen, gesättigten oder ungesättigten heterozyklischen Ring verbunden sind; und
a und b jeweils unabhängig eine ganze Zahl von 0 bis 3 sind.

2. Verbindung oder pharmazeutisch annehmbares Salz davon nach Anspruch 1, worin R³ ein Amino oder H ist.

3. Verbindung oder pharmazeutisch annehmbares Salz davon nach Anspruch 1 oder 2, wobei die Verbindung der Formel I eine Verbindung der Formel I-1, I-2, 1-3 oder 1-4 ist: 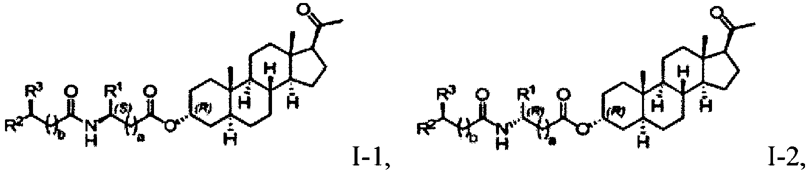 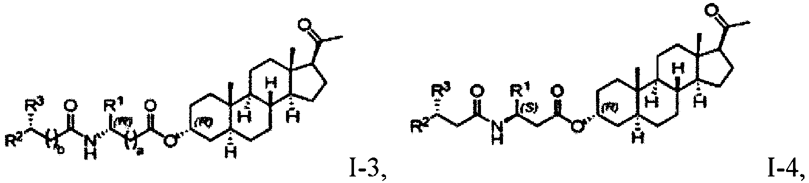 worin R¹, R², R³, a und b wie in Anspruch 1 oder 2 beschrieben sind.

4. Verbindung oder pharmazeutisch annehmbares Salz davon nach Anspruch 1 oder 2, wobei zumindest einer von R¹ und R² ein Isopropyl ist.

5. Verbindung oder pharmazeutisch annehmbares Salz davon nach Anspruch 4, worin R¹ ein Isopropyl ist.

6. Verbindung oder pharmazeutisch annehmbares Salz davon nach Anspruch 1, wobei die Verbindung aus der aus den folgenden bestehenden Gruppe ausgewählt ist:

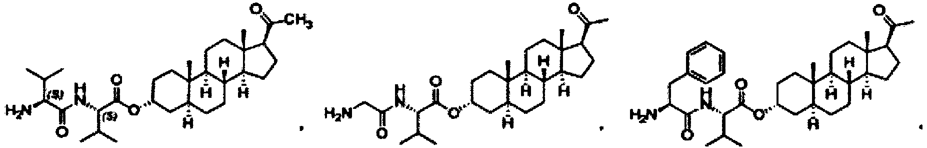

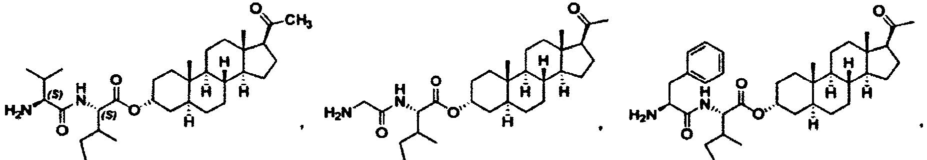

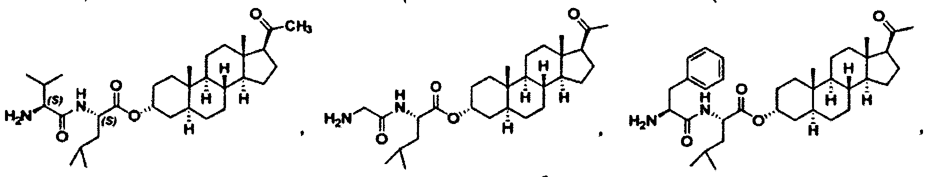

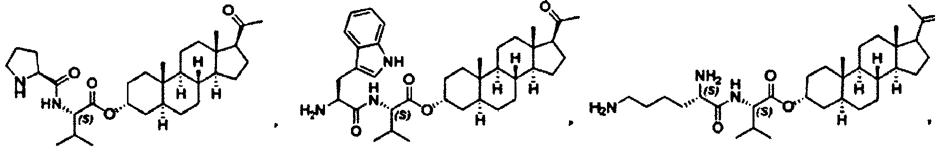

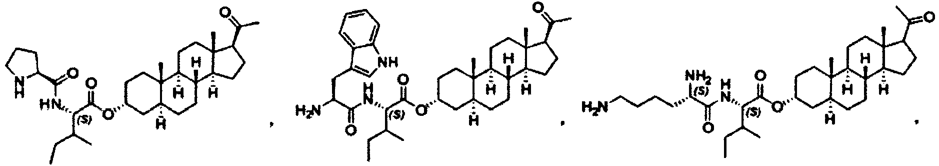

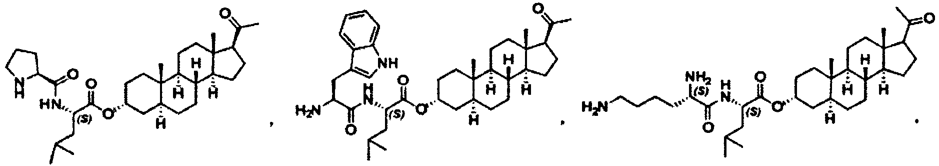

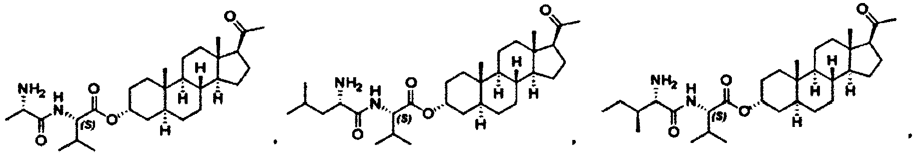

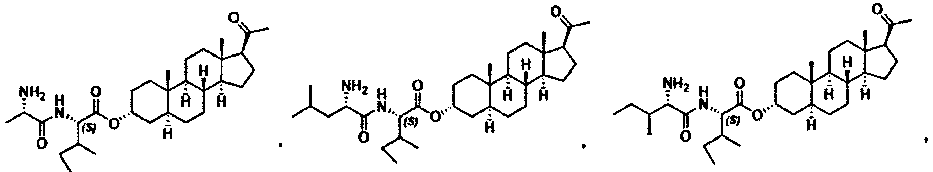

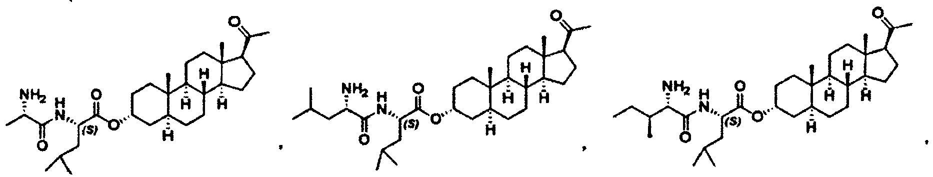

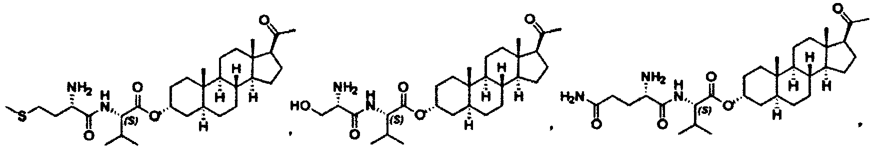

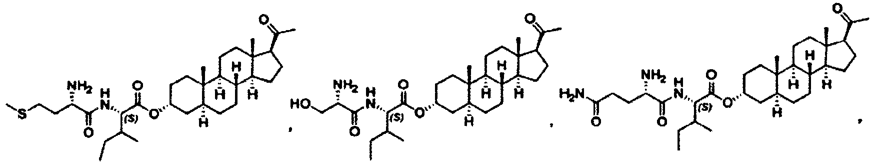

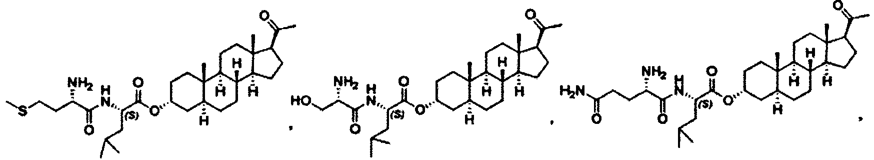

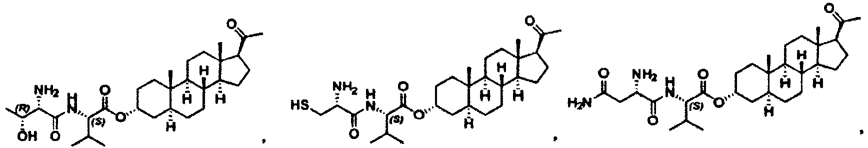

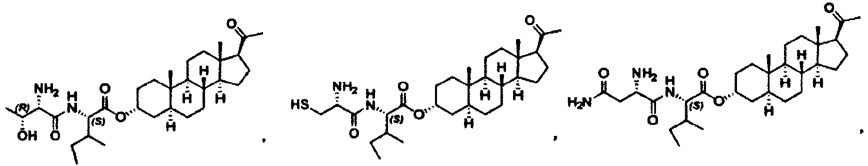

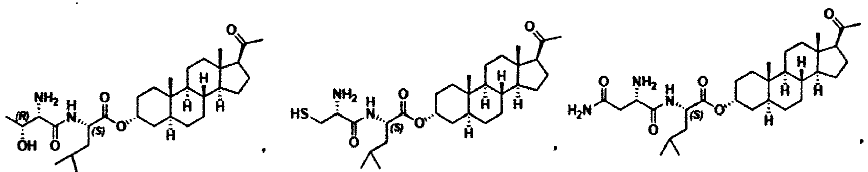

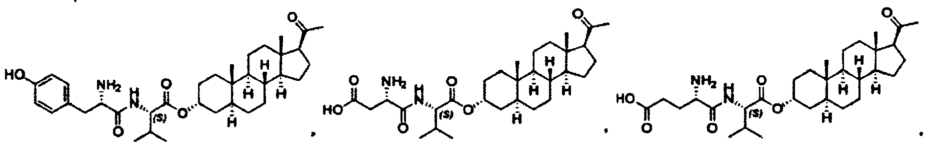

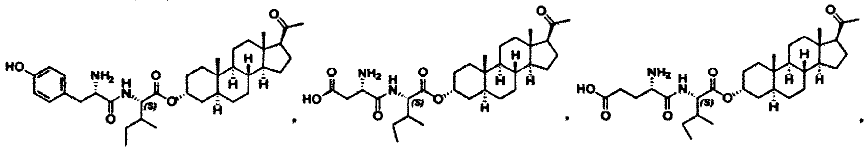

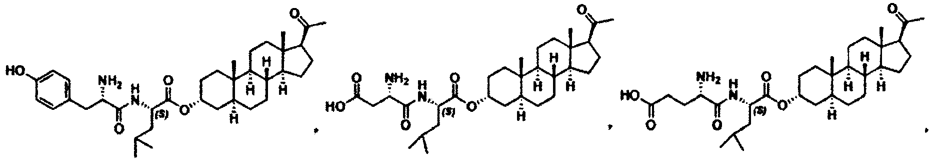

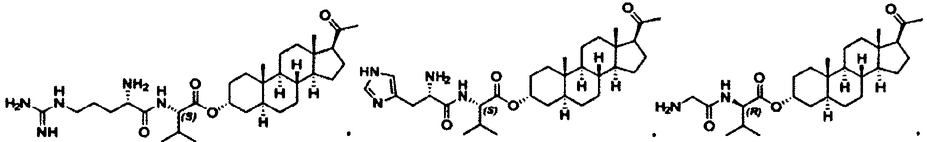

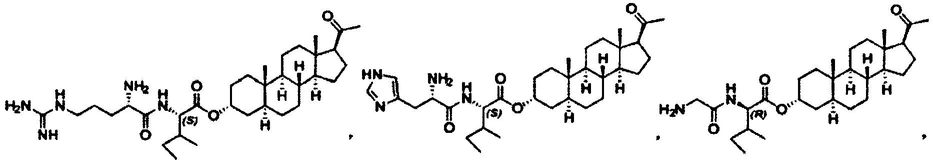

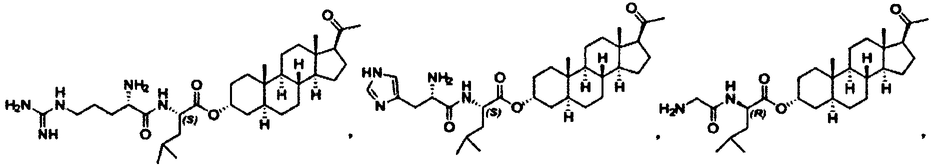

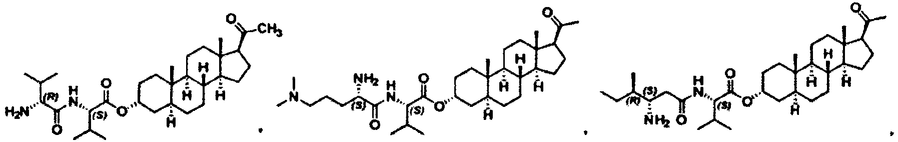

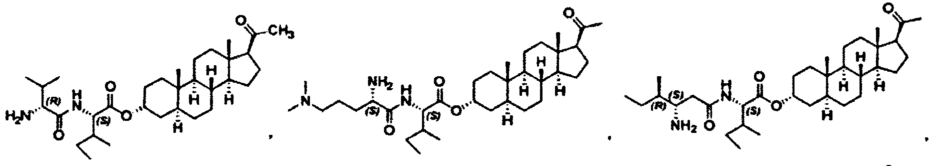

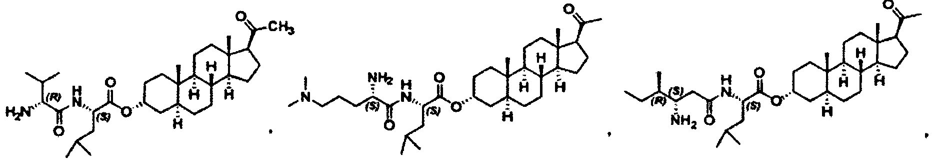

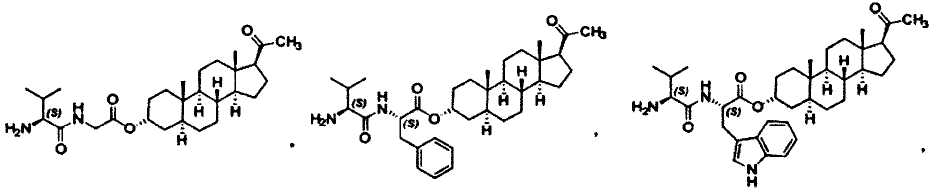

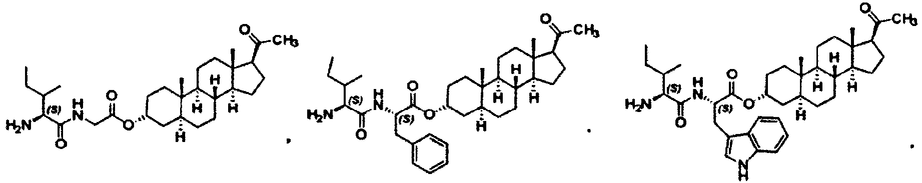

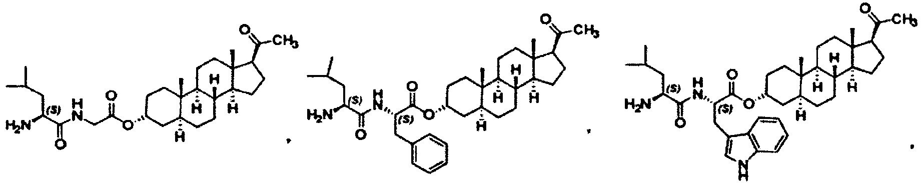

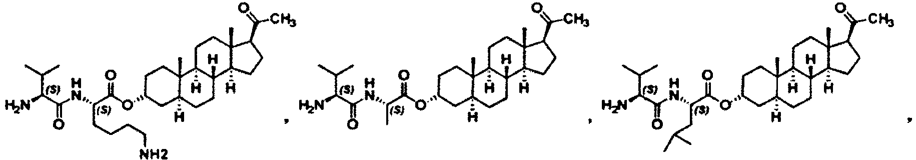

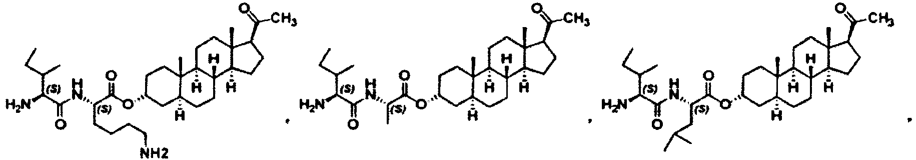

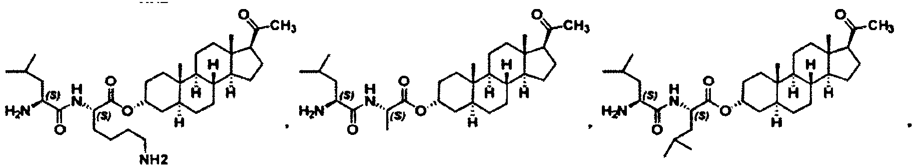

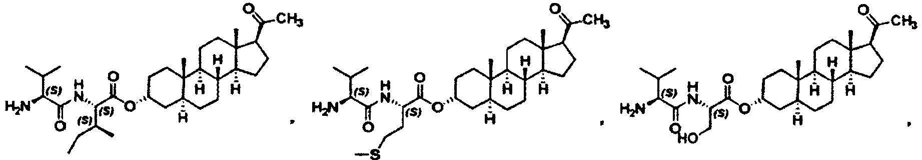

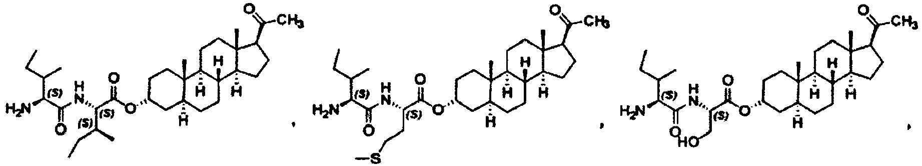

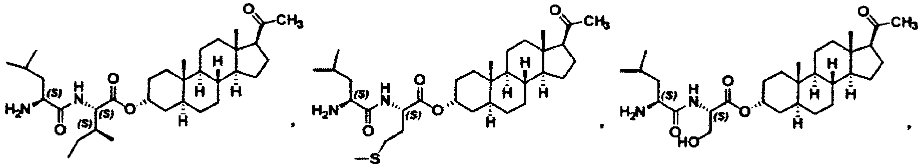

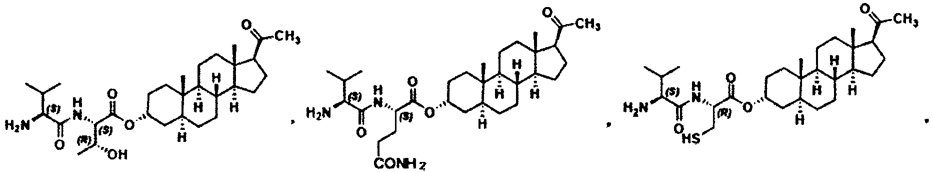

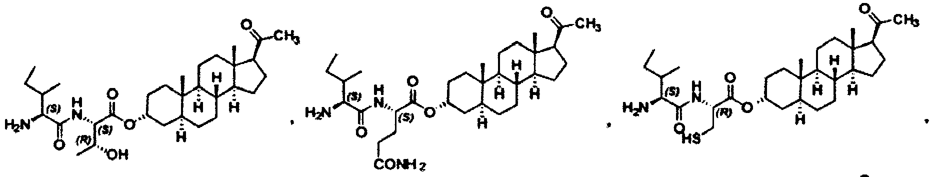

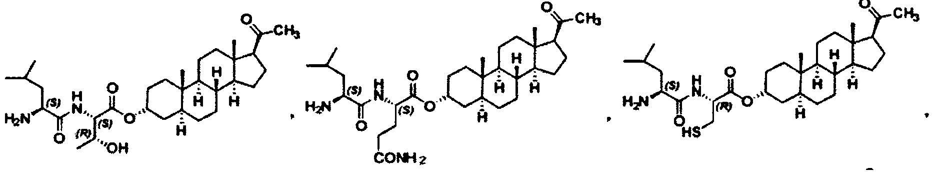

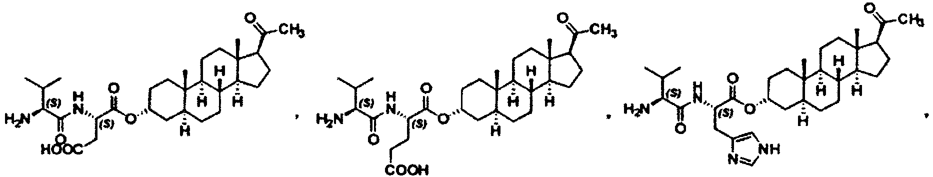

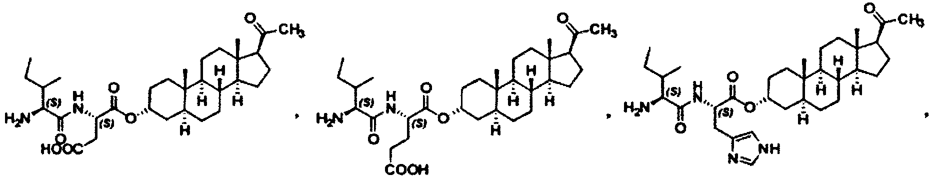

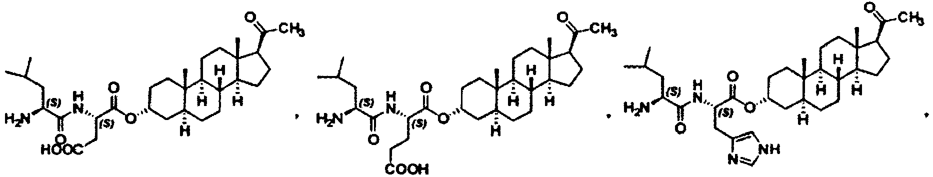

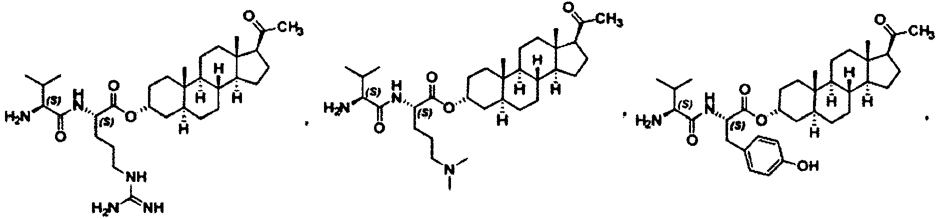

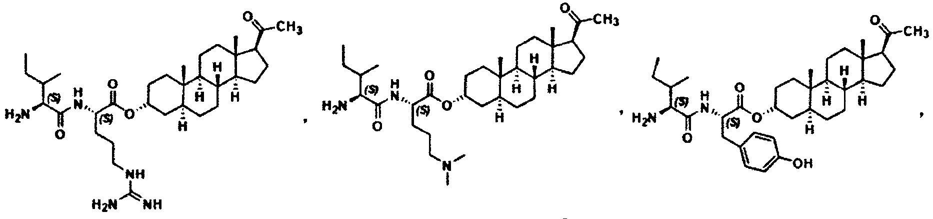

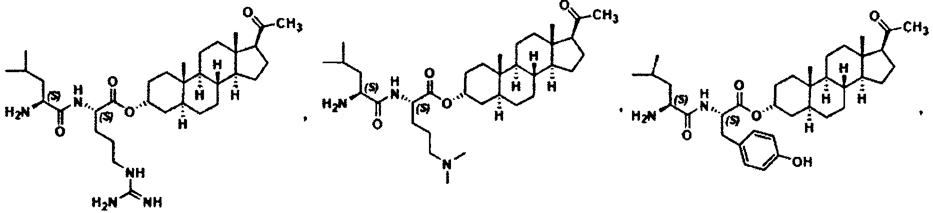

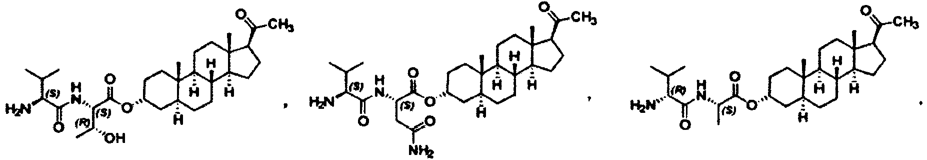

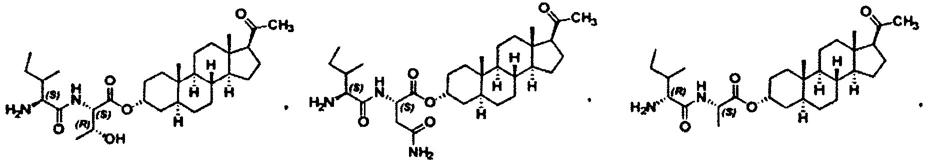

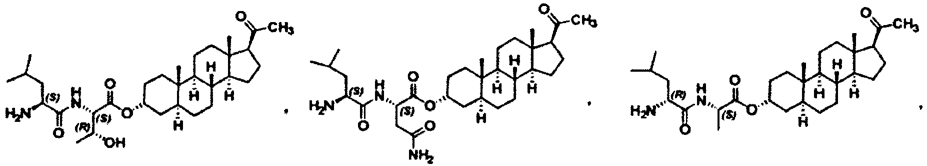

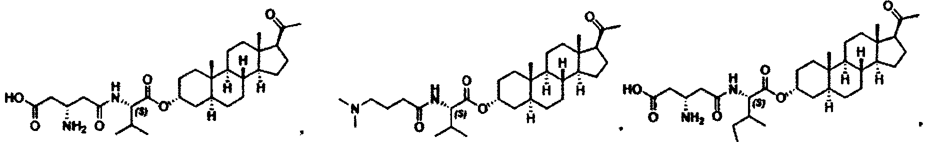

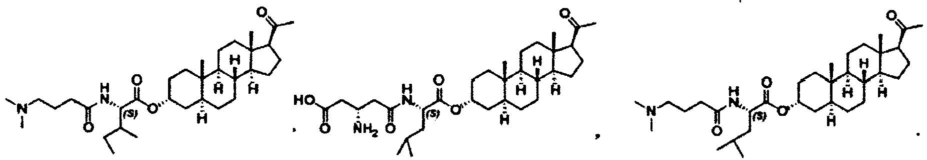

7. Verbindung oder pharmazeutisch annehmbares Salz davon nach Anspruch 6, wobei die Verbindung aus der aus den folgenden bestehenden Gruppe ausgewählt ist:

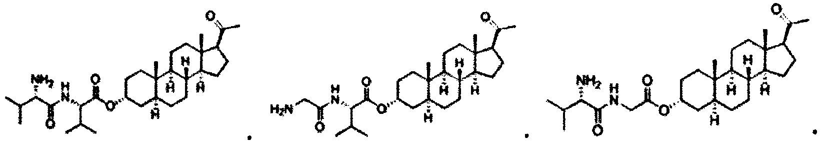

8. Pharmazeutische Zusammensetzung, die eine therapeutisch wirksame Dosis einer Verbindung oder eines pharmazeutisch annehmbaren Salzes davon nach einem der Ansprüche 1 bis 7 und einen pharmazeutisch annehmbaren Träger und/oder Hilfsstoff umfasst.

9. Verbindung oder pharmazeutisch annehmbares Salz davon nach einem der Ansprüche 1 bis 7 oder pharmazeutische Zusammensetzung nach Anspruch 8 zur Verwendung als Medikament zur Prävention oder Behandlung einer Störung des Zentralnervensystems.

10. Verbindung oder pharmazeutisch annehmbares Salz davon oder pharmazeutische Zusammensetzung zur Verwendung nach Anspruch 9, wobei die Störung des Zentralnervensystems Tremor, eine Schlafstörung, Depression, eine depressive Störung, eine bipolare Störung, eine Angststörung, eine Stressreaktion, eine posttraumatische Belastungsstörung, eine Zwangsstörung, Schizophrenie, eine schizoaffektive Störung, Epilepsie, ein epileptischer Anfall, eine Gedächtnisstörung und/oder kognitive Störung, Demenz, Dyskinesie, eine Persönlichkeitsstörung, auf eine Ursache zurückgeführter Autismus, Schmerz, eine traumatische Hirnverletzung, eine Gefäßerkrankung, eine Substanzmissbrauchsstörung und/oder ein Entzugssyndrom oder Tinnitus umfasst, aber nicht darauf eingeschränkt ist; oder
die Störung des Zentralnervensystems essentieller Tremor, Epilepsie, eine klinische Depression, eine postnatale oder postpartale Depression, eine atypische Depression, eine schwere psychotische Depression, eine katatonische Depression, eine saisonale affektive Störung, Dysthymie, eine doppelte Depression, eine depressive Persönlichkeitsstörung, eine wiederkehrende kurze Depression, eine leichte depressive Störung, eine bipolare Störung oder eine manisch-depressive Störung, eine posttraumatische Belastungsstörung, Depression aufgrund von chronischen medizinischen Leiden, eine behandlungsresistente Depression, eine therapieresistente Depression, Suizidneigung, Suizidgedanken, suizidales Verhalten, eine traumatische Hirnverletzung, eine generalisierte Angststörung, eine soziale Angststörung, eine Aufmerksamkeitsdefizit-Hyperaktivitätsstörung, Demenz, Morbus Huntington, Morbus Parkinson, neuropathischer Schmerz, ein verletzungsbedingtes Schmerzsyndrom, akuter Schmerz, Langzeitschmerz, einen Schlaganfall, Ischämie, eine Gefäßmalformation, Abhängigkeit von Opioiden, Kokain und/oder Alkohol oder Insomnie umfasst, aber nicht darauf eingeschränkt ist.

## Revendications

1. Composé de formule I, ou un sel pharmaceutiquement acceptable de celui-ci : 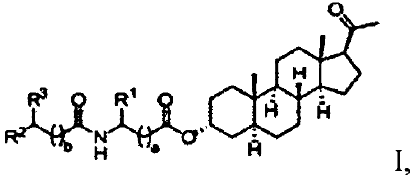 dans lequel,
chaque R¹ et R² est indépendamment H, un groupe alkyle en C₁ à C₁₀ substitué ou non substitué ou un groupe cycloalkyle substitué ou non substitué ; R³ est H, un atome d'halogène, un groupe hydroxyle, un groupe amino, un groupe nitryle ou un groupe sulfydryle ; ou R² et R³ sont liés pour former un cycle hétérocyclique à 5 à 6 chaînons saturé ou insaturé ; et
chaque a et b est indépendamment un nombre entier de 0 à 3.

2. Composé ou sel pharmaceutiquement acceptable de celui-ci selon la revendication 1, dans lequel R³ est un groupe amino ou H.

3. Composé ou sel pharmaceutiquement acceptable de celui-ci selon la revendication 1 ou 2, dans lequel le composé de formule I est le composé de formule I-1, I-2, I-3 ou I-4 : 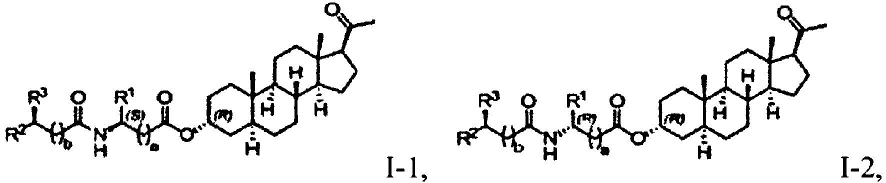 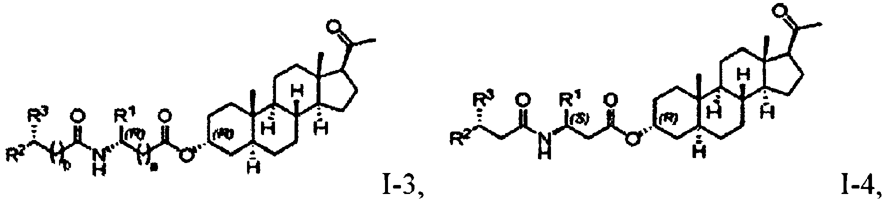 dans lequel R¹, R², R³, a et b sont tels que décrits dans la revendication 1 ou 2.

4. Composé ou sel pharmaceutiquement acceptable de celui-ci selon la revendication 1 ou 2, dans lequel au moins l'un de R¹ et R² est un groupe isopropyle.

5. Composé ou sel pharmaceutiquement acceptable de celui-ci selon la revendication 4, dans lequel R¹ est un groupe isopropyle.

6. Composé ou sel pharmaceutiquement acceptable de celui-ci selon la revendication 1, dans lequel le composé est choisi dans le groupe constitué par :

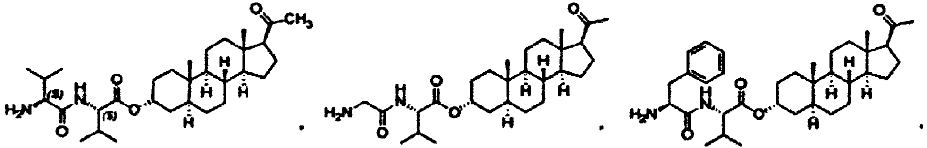

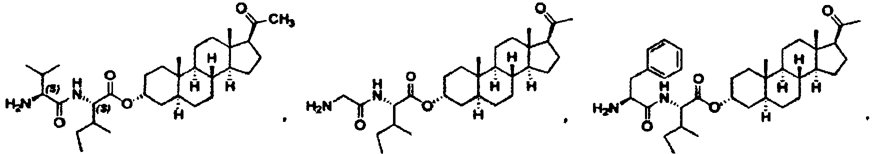

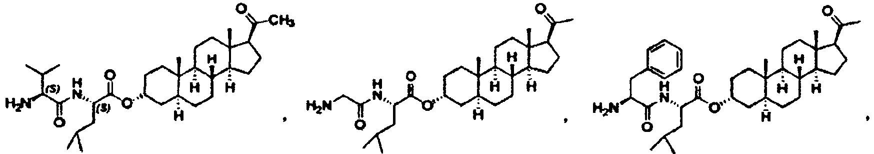

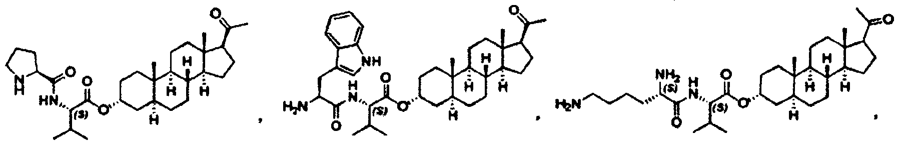

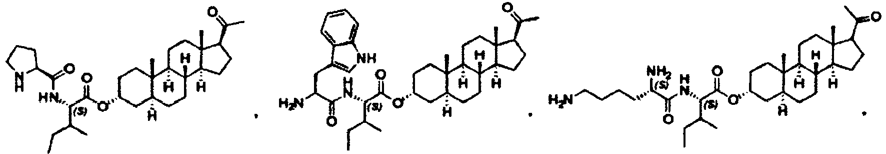

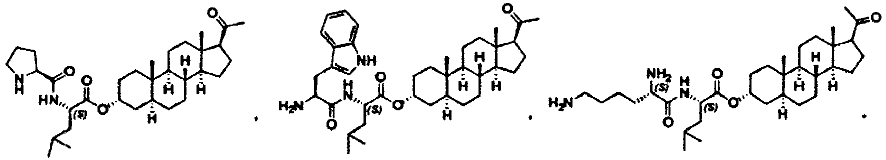

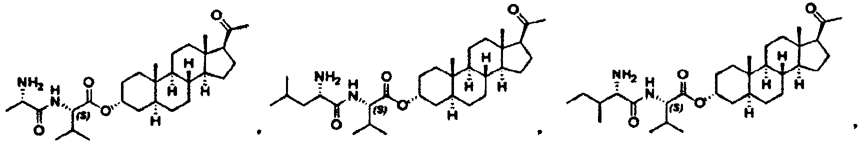

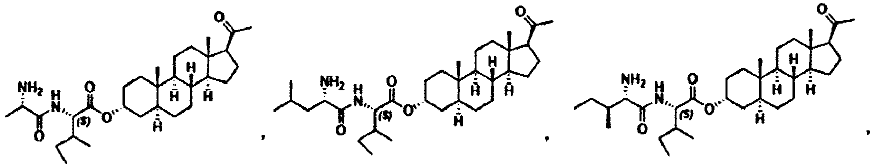

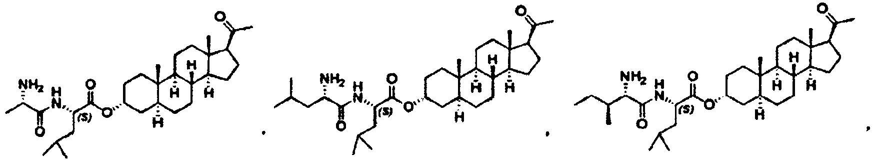

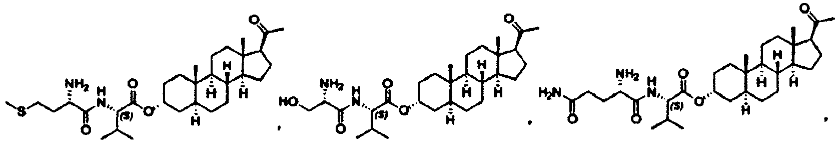

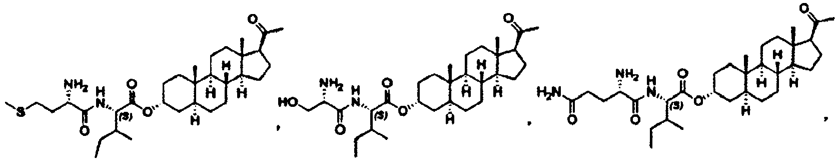

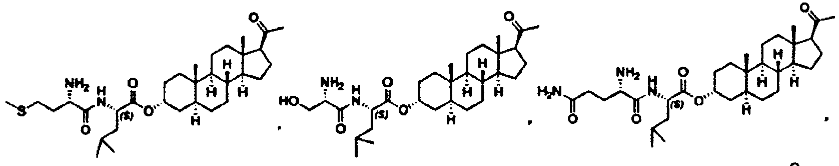

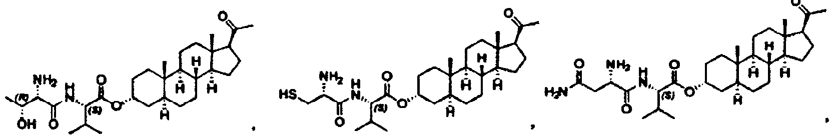

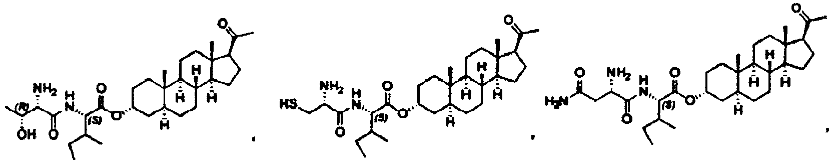

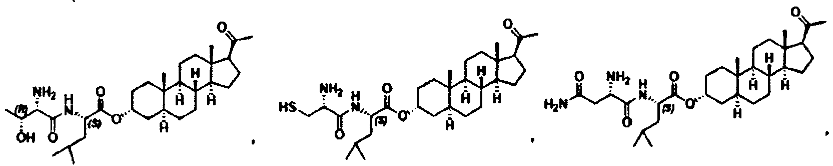

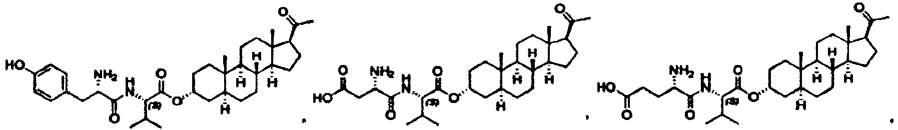

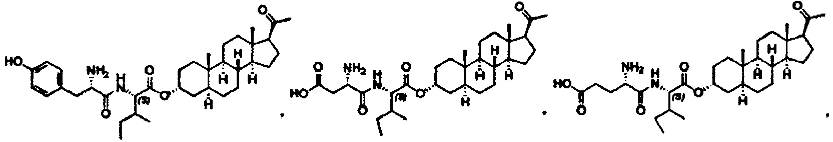

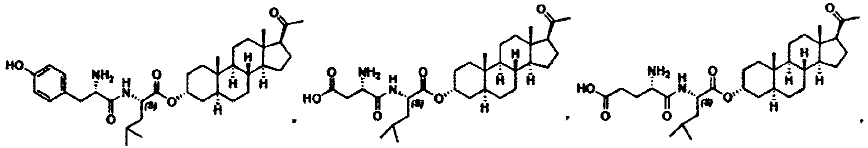

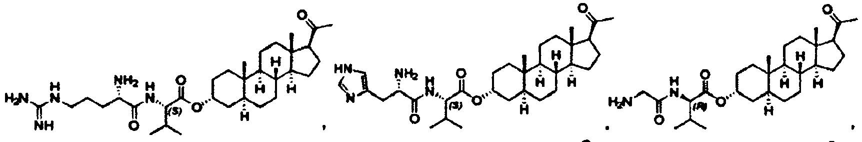

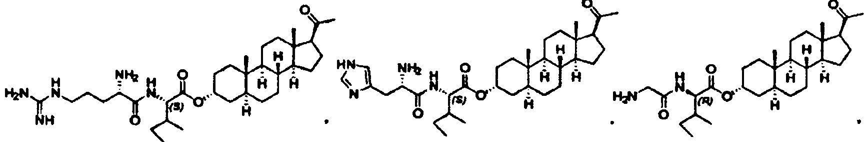

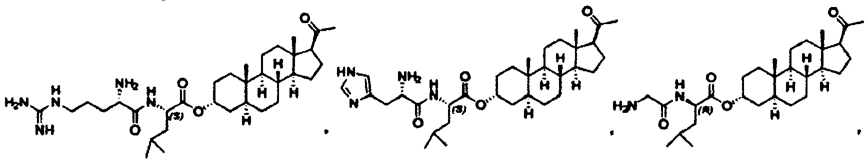

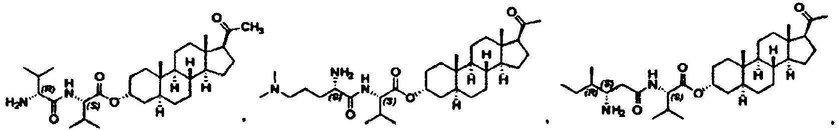

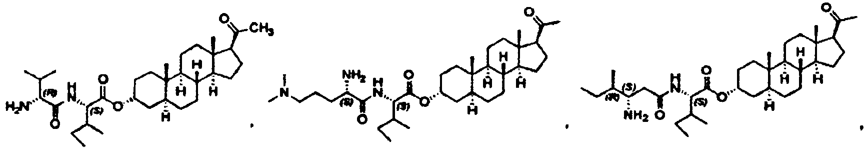

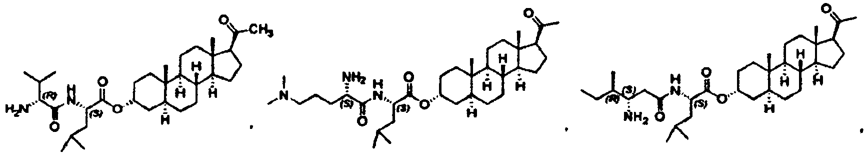

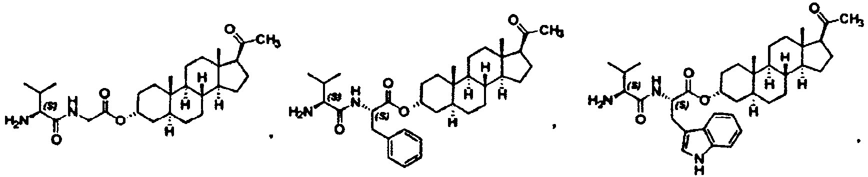

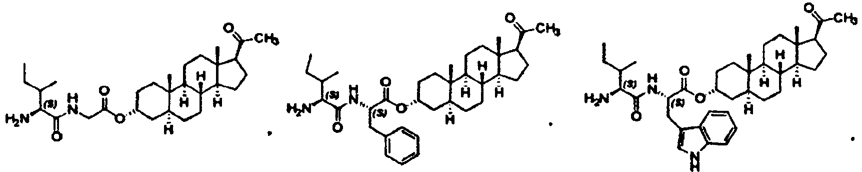

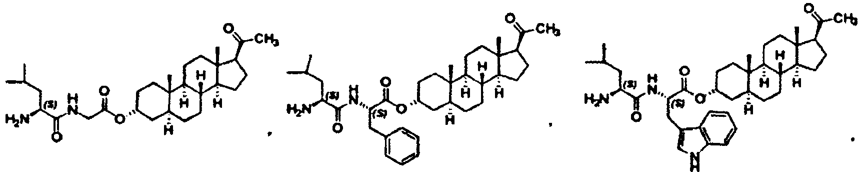

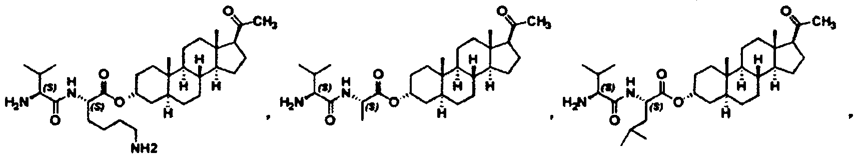

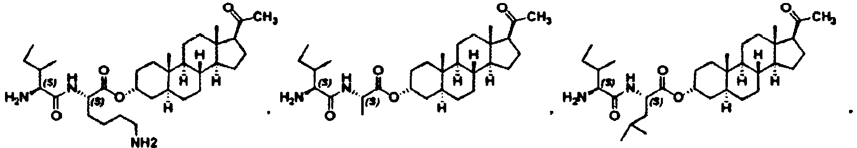

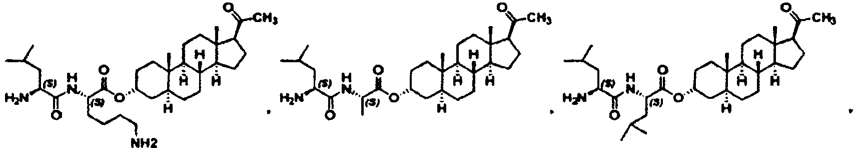

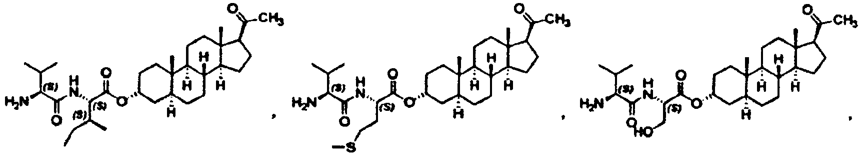

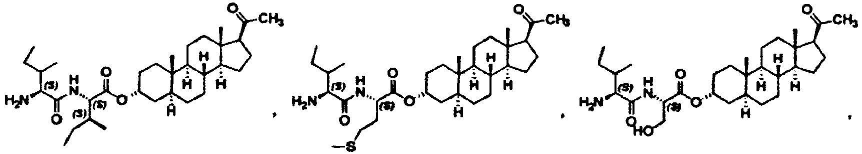

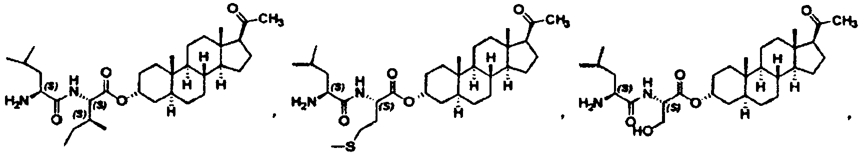

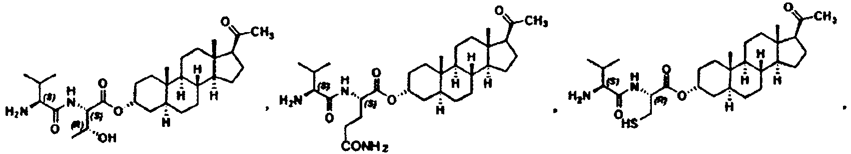

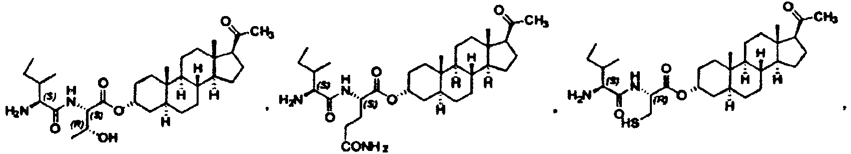

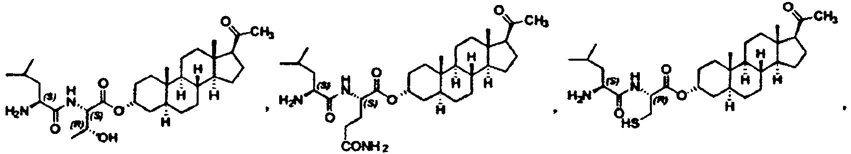

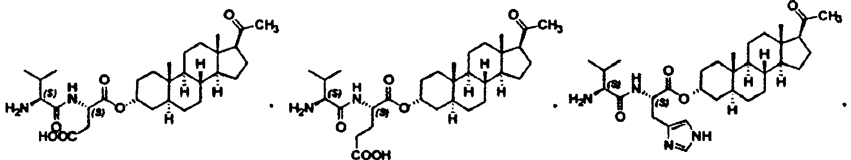

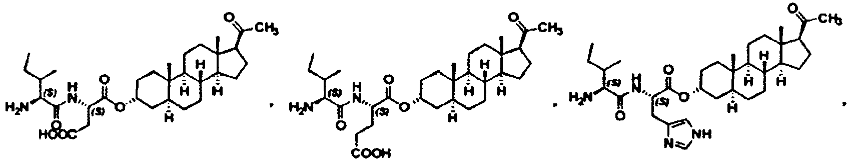

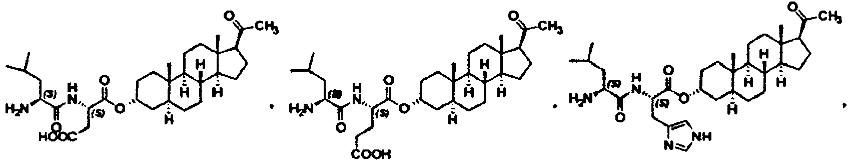

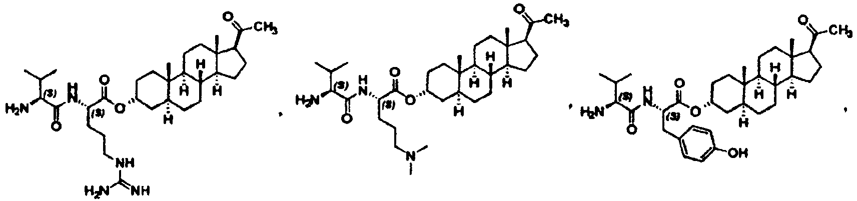

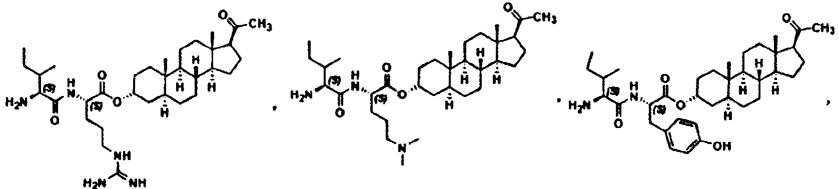

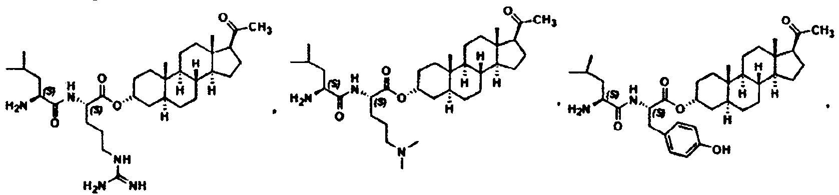

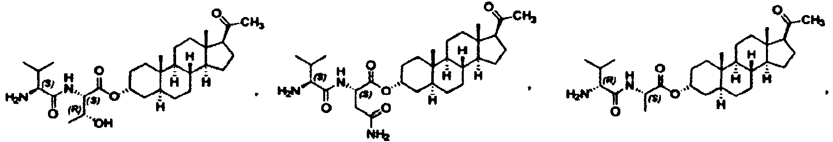

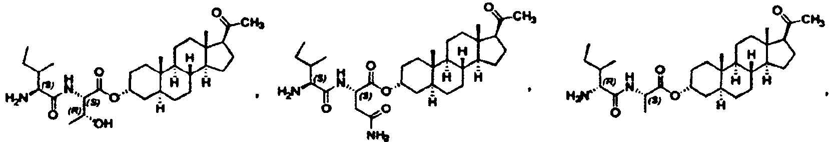

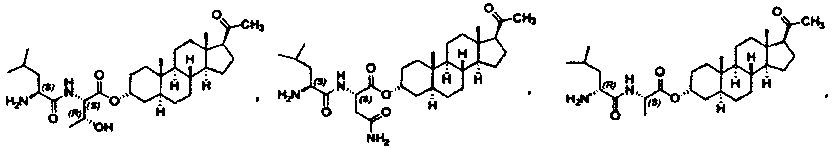

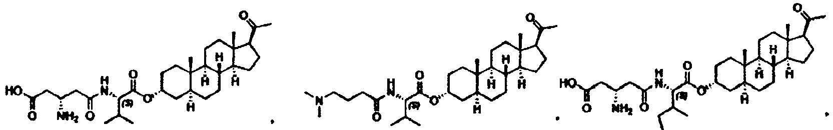

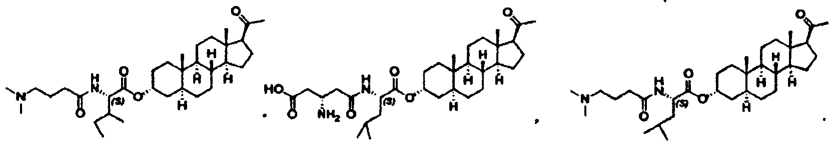

7. Composé ou sel pharmaceutiquement acceptable de celui-ci selon la revendication 6, dans lequel le composé est choisi dans le groupe constitué par :

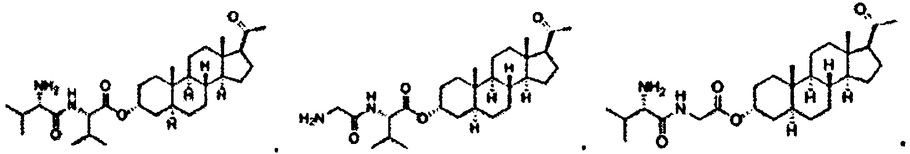

8. Composition pharmaceutique comprenant une dose thérapeutiquement efficace du composé ou du sel pharmaceutiquement acceptable de celui-ci selon l'une quelconque des revendications 1 à 7, et un excipient et/ou un véhicule pharmaceutiquement acceptable.

9. Composé ou sel pharmaceutiquement acceptable de celui-ci selon l'une quelconque des revendications 1 à 7, ou composition pharmaceutique selon la revendication 8 pour une utilisation en tant que médicament pour la prévention ou au traitement d'un trouble du système nerveux central.

10. Composé ou sel pharmaceutiquement acceptable de celui-ci ou composition pharmaceutique pour une utilisation selon la revendication 9, dans lesquels le trouble du système nerveux central comprend, sans toutefois s'y limiter, les tremblements, un trouble du sommeil, une dépression, un trouble dépressif, un trouble bipolaire, un trouble anxieux, une réponse au stress, un trouble de stress post-traumatique, un trouble obsessionnel compulsif, une schizophrénie, un trouble schizo-affectif, une épilepsie, une crise d'épilepsie, les troubles de la mémoire et/ou les troubles cognitifs, une démence, la dyskinésie, un trouble de la personnalité, un autisme, un autisme à cause unique, une douleur, une lésion cérébrale traumatique, une maladie vasculaire, un trouble lié à l'abus de substances et/ou un syndrome de sevrage ou des acouphènes ; ou
le trouble du système nerveux central comprend, sans toutefois s'y limiter, les tremblements essentiels, l'épilepsie, la dépression clinique, la dépression postnatale ou post-partum, la dépression atypique, la dépression majeure psychotique, la dépression catatonique, le trouble affectif saisonnier, la dysthymie, la dépression double, un trouble de la personnalité dépressive, la dépression brève récurrente, un trouble dépressif léger, un trouble bipolaire ou un trouble maniaco-dépressif, un trouble de stress post-traumatique, la dépression due à des affections médicales chroniques, la dépression résistante au traitement, la dépression réfractaire, une tendance suicidaire, les idées suicidaires, un comportement suicidaire, une lésion cérébrale traumatique, un trouble anxieux généralisé, un trouble d'anxiété sociale, un trouble déficitaire de l'attention avec hyperactivité, la démence, la maladie de Huntington, la maladie de Parkinson, la douleur neuropathique, un syndrome douloureux lié à une blessure, la douleur aiguë, la douleur chronique, l'accident vasculaire cérébral, l'ischémie, la malformation vasculaire, la dépendance aux opioïdes, à la cocaïne et/ou à l'alcool, ou l'insomnie.
